(19) **Europäisches Patentamt / European Patent Office / Office européen des brevets**

(11) **EP 4 495 140 A1**

(12) **EUROPEAN PATENT APPLICATION**
published in accordance with Art. 153(4) EPC

(43) Date of publication:
**22.01.2025 Bulletin 2025/04**

(21) Application number: **23769907.9**

(22) Date of filing: **17.03.2023**

(51) International Patent Classification (IPC):
*C07K 16/28* (2006.01)    *C07K 19/00* (2006.01)
*C12N 5/10* (2006.01)    *C12N 15/13* (2006.01)
*C12N 15/62* (2006.01)    *C12N 15/867* (2006.01)
*G01N 33/574* (2006.01)    *G01N 33/68* (2006.01)
*A61K 39/00* (2006.01)    *A61P 35/00* (2006.01)

(52) Cooperative Patent Classification (CPC):
**A61K 39/00; A61K 39/395; A61P 35/00;
C07K 16/00; C07K 16/28; C07K 19/00; C12N 5/10;
C12N 15/62; C12N 15/867; G01N 33/574;
G01N 33/68**

(86) International application number:
**PCT/CN2023/082174**

(87) International publication number:
**WO 2023/174405 (21.09.2023 Gazette 2023/38)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **18.03.2022 CN 202210272549**

(71) Applicant: **Sunshine Lake Pharma Co., Ltd.
Dongguan, Guangdong 523000 (CN)**

(72) Inventors:
• **DONG, Junji
  Dongguan, Guangdong 523871 (CN)**
• **REN, Zhiheng
  Dongguan, Guangdong 523871 (CN)**
• **ZHANG, Kuo
  Dongguan, Guangdong 523871 (CN)**
• **LI, Xiang
  Dongguan, Guangdong 523871 (CN)**
• **LIN, Shushan
  Dongguan, Guangdong 523871 (CN)**
• **PENG, Ju
  Dongguan, Guangdong 523871 (CN)**

• **XUE, Lixuan
  Dongguan, Guangdong 523871 (CN)**
• **ZHOU, Linjun
  Dongguan, Guangdong 523871 (CN)**
• **JIA, Xingfan
  Dongguan, Guangdong 523871 (CN)**
• **HUANG, Limei
  Dongguan, Guangdong 523871 (CN)**
• **LI, Kaifeng
  Dongguan, Guangdong 523871 (CN)**
• **ZHAO, Guanghui
  Dongguan, Guangdong 523871 (CN)**
• **CHEN, Shiyou
  Dongguan, Guangdong 523871 (CN)**
• **TAN, Xiumei
  Dongguan, Guangdong 523871 (CN)**
• **LI, Wenjia
  Dongguan, Guangdong 523871 (CN)**

(74) Representative: **Hoffmann Eitle
Patent- und Rechtsanwälte PartmbB
Arabellastraße 30
81925 München (DE)**

Remarks:
The complete document including Reference Table(s) and the Sequence Listing(s) can be downloaded from the EPO website

(54) **CLAUDIN18.2 HUMANIZED ANTIBODY AND APPLICATION THEREOF**

(57) The present invention provides a Claudin18.2 humanized antibody and application thereof. The antibody contains a chain variable region (CDR) sequence selected from at least one of the following or an amino acid sequence having at least 90% identity therewith: light chain CDR sequences: SEQ ID NOs: 1-3 and 7-9; and heavy chain CDR sequences: SEQ ID NOs: 4-6 and 10-12; The antibody or an antigen-binding fragment

**(Cont. next page)**

EP 4 495 140 A1

thereof have humanized modification. The humanized antibody according to embodiments of the present invention can specifically target and bind Claudin18.2, has the same in vitro activity as a human-mouse chimeric anti-Claudin 18.2 monoclonal antibody, meets the requirements of druggability, and has significantly prolonged in vivo half-life.

**Description**

**CROSS-REFERENCE TO RELATED APPLICATION**

This application claims the priority and benefits of Chinese Patent Application No. 202210272549.0, filed with the State Intellectual Property Office of China on March 18, 2022, which is incorporated herein by reference in its entirety.

**FIELD OF THE INVENTION**

The present invention relates to the field of biotechnology. Specifically, the present invention relates to Claudin18.2 humanized antibody and application thereof. More specifically, the present invention relates to antibodies capable of specifically recognizing Claudin18.2 or antigen-binding fragments thereof, nucleic acid molecules, expression vectors, recombinant cells, pharmaceutical compositions, pharmaceutical uses and kits for detecting Claudin18.2.

**BACKGROUND ART**

Claudins (CLDN) are a family of proteins whose role is to maintain tight junctions that control the exchange of molecules between cells. It is widely distributed in stomach, pancreas and lung tissues and can be used to diagnose and treat diseases. Claudin18.2 subtype is a gastric-specific subtype and has become an ideal target since it was found that Claudin18.2 is a highly selective molecule and is only widely expressed in cancer cells. Claudin18.2 is usually buried in the gastric mucosa and is basically inaccessible to monoclonal antibodies in normal tissues. The occurrence of malignant tumors can lead to the destruction of tight junctions, which exposing the Claudin18.2 epitopes on the surface of tumor cells and becoming a specific target. Therefore, Claudin18.2 confers specificity to targeted therapy.

At present, there are murine anti-Claudin 18.2 monoclonal antibodies prepared by hybridoma technology using immunized mice. However, mouse anti-Claudin 18.2 monoclonal antibodies have high immunogenicity when entering the human body, thus affecting the pharmacokinetics in the human body. Therefore, there is still a need to further develop more effective therapeutic antibodies against Claudin18.2.

**SUMMARY OF THE INVENTION**

This application is filed based on the inventor's discovery of the following issues and facts:
Claudins are widely distributed in the stomach, pancreas and lung tissues. When malignant tumors occur, the Claudin18.2 epitope on the surface of tumor cells is exposed. Therefore, Claudin18.2 can be used as an effective target for the development of new therapeutic drugs. Currently, there are murine Claudin18.2 monoclonal antibodies, but they have high immunogenicity; therefore, the inventors of the present application replaced the framework region (FR region) and constant region of the anti-Claudin18.2 monoclonal antibody with human ones, retained the CDR region of the variable region of the murine anti-Claudin18.2 monoclonal antibody, and obtained a series of anti-Claudin 18.2 humanized antibodies. The inventors found that the humanized antibodies obtained in the present application can not only specifically target and bind to Claudin18.2 and have the same binding activity as the chimeric antibodies, but also have a longer half-life *in vivo.*

Therefore, in the first aspect of the present invention, the present invention provides an antibody or antigen-binding fragment thereof capable of specifically recognizing Claudin18.2. According to an embodiment of the present invention, the antibody comprises at least one CDR sequence selected from the following or an amino acid sequence having at least 90% identity with it: the CDR sequences of light chain variable region: $X_1$SSQSLFNSGNQKNYLT (SEQ ID NO: 1), WASTRES (SEQ ID NO: 2), QNDYSYPLT (SEQ ID NO: 3), $X_4$SSQSLLNSGNQKNYLT (SEQ ID NO: 7), WASTRES (SEQ ID NO: 8) and QNDYSYPFT (SEQ ID NO: 9); the CDR sequences of heavy chain variable region: DYNMH (SEQ ID NO: 4),YINPNNGGTSYX$_2$QKFX$_3$G (SEQ ID NO: 5), TRYLAV (SEQ ID NO: 6), SYWMH (SEQ ID NO: 10), MHTPNSGSTNYX$_5$X$_6$KFX$_7$X$_8$ (SEQ ID No: 11) and RYYGSISPDY (SEQ ID NO: 12); wherein, $X_1$ is K or R, $X_2$ is N or S, $X_3$ is K or Q, $X_4$ is K or R, $X_5$ is N or A, $X_6$ is E or Q, $X_7$ is K or Q, $X_8$ is S or G, the antibody or antigen-binding fragment thereof has a humanization modification. It should be noted that the "humanization modification" described in the present application refers to changes in amino acids that can reduce the immunogenicity of the antibody or antigen-binding fragment thereof, including amino acid mutations, insertions, deletions, and additions of chemical groups. The above-mentioned antibody according to the embodiment of the present invention can not only specifically target and bind to Claudin18.2, and has the same binding activity as the chimeric antibody, but also has a longer half-life *in vivo.*

According to an embodiment of the present invention, the aforementioned antibody or antigen-binding fragment may comprise at least one of the following additional technical features:
According to an embodiment of the present invention, the antibody has a light chain variable region with an amino acid sequence as shown in SEQ ID NO: 57-59 or SEQ ID NO: 65-67, and the antibody has a heavy chain variable region with an amino acid sequence as shown in SEQ ID NO: 60-64 or SEQ ID NO: 68-72.

According to an embodiment of the present invention, the antibody comprises at least one of a heavy chain constant region and a light chain constant region, and at least a part of at least one of the heavy chain constant region and the light chain constant region is derived from at least one of a human antibody; the light chain constant region and the heavy chain constant region of the antibody are both derived from a human IgG antibody or a mutant thereof; in addition, the light chain constant region of the antibody is derived from light chain constant region of human Kappa; the heavy chain constant region of the antibody is derived from human IgG4 or IgG1, or a mutant of IgG4 or IgG1. In addition, the full-length sequence of the antibody constant region is shown in SEQ ID NO:73, 74 or 75.

According to an embodiment of the present invention, the antibody has a light chain with sequence as shown in any one of SEQ ID NO: 76-78 and a heavy chain with sequence as shown in any one of SEQ ID NO: 79-88.

According to an embodiment of the present invention, the antibody has a light chain with sequence as shown in any one of SEQ ID NO: 89-91 and a heavy chain with sequence as shown in any one of SEQ ID NO: 92-101.

In the second aspect of the present invention, the present invention provides a nucleic acid molecule. According to an embodiment of the present invention, the nucleic acid molecule encodes the antibody or antigen-binding fragment thereof provided in the first aspect. The antibodies obtained from the nucleic acid molecules of the embodiments of the present invention can not only specifically target and bind to Claudin18.2 with the same binding activity as the chimeric antibody, but also have a longer *in vivo* half-life and reduce the internalization ratio of the antibody.

In the third aspect of the present invention, the present invention provides an expression vector. According to an embodiment of the present invention, the expression vector carries the nucleic acid molecule described above. After the expression vector according to the embodiment of the present invention is introduced into a suitable recipient cell, it can effectively realize the expression of the aforementioned antibody or antigen-binding fragment thereof that specifically recognizes Claudin18.2 under the mediation of the regulatory system, thereby realizing the mass acquisition of the antibody or antigen-binding fragment *in vitro.*

In the fourth aspect of the present invention, the present invention provides an immune cell. According to an embodiment of the present invention, the immune cell carries the nucleic acid molecule described above, or expresses the antibody or antigen-binding fragment thereof described above. According to the embodiments of the present invention, the immune cell can express the aforementioned antibody or antigen-binding fragment thereof that specifically recognize Claudin18.2 in large quantities, so as to obtain the antibody or antigen-binding fragment thereof in large quantities in vitro. Furthermore, the immune cell can be reinfused *in vivo* to treat Claudin18.2-related diseases.

In the fifth aspect of the present invention, the present invention provides a recombinant cell. According to an embodiment of the present invention, the recombinant cell carries the nucleic acid molecule described above, or expresses the antibody or antigen-binding fragment thereof described above. The recombinant cells according to the embodiments of the present invention can be used for the in vitro expression and mass acquisition of the aforementioned antibodies or antigen-binding fragments specifically recognizing Claudin18.2.

In the sixth aspect of the present invention, the present invention provides a CAR-T cell. According to an embodiment of the present invention, the CAR-T cell includes a chimeric antigen receptor, wherein the chimeric antigen receptor includes: an extracellular region, the extracellular region includes a heavy chain variable region and a light chain variable region of a single-chain antibody, wherein the light chain variable region has an amino acid sequence as shown in SEQ ID NO: 57-59 or SEQ ID NO: 65-67, and the heavy chain variable region has an amino acid sequence as shown in SEQ ID NO: 60-64 or SEQ ID NO: 68-72; a transmembrane region, wherein the transmembrane region is connected to the extracellular region and embedded in the cell membrane of the T lymphocyte; and an intracellular region, wherein the intracellular region is connected to the transmembrane region. The CAR-T cell expressing this chimeric antigen receptor can kill tumor cells expressing Claudin18.2.

In the seventh aspect of the present invention, the present invention provides a pharmaceutical composition. According to an embodiment of the present invention, the pharmaceutical composition includes the aforementioned antibody, the nucleic acid molecule, the expression vector, the immune cell, the recombinant cell or the CAR-T cell. As mentioned above, the antibody, as well as the nucleic acid molecule, the expression vector, the immune cell, the recombinant cell or the CAR-T cell that can obtain the antibody after expression can not only specifically target and bind to Claudin18.2 with the same binding activity as the chimeric antibody, but also have a longer *in vivo* half-life. Therefore, the antibody or expressed antibody contained in the pharmaceutical composition according to the embodiment of the present invention can specifically target and bind to Claudin18.2, exhibit strong specificity, and exert a good targeting effect, thereby realizing the biological effects of other drugs in the pharmaceutical composition, such as the activity inhibition of Claudin18.2 molecules, the killing of cells expressing Claudin18.2 molecules, and the like. In addition, the pharmaceutical composition according to the embodiments of the present invention can play a diagnostic role, relying on the combination of diagnostic reagents and antibody capable of specifically targeting Claudin18.2 proposed in the first aspect of the present invention. This combination plays a role in the diagnosis of areas of the organism with the abnormal expression of Claudin18.2. For instance, by combining with diagnostic nuclides, nanomaterials, *etc.,* it enables the visualization of cells, tissues, and organs in the biological organism that abnormally expressing Claudin18.2, thereby assisting medical workers or scientific researchers to make more accurate judgments of the lesions.

In the eighth aspect of the present invention, the present invention provides the use of the aforementioned antibody, the aforementioned nucleic acid molecule, the aforementioned expression vector, immune cell, recombinant cell, CAR-T cell or the aforementioned pharmaceutical composition in the manufacture of of a medicament for the treatment or prevention of Claudin18.2 related diseases.

Or the present invention provides a method for treating or preventing Claudin18.2 related diseases, wherein the method comprises administering the aforementioned antibody, the aforementioned nucleic acid molecule, the aforementioned expression vector, immune cell, recombinant cell, CAR-T cell, or the aforementioned pharmaceutical composition.

Or the present invention provides the aforementioned antibody, the aforementioned nucleic acid molecule, the aforementioned expression vector, immune cell, recombinant cell, CAR-T cell, or the aforementioned pharmaceutical composition for preparing a drug for treating or preventing Claudin18.2 related diseases.

The Claudin18.2 related diseases include: at least one of gastric cancer, esophageal cancer, pancreatic cancer, lung cancer, colon cancer and rectal cancer.

As mentioned above, the antibody, as well as the nucleic acid molecule, the expression vector, the immune cell, the recombinant cell or the CAR-T cell that can obtain the antibody after expression can not only specifically target and bind to Claudin18.2, and have the same binding activity as the chimeric antibody, but also have a longer *in vivo* half-life. Therefore, drugs containing the above substances can also effectively target and bind to Claudin18.2, have a longer half-life *in vivo,* and can effectively kill cells expressing Claudin18.2 molecules.

In the ninth aspect of the present invention, the present invention provides a kit for detecting Claudin 18.2. According to an embodiment of the present invention, the kit includes any one of the antibody described above. The aforementioned Claudin18.2 antibodies can specifically target and bind to Claudin18.2. The kit according to the embodiment of the present invention can achieve specific detection of Claudin18.2. For example, when the antibody is bound with a fluorophore, a fluorescent detection device can be used to realize the localization or real-time detection of Claudin18.2; when the antibody is bound with biotin and other markers, the qualitative or quantitative detection of Claudin18.2 can be achieved by color development reagents; the antibody can also be combined with anti-antibody to achieve a sandwich or double-sandwich method, and then achieve signal step-by-step amplification to detect Claudin 18.2.

In the tenth aspect of the present invention, the present invention provides a use of the aforementioned antibody, the aforementioned nucleic acid molecule, the aforementioned expression vector or the aforementioned recombinant cell in the manufacture of a kit for detecting Claudin 18.2 or diagnosing Claudin 18.2 related diseases. According to the embodiment of the present invention, the kit can directly detect the expression level of Claudin18.2, such as high expression, low expression, and no expression, thereby realizing the diagnosis of the disease. It can also be combined with other diagnostic reagents to obtain the status of organisms, tissues, and cells, such as combined with diagnostic nuclides, to visualize the number of cells expressing Claudin18.2 in the body, the size and location of the tissue, *etc.*

**Description of the drawings**

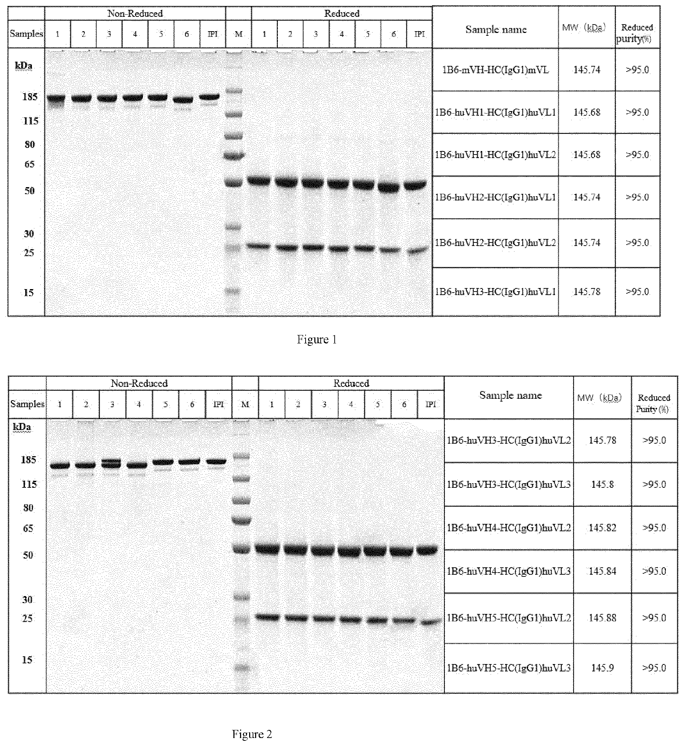

Figure 1 is a diagram showing the results of non-reduced and reduced SDS-PAGE identification of protein samples 1B6-mVH-HC (IgG1) mVL, 1B6-huVH1-HC (IgG1) huVL1, 1B6-huVH1-HC (IgG1) huVL2, 1B6-huVH2-HC (IgG1) huVL1, 1B6-huVH2-HC (IgG1) huVL2, and 1B6-huVH3-HC (IgG1) huVL1 obtained through a Protein A column according to an embodiment of the present invention, wherein Samples represents samples;

Figure 2 is a diagram showing the results of non-reduced and reduced SDS-PAGE identification of protein samples 1B6-huVH3-HC (IgG1) huVL2, 1B6-huVH3-HC (IgG1) huVL3, 1B6-huVH4-HC (IgG1) huVL2, 1B6-huVH4-HC (IgG1) huVL3, 1B6-huVH5-HC (IgG1) huVL2 and 1B6-huVH5-HC (IgG1) huVL3 obtained through a Protein A column according to an embodiment of the present invention, wherein Samples represents samples;

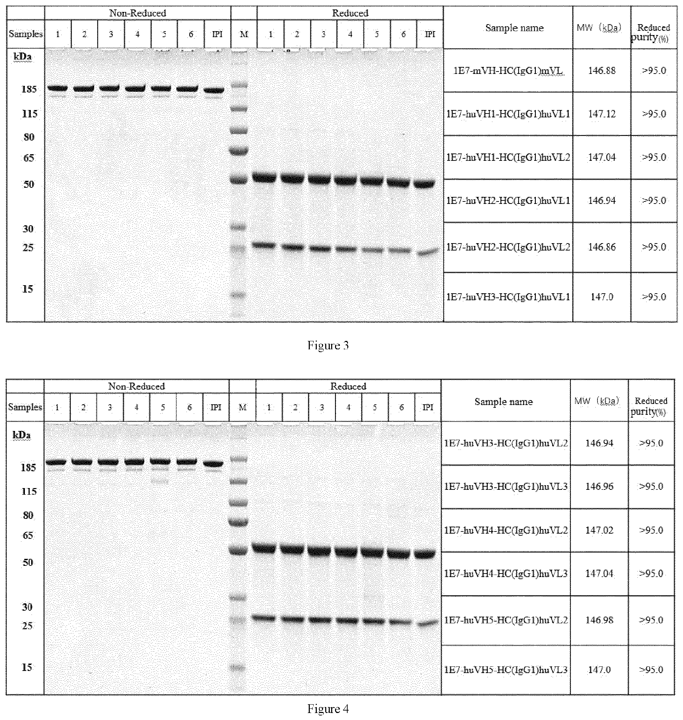

Figure 3 is a diagram showing the results of non-reduced and reduced SDS-PAGE identification of protein samples 1E7-mVH-HC (IgG1) mVL, 1E7-huVH1-HC (IgG1) huVL1, 1E7-huVH1-HC (IgG1) huVL2, 1E7-huVH2-HC (IgG1) huVL1, 1E7-huVH2-HC (IgG1) huVL2 and 1E7-huVH3-HC (IgG1) huVL1 obtained through a Protein A column according to an embodiment of the present invention, wherein Samples represents samples;

Figure 4 is a diagram showing the results of non-reduced and reduced SDS-PAGE identification of protein samples 1E7-huVH3-HC (IgG1) huVL2, 1E7-huVH3-HC (IgG1) huVL3, 1E7-huVH4-HC (IgG1) huVL2, 1E7-huVH4-HC (IgG1) huVL3, 1E7-huVH5-HC (IgG1) huVL2 and 1E7-huVH5-HC (IgG1) huVL3 obtained through a Protein A column according to an embodiment of the present invention, wherein Samples represents samples;

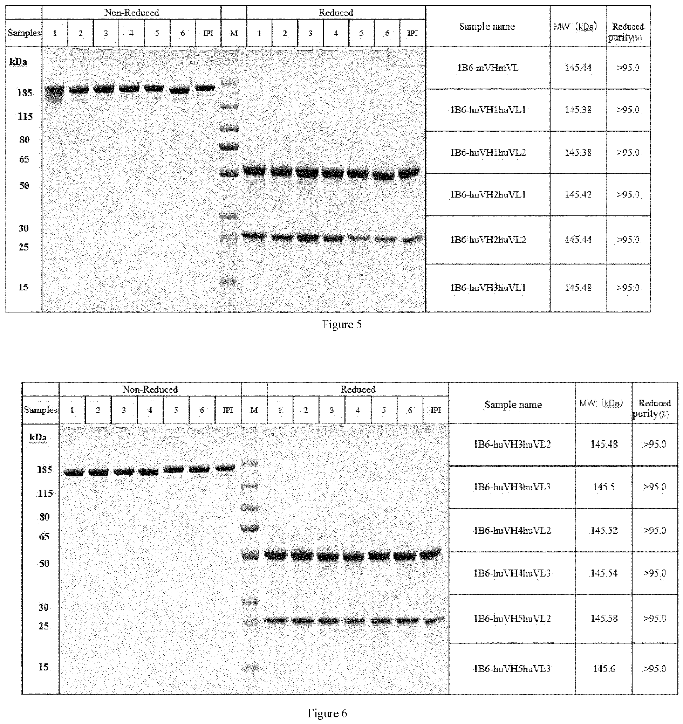

Figure 5 is a diagram showing the results of non-reduced and reduced SDS-PAGE identification of protein samples 1B6-mVHmVL, 1B6-huVH1huVL1, 1B6-huVH1huVL2, 1B6-huVH2huVL1, 1B6-huVH2huVL2 and 1B6-huVH3-

huVL1 obtained through a Protein A column according to an embodiment of the present invention, wherein Samples represents samples;

Figure 6 is a diagram showing the results of non-reduced and reduced SDS-PAGE identification of protein samples 1B6-mVHmVL, 1B6-huVH1huVL1, 1B6-huVH1huVL2, 1B6-huVH2huVL1, 1B6-huVH2huVL2 and 1B6-huVH3-huVL1 obtained through a Protein A column according to an embodiment of the present invention, wherein Samples represents samples;

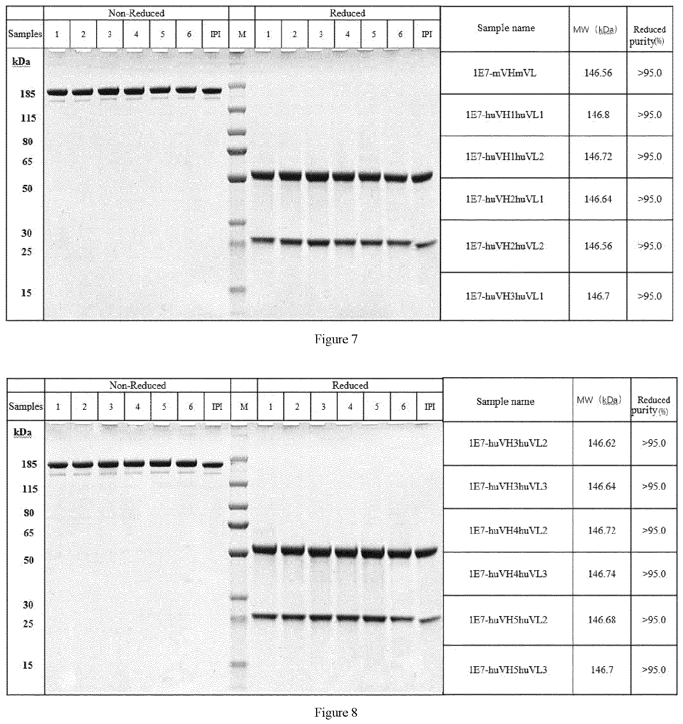

Figure 7 is a diagram showing the results of non-reduced and reduced SDS-PAGE identification of protein samples 1E7-mVHmVL, 1E7-huVH1huVL1, 1E7-huVH1huVL2, 1E7-huVH2huVL1, 1E7-huVH2huVL2 and 1E7-huVH3-huVL1 obtained through a Protein A column according to an embodiment of the present invention, wherein Samples represents samples;

Figure 8 is a diagram showing the results of non-reduced and reduced SDS-PAGE identification of protein samples 1E7-huVH3huVL2, 1E7-huVH3huVL3, 1E7-huVH4huVL2, 1E7-huVH4huVL3, 1E7-huVH5huVL2 and 1E7-huVH5-huVL3 obtained through a Protein A column according to an embodiment of the present invention, wherein Samples represents samples;

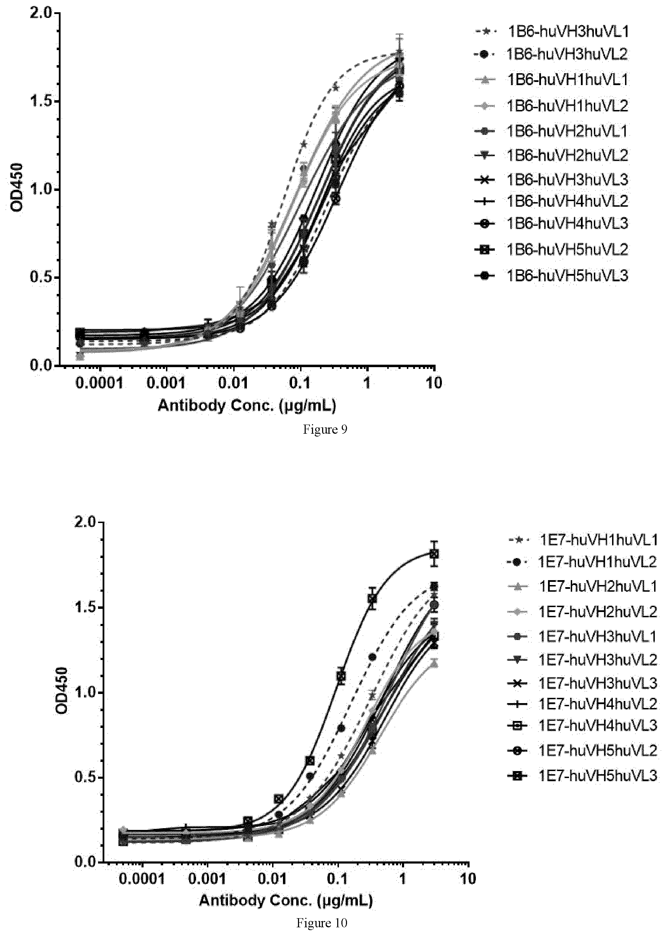

Figure 9 is an analysis diagram of ELISA test results of humanized antibodies 1B6-huVH1huVL1, 1B6-huVH1-huVL2,1B6-huVH2huVL1, 1B6-huVH2huVL2, 1B6-huVH3huVL1, 1B6-huVH3huVL2, 1B6-huVH3huVL3, 1B6-huVH4huVL2, 1B6-huVH4huVL3, 1B6-huVHShuVL2, and 1B6-huVH5huVL3 according to an embodiment of the present invention;

Figure 10 is an analysis diagram of ELISA test results of humanized antibodies 1E7-huVH1huVL1, 1E7-huVH1-huVL2, 1E7-huVH2huVL1, 1E7-huVH2huVL2, 1E7-huVH3huVL1 , 1E7-huVH3huVL2, 1E7-huVH3huVL3, 1E7-huVH4huVL2, 1E7-huVH4huVL3, 1E7-huVH5huVL2 and 1E7-huVH5huVL3 according to an embodiment of the present invention;

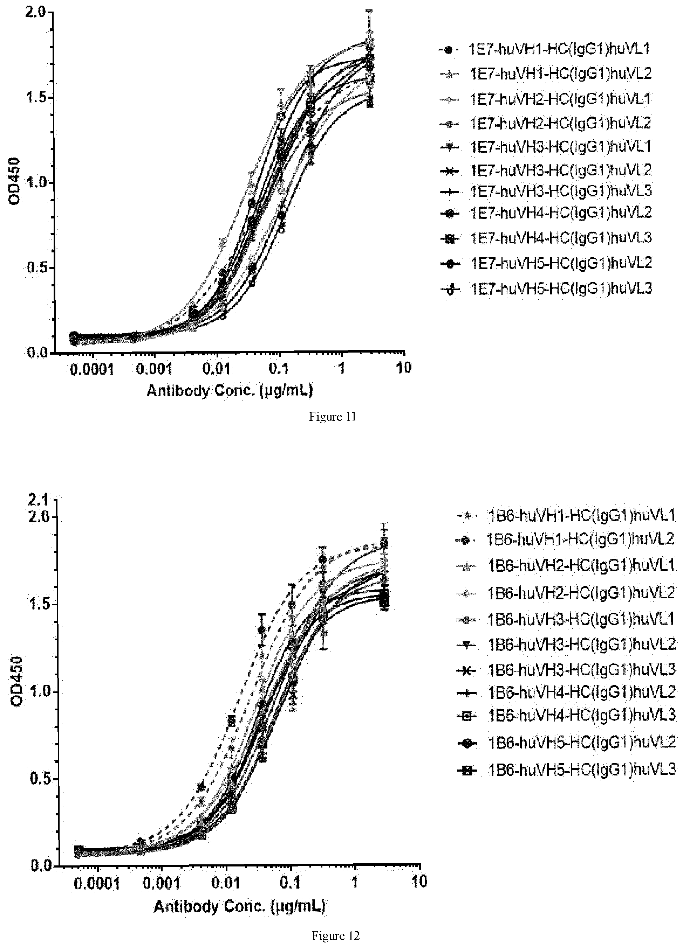

Figure 11 is an analysis diagram of ELISA test results of humanized antibodies 1E7-huVH1-HC (IgG1) huVL1, 1E7-huVH1-HC (IgG1) huVL2, 1E7-huVH2-HC (IgG1) huVL1, 1E7-huVH2-HC (IgG1) huVL2, 1E7-huVH3-HC (IgG1) huVL1, 1E7-huVH3-HC (IgG1) huVL2, 1E7-huVH3-HC (IgG1) huVL3, 1E7-huVH4-HC (IgG1) huVL2, 1E7-huVH4-HC (IgG1) huVL3, 1E7-huVH5-HC (IgG1) huVL2 and 1E7-huVH5-HC (IgG1) huVL3 according to an embodiment of the present invention;

Figure 12 is an analysis diagram of ELISA test results of humanized antibodies 1B6-huVH1-HC (IgG1) huVL1, 1B6-huVH1-HC (IgG1) huVL2, 1B6-huVH2-HC (IgG1) huVL1, 1B6-huVH2-HC (IgG1) huVL2, 1B6-huVH3-HC (IgG1) huVL1, 1B6-huVH3-HC (IgG1) huVL2, 1B6-huVH3-HC (IgG1) huVL3, 1B6-huVH4-HC (IgG1) huVL2, 1B6-huVH4-HC (IgG1) huVL3, 1B6-huVH5-HC (IgG1) huVL2 and 1B6-huVH5-HC (IgG1) huVL3 according to an embodiment of the present invention;

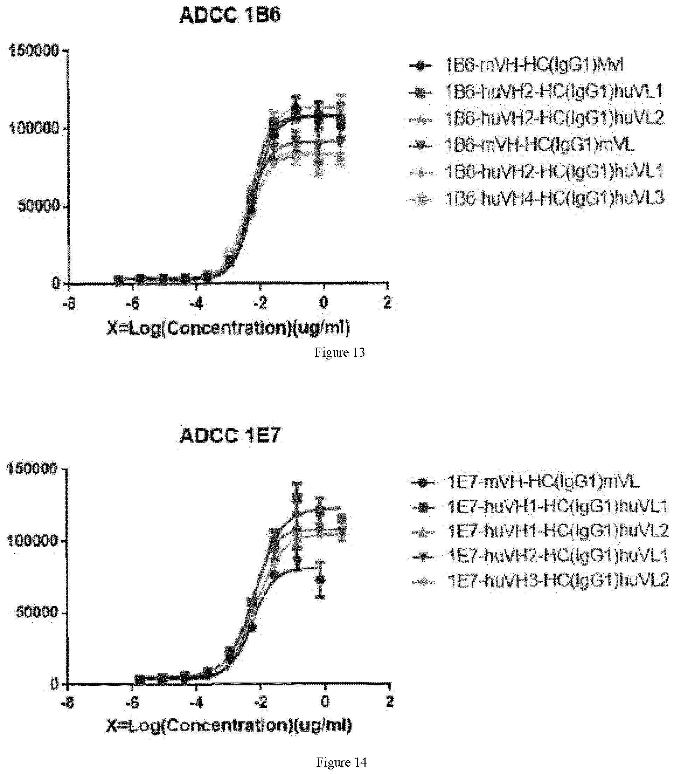

Figure 13 is an analysis diagram of the ADCC activity evaluation results of the chimeric antibody 1B6-mVH-HC (IgG1) mVL, the humanized antibodies 1B6-huVH2-HC (IgG1) huVL1, 1B6-huVH2-HC (IgG1) huVL2, and 1B6-huVH4-HC (IgG1) huVL3 according to an embodiment of the present invention, wherein the abscissa represents the Log value of the antibody concentration ($\mu$g/mL), and the ordinate represents the intensity of the luminescent signal;

Figure 14 is an analysis diagram of the ADCC activity evaluation results of the chimeric antibody 1E7-mVH-HC (IgG1) mVL, the humanized antibodies 1E7-huVH1-HC (IgG1) huVL1, 1E7-huVH1-HC (IgG1) huVL2, 1E7-huVH2-HC (IgG1) huVL1, 1E7-huVH3-HC (IgG1) huVL2 according to an embodiment of the present invention;

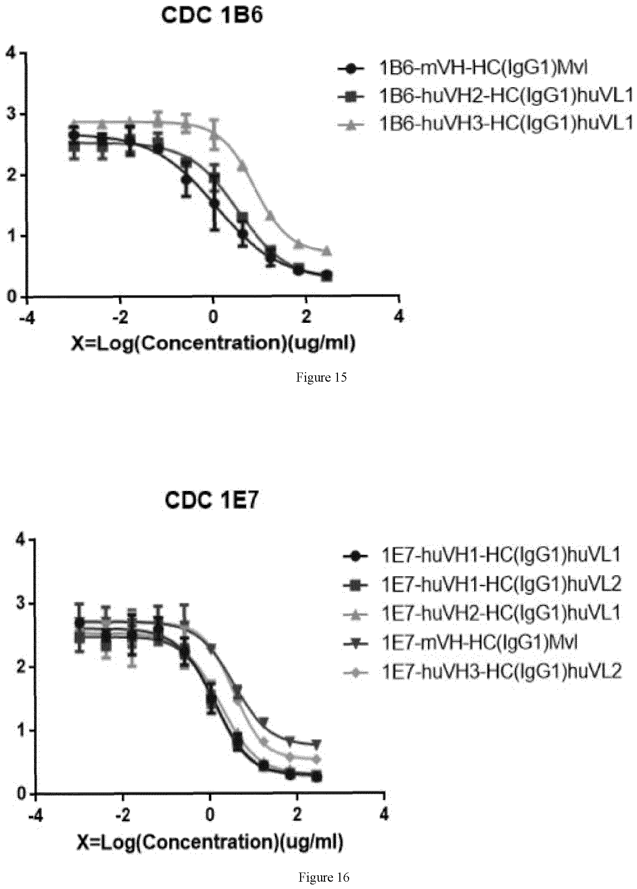

Figure 15 is an analysis diagram of the CDC activity evaluation results of the chimeric antibody 1B6-mVH-HC (IgG1) mVL, the humanized antibody 1B6-huVH2-HC (IgG1) huVL1 and 1B6-huVH3-HC (IgG1) huVL1 according to an embodiment of the present invention, wherein the abscissa represents the Log value of the antibody concentration ($\mu$g/mL), and the ordinate represents OD450;

Figure 16 is an analysis diagram of the CDC activity evaluation results of the chimeric antibody 1E7-mVH-HC (IgG1) mVL, the humanized antibody 1E7-huVH1-HC (IgG1) huVL1, 1E7-huVH1-HC (IgG1) huVL2, 1E7-huVH2-HC (IgG1) huVL1 and 1E7-huVH3-HC (IgG1) huVL2 according to an embodiment of the present invention, wherein the abscissa represents the Log value of the antibody concentration ($\mu$g/mL), and the ordinate represents OD450;

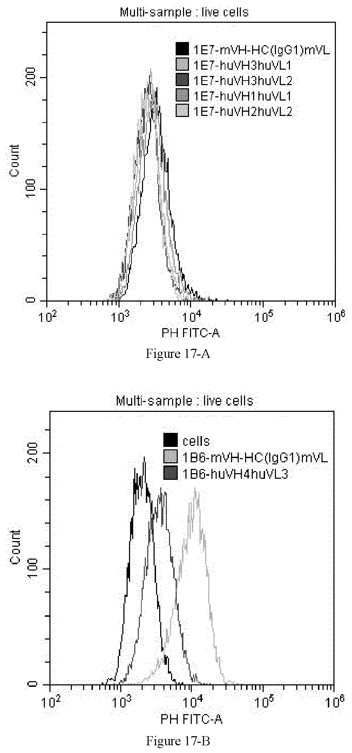

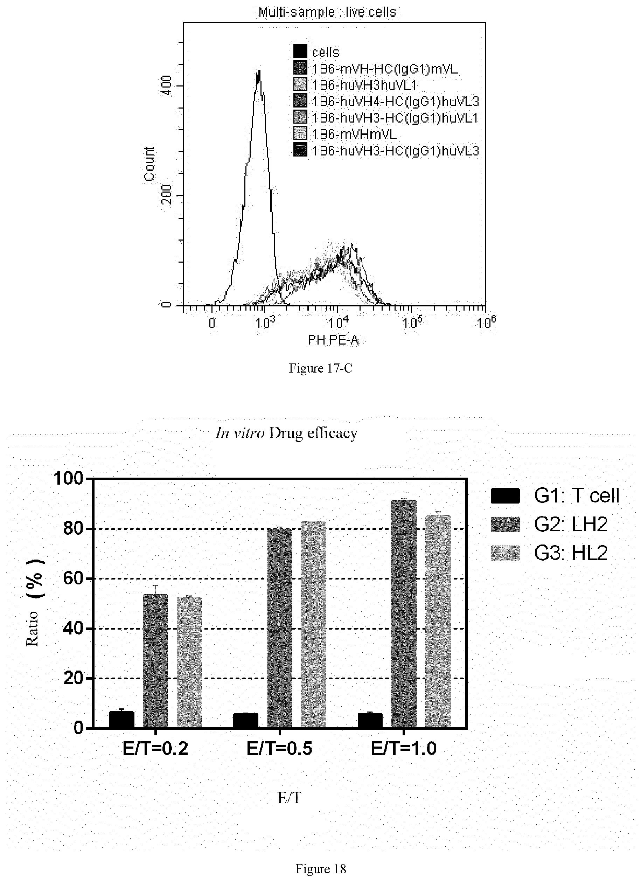

Figure 17 is a diagram showing the internalization detection results of the chimeric antibody according to an embodiment of the present invention, wherein:

17-A represents the internalization detection diagram of the antibodies 1E7-mVH-HC (IgG1) mVL, 1E7-huVH3-huVL1, 1E7-huVH3huVL2, 1E7-huVH1huVL1 and 1E7-huVH2huVL2;

17-B represents the internalization detection diagram of the antibodies 1B6-mVH- HC (IgG1) mVL and 1B6-huVH4huVL3;

17-C represents the internalization detection diagram of the antibodies 1B6-mVH- HC (IgG1) mVL, 1B6-huVH3huVL1, 1B6-huVH4-HC (IgG1) huVL3, 1B6-huVH3-HC (IgG1) huVL1, 1B6-mVHmVL and 1B6-huVH3-HC (IgG1) huVL3;

Figure 18 is a study on the *in vitro* effects of humanized claudin18.2 antibody CAR-T;
Figure 19 is a study on the *in vivo* effects of humanized claudin18.2 antibody CAR-T.

**EXAMPLES**

The embodiments of the present invention are described in detail below. Examples of the embodiments are shown in the accompanying drawings, in which the same or similar reference numerals indicate the same or similar elements or elements with the same or similar functions. The embodiments described below with reference to the accompanying drawings are exemplary and are intended to explain the present invention and should not be construed as limiting the present invention.

In the process of describing the present invention, the relevant terms in this document are explained and illustrated. These explanations and illustrations are only for the convenience of understanding the scheme and cannot be regarded as limitations on the protection scheme of the present invention.

**Antibody**

As used herein, the term "antibody" is an immunoglobulin molecule capable of binding to a specific antigen. It consists of two light chains with lighter molecular weight and two heavy chains with heavier molecular weight. The heavy (H) and light (L) chains are linked by disulfide bonds to form a tetrapeptide chain molecule. Among them, the amino acid sequence of the amino terminal (N-terminal) of the peptide chain varies greatly, which is called the variable region (V region). The carboxyl terminal (C-terminal) is relatively stable with little change, which is called the constant region (C region). The V regions of the L and H chains are referred to as VL and VH, respectively.

Some regions in the variable region have a higher degree of change in amino acid composition and arrangement order. They are called hypervariable regions (HVR). Hypervariable regions are where antigens and antibodies bind, so they are also called complementarity-determining region (CDR). There are three CDRs on both the heavy and light chain variable regions.

It should be noted that "immunogenicity" refers to the ability to induce an immune response, that is, the ability of an antigen to stimulate specific immune cells, causing them to activate, proliferate, and differentiate, ultimately producing immune effector substances such as antibodies and sensitized lymphocytes. The inventors of the present application further humanized (humanized modification) the screened murine monoclonal antibody sequences to obtain humanized antibodies, which not only retain the affinity and specificity of the parent mouse monoclonal antibody, but also greatly reduce its immunogenicity, improve its safety, and prolong its half-life.

In some embodiments, the invention provides an antibody or antigen-binding fragment capable of specifically recognizing Claudin18.2, wherein the antibody comprises at least one CDR sequence selected from the following or an amino acid sequence having at least 90% identity with it: the CDR sequence of light chain variable region: $X_1$SSQSLFNSGNQKNYLT (SEQ ID NO: 1), WASTRES (SEQ ID NO: 2), QNDYSYPLT (SEQ ID NO: 3), $X_4$SSQSLLNSGNQKNYLT (SEQ ID NO: 7), WASTRES (SEQ ID NO: 8) and QNDYSYPFT (SEQ ID NO: 9); the CDR sequence of heavy chain variable region: DYNMH (SEQ ID NO: 4), YINPNNGGTSYX$_2$QKFX$_3$G (SEQ ID NO: 5), TRYLAV (SEQ ID NO: 6), SYWMH (SEQ ID NO: 10), MIHPNSGSTNYX$_5$X$_6$KFX$_7$X$_8$ (SEQ ID No: 11) and RYYGSISPDY (SEQ ID NO: 12); wherein, $X_1$ is K or R, $X_2$ is N or S, $X_3$ is K or Q, $X_4$ is K or R, $X_5$ is N or A, $X_6$ is E or Q, $X_7$ is K or Q, $X_8$ is S or G, the antibody or antigen-binding fragment thereof has a humanization modification. It should be noted that the "humanization modification" described in the present application refers to changes in amino acids that can reduce the immunogenicity of the antibody or antigen-binding fragment thereof, including amino acid mutations, insertions, deletions, and additions of chemical groups. The above-mentioned antibody according to the embodiment of the present invention has higher ADCC activity and CDC activity, smaller EC50 and IC50 values, can not only specifically target and bind to Claudin18.2, and has the same binding activity as the chimeric antibody, but also has a longer half-life *in vivo.* In these embodiments, the antibodies or antigen-binding fragments provided by the present invention have conservative amino acid substitutions. "Antigen-binding fragment" refers to an antibody fragment that retains the ability to specifically bind to an antigen (ROR2). "Conservative amino acid substitution" refers to the replacement of an amino acid with a residue that is biologically, chemically, or structurally similar to another amino acid. Biologically similar means that the substitution does not destroy the biological activity of the Claudin18.2 antibody or the Claudin18.2 antigen. Structural similarity refers to side chains with similar lengths of amino acids, such as alanine, glycine, or serine, or side chains of similar size. Chemical similarity means that amino acids have the same charge or are both hydrophilic or hydrophobic. For example, the hydrophobic residues isoleucine, valine, leucine or methionine are substituted with each other. Alternatively, polar amino acids can be used. For example, lysine is substituted with arginine, aspartic acid is substituted with glutamic acid, asparagine is substituted with glutamine, threonine is substituted with serine, *etc.*

The "1B6-mVH-HC (IgG1) mVL" described herein can be understood as a chimeric antibody comprising a light chain and a heavy chain, wherein the variable regions of the light chain and the heavy chain are both the variable regions of the murine antibody 1B6, and the constant region is from human IgG1; the "1B6-mVHmVL" described herein can be understood as a chimeric antibody comprising a light chain and a heavy chain, wherein the variable regions of the light chain and the heavy chain are both the variable regions of the murine antibody 1B6, and the constant region is from human IgG4; the "1E7-mVH-HC (IgG1) mVL" described herein can be understood as a chimeric antibody comprising a light chain and a heavy chain, wherein the variable regions of the light chain and the heavy chain are both the variable regions of the murine antibody 1E7, and the heavy chain constant region is from human IgG1; the "1E7-mVHmVL" described herein can be understood as a chimeric antibody comprising a light chain and a heavy chain, wherein the variable regions of the light chain and the heavy chain are both the variable regions of the murine antibody 1E7, and the constant region is from human IgG4; "1B6" and "1E7" can be understood as double-chain antibodies formed by VH-HC-VL-LC.

The naming method of humanized antibodies in this application is "name of murine antibody-name of heavy chain variable region-heavy chain constant region (name of antibody)-light chain variable region", wherein the light chain constant region is defaulted to the Kappa chain constant region and is not released with a label. When the heavy chain constant region is derived from a human IgG1 antibody, the heavy chain constant region is expressed as HC (IgG1). When the heavy chain constant region is derived from a human IgG4 antibody, the heavy chain constant region is not released. For example, the human antibody "1B6-huVH1-HC (IgG1) huVL1" can be understood as a humanized antibody obtained by replacing the framework region and constant region of the murine antibody 1B6 with the framework region and constant region of the human IgG1 antibody. Preferably, the amino acid sequence of the light chain variable region of the antibody can be shown in SEQ ID NO: 57, the amino acid sequence of the heavy chain variable region of the antibody can be shown in SEQ ID NO: 60, the light chain amino acid sequence of the antibody can be shown in SEQ ID NO: 76, the heavy chain amino acid sequence of the antibody can be shown in SEQ ID NO: 79; the human antibody "1B6-huVH1huVL1" can be understood as a humanized antibody obtained by replacing the framework region and constant region of the murine antibody 1B6 with the framework region and constant region of the human IgG4 antibody. Preferably, the amino acid sequence of the light chain variable region of the antibody can be shown in SEQ ID NO: 57, the amino acid sequence of the heavy chain variable region of the antibody can be shown in SEQ ID NO: 60, the light chain amino acid sequence of the antibody can be shown in SEQ ID NO: 76, the heavy chain amino acid sequence of the antibody can be shown in SEQ ID NO: 84;

the human antibody "1E7-huVH1-HC (IgG1) huVL1" can be understood as a humanized antibody obtained by replacing the framework region and constant region of the murine antibody 1E7 with the framework region and constant region of the human IgG1 antibody. Preferably, the amino acid sequence of the light chain variable region of the antibody can be shown in SEQ ID NO: 65, the amino acid sequence of the heavy chain variable region of the antibody can be shown in SEQ ID NO: 68, the light chain amino acid sequence of the antibody can be shown in SEQ ID NO: 89, the heavy chain amino acid sequence of the antibody can be shown in SEQ ID NO: 92; the human antibody "1E7-huVH1huVL1" can be understood as a humanized antibody obtained by replacing the framework region and constant region of the murine antibody 1E7 with the framework region and constant region of the human IgG4 antibody. Preferably, the amino acid sequence of the light chain variable region of the antibody can be shown in SEQ ID NO: 65, the amino acid sequence of the heavy chain variable region of the antibody can be shown in SEQ ID NO: 68, the light chain amino acid sequence of the antibody can be shown in SEQ ID NO: 89, the heavy chain amino acid sequence of the antibody can be shown in SEQ ID NO: 97.

According to some specific embodiments of the present invention, the aforementioned antibody or antigen-binding fragment may further comprise at least one of the following additional technical features:

According to some specific embodiments of the present invention, the antibody comprises: the light chain variable region CDR1, CDR2, and CDR3 having sequences shown in SEQ ID NO: 1, 2 and 3 respectively or having at least 90% identity with SEQ ID NO: 1, 2 and 3; or the light chain variable region CDR1, CDR2, and CDR3 having sequences shown in SEQ ID NO: 7, 8 and 9 respectively or having at least 90% identity with SEQ ID NO: 7, 8 and 9.

According to some specific embodiments of the present invention, the antibody comprises: the heavy chain variable region CDR1, CDR2, and CDR3 having sequences shown in SEQ ID NO: 4, 5 and 6 respectively or having at least 90% identity with SEQ ID NO: 4, 5 and 6; or the heavy chain variable region CDR1, CDR2, and CDR3 having sequences shown in SEQ ID NO: 10, 11 and 12 respectively or having at least 90% identity with SEQ ID NO: 10, 11 and 12.

According to some specific embodiments of the present invention, the antibody comprises at least one of a heavy chain framework region sequence and a light chain framework region sequence, at least a part of at least one of the heavy chain framework region sequence and the light chain framework region sequence is derived from at least one of a human antibody or a mutant thereof. According to some specific embodiments of the present invention, the inventors performed humanized modification on the light chain framework region and/or the heavy chain framework region of the murine monoclonal antibody.

According to some specific embodiments of the present invention, the antibody comprises: the light chain framework region FRL1, FRL2, FRL3, and FRL4 having sequences shown in SEQ ID NO: 13-16 respectively, or the light chain framework region FRL1, FRL2, FRL3, and FRL4 having sequences shown in SEQ ID NO: 21-24 respectively,

DIX$_9$MTQSPSSLX$_{10}$X$_{11}$X$_{12}$X$_{13}$GX$_{14}$X$_{15}$VTX$_{16}$SX$_{17}$C (SEQ ID NO: 13),
WYQQKPGX$_{18}$X$_{19}$PKLLIY (SEQ ID NO: 14),
GVPX$_{20}$DRFX$_{21}$GSGSGTDFTLTISSX$_{22}$QX$_{23}$EDX$_{24}$AX$_{25}$YX$_{26}$C (SEQ ID NO: 15),
FGX$_{27}$GTKLEX$_{28}$K (SEQ ID NO: 16);
DIX$_{53}$MTQSPSSLSX$_{54}$X$_{55}$AX$_{56}$GX$_{57}$X$_{58}$VTX$_{59}$X$_{60}$C (SEQ ID NO: 21),
WYQQKPGX$_{61}$X$_{62}$PKLLIY (SEQ ID NO: 22),
GVPX$_{63}$RFX$_{64}$GSGSGTDFTLTISSX$_{65}$QX$_{66}$EDX$_{67}$AX$_{68}$YYC (SEQ ID NO: 23),
FGX$_{69}$GTKLEIK (SEQ ID NO: 24),
wherein, X$_9$ is V or Q, X$_{10}$ is S or T, X$_{11}$ is V or A, X$_{12}$ is T or S, X$_{13}$ is A or V, X$_{14}$ is E or D, X$_{15}$ is K or R, X$_{16}$ is M or I, X$_{17}$ is S or T, X$_{18}$ is Q or K, X$_{19}$ is P or A, X$_{20}$ is D or S, X$_{21}$ is T or S, X$_{22}$ is V or L, X$_{23}$ is A or P, X$_{24}$ is L or F, X$_{25}$ is V or T, X$_{26}$ is F or Y, X$_{27}$ is A or G, X$_{28}$ is L or I, X$_{53}$ is V or Q, X$_{54}$ is V or A, X$_{55}$ is T or S, X$_{56}$ is A or V, X$_{57}$ is E or D, X$_{58}$ is K or R, X$_{59}$ is M or I, X$_{60}$ is S or T, X$_{61}$ is Q or K, X$_{62}$ is P or A, X$_{63}$ is D or S, X$_{64}$ is T or S, X$_{65}$ is V or L, X$_{66}$ is A or P, X$_{67}$ is L or F, X$_{68}$ is V or T, X$_{69}$ is S or G. The condition is that when the antibody comprises: the light chain variable region CDR1, CDR2, and CDR3 sequences shown in KSSQSLFNSGNQKNYLT (SEQ ID NO: 29), WASTRES (SEQ ID NO: 30), and QNDYSYPLT (SEQ ID NO: 31), respectively, X$_9$ is V, X$_{10}$ is T, X$_{11}$ is V, X$_{12}$ is T, X$_{13}$ is A, X$_{14}$ is E, X$_{15}$ is K, X$_{16}$ is M, X$_{17}$ is S, X$_{18}$ is Q, X$_{19}$ is P, X$_{20}$ is D, X$_{21}$ is T, X$_{22}$ is V, X$_{23}$ is A, X$_{24}$ is L, X$_{25}$ is V, X$_{26}$ is F, X$_{27}$ is A, and X$_{25}$ is L are not established at the same time; when the antibody comprises: the light chain variable region CDR1, CDR2, and CDR3 sequences shown in KSSQSLLNSGNQKNYLT (SEQ ID NO: 35), WASTRES (SEQ ID NO: 36), QNDYSYPFT (SEQ ID NO: 37), respectively, X$_{53}$ is V, X$_{54}$ is V, X$_{55}$ is T, X$_{56}$ is A, X$_{57}$ is E, X$_{58}$ is K, X$_{59}$ is M, X$_{60}$ is S, X$_{61}$ is Q, X$_{62}$ is P, X$_{63}$ is D, X$_{64}$ is T, X$_{65}$ is V, X$_{66}$ is A, X$_{67}$ is L, X$_{68}$ is V, X$_{69}$ is S, which are not established at the same time.

According to some specific embodiments of the present invention, the antibody comprises: the light chain framework region FRL1, FRL2, FRL3, and FRL4 having sequences shown in SEQ ID NO: 41-44 respectively, or the light chain framework region FRL1, FRL2, FRL3, and FRL4 having sequences shown in SEQ ID NO: 49-52 respectively,

DIX$_{90}$MTQSPSSLSX$_{91}$TAGEKVTX$_{92}$SC (SEQ ID NO: 41),
WYQQKPGQPPKLLIY (SEQ ID NO: 42),
GVPX$_{93}$DRFTGSGSGTDFTLTISSX$_{94}$QAEDLAVYX$_{95}$C (SEQ ID NO: 43),
FGAGTKLELK (SEQ ID NO: 44); or
the light chain framework region FRL1, FRL2, FRL3, and FRL4 having sequences shown in SEQ ID NO: 49-52 respectively,
DIX$_{107}$MTQSPSSLSX$_{108}$TAGEKVTX$_{109}$SC (SEQ ID NO: 49),
WYQQKPGX$_{110}$X$_{111}$PKLLIY (SEQ ID NO: 50),
GVPX$_{112}$RFTGSGSGTDFTLTISSX$_{113}$QAEDLAVYYC (SEQ ID NO: 51),
FGSGTKLEIK (SEQ ID NO: 52), wherein, X$_{90}$ is V or Q, X$_{91}$ is V or A, X$_{92}$ is M or I, X$_{93}$ is D or S, X$_{94}$ is V or L, X$_{95}$ is F or Y, X$_{107}$ is V or Q, X$_{108}$ is V or A, X$_{109}$ is M or I, X$_{110}$ is Q or K, X$_{111}$ is P or A, X$_{112}$ is D or S, X$_{113}$ is V or L.

According to some specific embodiments of the present invention, the antibody comprises: the heavy chain framework region FRH1, FRH2, FRH3, and FRH4 having sequences shown in SEQ ID NO: 17-20 respectively, or the heavy chain framework region FRH1, FRH2, FRH3, and FRH4 having sequences shown in SEQ ID NO: 25-28 respectively,

X$_{29}$VQLX$_{30}$QSGX$_{31}$EX$_{32}$X$_{33}$X$_{34}$PGASVKX$_{35}$SCKASGYTFT (SEQ ID NO: 17),
WVX$_{36}$QX$_{37}$X$_{38}$GX$_{39}$X$_{40}$LEWX$_{41}$G (SEQ ID NO: 18),
X$_{42}$X$_{43}$TX$_{44}$TX$_{45}$X$_{46}$X$_{47}$SX$_{48}$STAYWLX$_{49}$SLX$_{50}$SEDX$_{51}$AVYYCVT (SEQ ID NO: 19),
WGX$_{52}$GTTVTVSS (SEQ ID NO: 20);
QVQLX$_{70}$QX$_{71}$GX$_{72}$EX$_{73}$X$_{74}$KPGASVKX$_{75}$SCKASGYTFT (SEQ ID NO: 25),
WVX$_{76}$QX$_{77}$PGQGLEWX$_{78}$G (SEQ ID NO: 26),
X$_{79}$X$_{80}$X$_{81}$TX$_{82}$TVDX$_{83}$SX$_{84}$STX$_{85}$YMX$_{86}$LSSLX$_{87}$SEDX$_{88}$AVYYCAR (SEQ ID NO: 27),
WGQGTTX$_{89}$TVSS (SEQ ID NO: 28), wherein, X$_{29}$ is E or Q, X$_{30}$ is Q or V, X$_{31}$ is P or A, X$_{32}$ is L or V, X$_{33}$ is V or K, X$_{34}$ is R or K, X$_{35}$ is M or V, X$_{36}$ is K or R, X$_{37}$ is S or A, X$_{38}$ is H or P, X$_{39}$ is K or Q, X$_{40}$ is S or R, X$_{41}$ is I or M, X$_{42}$ is K or R, X$_{43}$ is A or V, X$_{44}$ is L or I, X$_{45}$ is V or R, X$_{46}$ is N or D, X$_{47}$ is K or T, X$_{48}$ is S or A, X$_{49}$ is R or S, X$_{50}$ is T or R, X$_{51}$ is S or T, X$_{52}$ is T or Q, X$_{70}$ is Q or V, X$_{71}$ is P or S, X$_{72}$ is S or A, X$_{73}$ is L or V, X$_{74}$ is V or K, X$_{75}$ is L or V, X$_{76}$ is K or R, X$_{77}$ is R or A, X$_{78}$ is I or M, X$_{79}$ is S or G, X$_{80}$ is R or K, X$_{81}$ is A or V, X$_{82}$ is L or M, X$_{83}$ is K or T, X$_{84}$ is S or T, X$_{85}$ is A or V, X$_{86}$ is Q or E, X$_{87}$ is T or R, X$_{88}$ is S or T, X$_{89}$ is L or V; the condition is that when the antibody comprises: the heavy chain variable region CDR1, CDR2, and CDR3 sequences shown in DYNMH (SEQ ID NO: 32), YINPNNGGTSYNQKFKG (SEQ ID NO: 33), TRYLAV (SEQ ID NO: 34), respectively, X$_{29}$ is E, X$_{30}$ is Q, X$_{31}$ is P, X$_{32}$ is L, X$_{33}$ is V, X$_{34}$ is R, X$_{35}$ is M, X$_{36}$ is K, X$_{37}$ is S, X$_{38}$ is H, X$_{39}$ is K, X$_{40}$ is S, X$_{41}$ is I, X$_{42}$ is K, X$_{43}$ is A, X$_{44}$ is L, X$_{45}$ is V, X$_{46}$ is N, X$_{47}$ is K, X$_{48}$ is S, X$_{49}$ is R, X$_{50}$ is T, X$_{51}$ is

S, $X_{52}$ is T, which are not established at the same time; when the antibody comprises: the heavy chain variable region CDR1, CDR2, and CDR3 sequences shown in SYWMH (SEQ ID NO: 38), MIHPNSGSTNYNEKFKS (SEQ ID NO: 39), RYYGSISPDY (SEQ ID NO: 40), respectively, $X_{70}$ is Q, $X_{71}$ is P, $X_{72}$ is S, $X_{73}$ is L, $X_{74}$ is V, $X_{75}$ is L, $X_{76}$ is K, $X_{77}$ is R, $X_{78}$ is I, $X_{79}$ is S, $X_{80}$ is K, $X_{81}$ is A, $X_{82}$ is L, $X_{83}$ is K, $X_{84}$ is S, $X_{ss}$ is A, $X_{86}$ is Q, $X_{87}$ is T, $X_{ss}$ is S, $X_{59}$ is L, which are not established at the same time.

According to some specific embodiments of the present invention, the antibody comprises: the heavy chain framework region FRH1, FRH2, FRH3, and FRH4 having sequences shown in SEQ ID NO: 45-48 respectively, or the heavy chain framework region FRH1, FRH2, FRH3, and FRH4 having sequences shown in SEQ ID NO: 53-56 respectively,

$X_{96}$VQLQQSGPELVRPGASVKX$_{97}$SCKASGYTFT (SEQ ID NO: 45),
WVKQSHGX$_{98}$X$_{99}$LEWX$_{100}$G (SEQ ID NO: 46),
$X_{101}$X$_{102}$TX$_{103}$TVX$_{104}$X$_{105}$SX$_{106}$STAYMELRSLTSEDSAVYYCVT (SEQ ID NO: 47),
WGTGTTVTVSS (SEQ ID NO: 48),
QVQLQQPGSELVKPGASVKX$_{114}$SCKASGYTFT (SEQ ID NO: 53),
WVKQX$_{115}$PGQGLEWX$_{116}$G (SEQ ID NO: 54),
$X_{117}$X$_{118}$TX$_{119}$TVDX$_{120}$SSSTX$_{121}$YMQLSSLTSEDSAVYYCAR (SEQ ID NO: 55),
WGQGTTLTVSS (SEQ ID NO: 56), wherein, $X_{96}$ is E or Q, $X_{97}$ is M or V, $X_{98}$ is K or Q, $X_{99}$ is S or R, $X_{100}$ is I or M, $X_{101}$ is K or R, $X_{102}$ is A or V, $X_{103}$ is L or I, $X_{104}$ is N or D, $X_{105}$ is K or T, $X_{106}$ is S or A, $X_{114}$ is L or V, $X_{115}$ is R or A, $X_{116}$ is I or M, $X_{117}$ is R or K, $X_{118}$ is A or V, $X_{119}$ is L or M, $X_{120}$ is K or T, $X_{121}$ is A or V.

According to some specific embodiments of the present invention, the antibody has a light chain variable region with an amino acid sequence shown in SEQ ID NO: 57-59 or SEQ ID NO: 65-67.

1B6 humanized antibody light chain variable region 1 (1B6-huVL1):

DIVMTQSPSSLSVSVGDRVTMTCKSSQSLFNSGNQKNYLTWYQQKPGKAPKLLIYW
ASTRESGVPDRFSGSGSGTDFTLTISSVQPEDFATYFCQNDYSYPLTFGGGTKLEIK (SEQ ID NO: 57).

1B6 humanized antibody light chain variable region 2 (1B6-huVL2):

DIQMTQSPSSLSASVGDRVTMTCKSSQSLFNSGNQKNYLTWYQQKPGKAPKLLIYW
ASTRESGVPSRFSGSGSGTDFTLTISSLQPEDFATYYCQNDYSYPLTFGGGTKLEIK (SEQ ID NO: 58).

1B6 humanized antibody light chain variable region 3 (1B6-huVL3):

DIQMTQSPSSLSASVGDRVTITCRSSQSLFNSGNQKNYLTWYQQKPGKAPKLLIYWA
STRESGVPSRFSGSGSGTDFTLTISSLQPEDFATYYCQNDYSYPLTFGGGTKLEIK (SEQ ID NO: 59).

1E7 humanized antibody light chain variable region 1 (1E7-huVL1):

DIVMTQSPSSLSVSVGDRVTMTCKSSQSLLNSGNQKNYLTWYQQKPGQPPKLLIYW
ASTRESGVPDRFSGSGSGTDFTLTISSVQPEDFATYYCQNDYSYPFTFGGGTKLEIK (SEQ ID NO: 65).

1E7 humanized antibody light chain variable region 2 (1E7-huVL2):

DIQMTQSPSSLSASVGDRVTMTCKSSQSLLNSGNQKNYLTWYQQKPGKAPKLLIYW

ASTRESGVPSRFSGSGSGTDFTLTISSLQPEDFATYYCQNDYSYPFTFGGGTKLEIK　(SEQ

ID NO: 66).

1E7 humanized antibody light chain variable region 3 (1E7-huVL3):

DIQMTQSPSSLSASVGDRVTITCRSSQSLLNSGNQKNYLTWYQQKPGKAPKLLIYWA

STRESGVPSRFSGSGSGTDFTLTISSLQPEDFATYYCQNDYSYPFTFGGGTKLEIK (SEQ ID

NO: 67).

According to some specific embodiments of the present invention, the antibody has a heavy chain variable region with an amino acid sequence shown in SEQ ID NO: 60-64 or SEQ ID NO: 68-72.

1B6 humanized antibody heavy chain variable region 1 (1B6-huVH1):

EVQLVQSGAEVKKPGASVKMSCKASGYTFTDYNMHWVRQAPGKSLEWIGYINPN

NGGTSYNQKFKGKATLTVNKSSSTAYMELSSLRSEDTAVYYCVTTRYLAVWGQGTTVTV

SS (SEQ ID NO: 60).

1B6 humanized antibody heavy chain variable region 2 (1B6-huVH2):

QVQLVQSGAEVKKPGASVKMSCKASGYTFTDYNMHWVRQAPGQRLEWIGYINPN

NGGTSYNQKFKGKATLTVDTSASTAYMELSSLRSEDTAVYYCVTTRYLAVWGQGTTVTV

SS (SEQ ID NO: 61).

1B6 humanized antibody heavy chain variable region 3 (1B6-huVH3):

QVQLVQSGAEVKKPGASVKVSCKASGYTFTDYNMHWVRQAPGQRLEWIGYINPN

NGGTSYNQKFKGRVTITVDTSASTAYMELSSLRSEDTAVYYCVTTRYLAVWGQGTTVTV

SS (SEQ ID NO: 62).

1B6 humanized antibody heavy chain variable region 4 (1B6-huVH4):

QVQLVQSGAEVKKPGASVKVSCKASGYTFTDYNMHWVRQAPGQRLEWMGYINPN

NGGTSYNQKFQGRVTITVDTSASTAYMELSSLRSEDTAVYYCVTTRYLAVWGQGTTVTV

SS (SEQ ID NO: 63).

1B6 humanized antibody heavy chain variable region 5 (1B6-huVH5):

QVQLVQSGAEVKKPGASVKVSCKASGYTFTDYNMHWVRQAPGQRLEWMGYINPN

NGGTSYSQKFQGRVTITRDTSASTAYMELSSLRSEDTAVYYCVTTRYLAVWGQGTTVTVS

S (SEQ ID NO: 64).

1E7 humanized antibody heavy chain variable region 1 (1E7-huVL1):

QVQLVQSGAEVKKPGASVKLSCKASGYTFTSYWMHWVRQRPGQGLEWIGMIHPN SGSTNYNEKFKSKATLTVDKSTSTAYMELSSLRSEDTAVYYCARRYYGSISPDYWGQGTT

VTVSS (SEQ ID NO: 68).

1E7 humanized antibody heavy chain variable region 2 (1E7-huVL2):

QVQLVQSGAEVKKPGASVKLSCKASGYTFTSYWMHWVRQAPGQGLEWIGMIHPN SGSTNYNEKFKSRATLTVDTSTSTAYMELSSLRSEDTAVYYCARRYYGSISPDYWGQGTT

VTVSS (SEQ ID NO: 69).

1E7 humanized antibody heavy chain variable region 3 (1E7-huVL3):

QVQLVQSGAEVKKPGASVKVSCKASGYTFTSYWMHWVRQAPGQGLEWIGMIHPN SGSTNYNEKFKSRVTMTVDTSTSTAYMELSSLRSEDTAVYYCARRYYGSISPDYWGQGTT

VTVSS (SEQ ID NO: 70).

1E7 humanized antibody heavy chain variable region 4 (1E7-huVL4):

QVQLVQSGAEVKKPGASVKVSCKASGYTFTSYWMHWVRQAPGQGLEWMGMIHP NSGSTNYNEKFKSRVTMTVDTSTSTVYMELSSLRSEDTAVYYCARRYYGSISPDYWGQG

TTVTVSS (SEQ ID NO: 71).

1E7 humanized antibody heavy chain variable region 5 (1E7-huVL5):

QVQLVQSGAEVKKPGASVKVSCKASGYTFTSYWMHWVRQAPGQGLEWMGMIHP NSGSTNYAQKFQGRVTMTRDTSTSTVYMELSSLRSEDTAVYYCARRYYGSISPDYWGQG

TTVTVSS (SEQ ID NO: 72).

According to some specific embodiments of the present invention, the antibody comprises at least one of a heavy chain constant region and a light chain constant region, and at least a part of at least one of the heavy chain constant region and the light chain constant region is derived from at least one of a human antibody.

According to some specific embodiments of the present invention, both the light chain constant region and the heavy chain constant region of the antibody are derived from a human IgG antibody or a mutant thereof.

According to some specific embodiments of the present invention, the light chain constant region of the antibody is derived from light chain constant region of human Kappa; the heavy chain constant region of the antibody is derived from human IgG4 or IgG1, or a mutant of IgG4 or IgG1.

According to some specific embodiments of the present invention, the full-length sequence of the antibody constant region is shown in SEQ ID NO:73, 74 or 75.

The light chain constant region LC (Kappa):

RTVAAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC (SEQ ID NO: 73).

The IgG1 heavy chain constant region HC:

ASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAPELL GGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPS RDELTKNQVSLTCLVKGFYPSDIAVEWESNGQPENNYKTTPPVLDSDGSFFLYSKLTVDKS RWQQGNVFSCSVMHEALHNHYTQKSLSLSPGK (SEQ ID NO: 74).

The IgG4 heavy chain constant region HC:

ASTKGPSVFPLAPCSRSTSESTAALGCLVKDYFPEPVTVSWNSGALTSGVHTFPAVLQ SSGLYSLSSVVTVPSSSLGTKTYTCNVDHKPSNTKVDKRVESKYGPPCPPCPAPEFLGGPS VFLFPPKPKDTLMISRTPEVTCVVVDVSQEDPEVQFNWYVDGVEVHNAKTKPREEQFNS TYRVVSVLTVLHQDWLNGKEYKCKVSNKGLPSSIEKTISKAKGQPREPQVYTLPPSQEE MTKNQVSLTCLVKGFYPSDIAVEWESNGQPENNYKTTPPVLDSDGSFFLYSRLTVDKSRW QEGNVFSCSVMHEALHNHYTQKSLSLSLGK (SEQ ID NO: 75).

According to some specific embodiments of the present invention, the antibody has a light chain with an amino acid sequence as shown in any one of SEQ ID NO: 76-78 and a heavy chain with an amino acid sequence as shown in any one of SEQ ID NO: 79-88.

1B6 humanized antibody light chain 1B6-huVL1-LC (Kappa):

DIVMTQSPSSLSVSVGDRVTMTCKSSQSLFNSGNQKNYLTWYQQKPGKAPKLLIYW ASTRESGVPDRFSGSGSGTDFTLTISSVQPEDFATYFCQNDYSYPLTFGGGTKLEIK**RTVAA PSVFIFPPSDEQLKSGTASVVCLLNNFYPREAKVQWKVDNALQSGNSQESVTEQDSK DSTYSLSSTLTLSKADYEKHKVYACEVTHQGLSSPVTKSFNRGEC** (SEQ ID NO: 76).

1B6 humanized antibody light chain 1B6-huVL2-LC (Kappa):

DIQMTQSPSSLSASVGDRVTMTCKSSQSLFNSGNQKNYLTWYQQKPGKAPKLLIYW ASTRESGVPSRFSGSGSGTDFTLTISSLQPEDFATYYCQNDYSYPLTFGGGTKLEIK**RTVAA PSVFIFPPSDEQLKSGTASVVCLLNNFYPREAKVQWKVDNALQSGNSQESVTEQDSK DSTYSLSSTLTLSKADYEKHKVYACEVTHQGLSSPVTKSFNRGEC** (SEQ ID NO: 77).

1B6 humanized antibody light chain 1B6-huVL3-LC (Kappa):

DIQMTQSPSSLSASVGDRVTITCRSSQSLFNSGNQKNYLTWYQQKPGKAPKLLIYWA STRESGVPSRFSGSGSGTDFTLTISSLQPEDFATYYCQNDYSYPLTFGGGTKLEIK**RTVAAP SVFIFPPSDEQLKSGTASVVCLLNNFYPREAKVQWKVDNALQSGNSQESVTEQDSKD STYSLSSTLTLSKADYEKHKVYACEVTHQGLSSPVTKSFNRGEC** (SEQ ID NO: 78).

1B6 humanized antibody heavy chain 1B6-hu VH1-HC (IgG1):

EVQLVQSGAEVKKPGASVKMSCKASGYTFTDYNMHWVRQAPGKSLEWIGYINPN NGGTSYNQKFKGKATLTVNKSSSTAYMELSSLRSEDTAVYYCVTTRYLAVWGQGTTVTV SS**ASTKGPSVFPLAPSSKSTSGGTAALGCLVKDYFPEPVTVSWNSGALTSGVHTFPAV LQSSGLYSLSSVVTVPSSSLGTQTYICNVNHKPSNTKVDKKVEPKSCDKTHTCPPCPA PELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQP REPQVYTLPPSRDELTKNQVSLTCLVKGFYPSDIAVEWESNGQPENNYKTTPPVLDS DGSFFLYSKLTVDKSRWQQGNVFSCSVMHEALHNHYTQKSLSLSPGK** (SEQ ID NO: 79).

1B6 humanized antibody heavy chain 1B6-huVH2-HC (IgG1):

QVQLVQSGAEVKKPGASVKMSCKASGYTFTDYNMHWVRQAPGQRLEWIGYINPN NGGTSYNQKFKGKATLTVDTSASTAYMELSSLRSEDTAVYYCVTTRYLAVWGQGTTVTV SS**ASTKGPSVFPLAPSSKSTSGGTAALGCLVKDYFPEPVTVSWNSGALTSGVHTFPAV LQSSGLYSLSSVVTVPSSSLGTQTYICNVNHKPSNTKVDKKVEPKSCDKTHTCPPCPA PELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQP REPQVYTLPPSRDELTKNQVSLTCLVKGFYPSDIAVEWESNGQPENNYKTTPPVLDS DGSFFLYSKLTVDKSRWQQGNVFSCSVMHEALHNHYTQKSLSLSPGK** (SEQ ID NO: 80).

1B6 humanized antibody heavy chain 1B6-huVH3-HC (IgG1):

QVQLVQSGAEVKKPGASVKVSCKASGYTFTDYNMHWVRQAPGQRLEWIGYINPN NGGTSYNQKFKGRVTITVDTSASTAYMELSSLRSEDTAVYYCVTTRYLAVWGQGTTVTV SSASTKGPSVFPLAPSSKSTSGGTAALGCLVKDYFPEPVTVSWNSGALTSGVHTFPAV LQSSGLYSLSSVVTVPSSSLGTQTYICNVNHKPSNTKVDKKVEPKSCDKTHTCPPCPA PELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQP

REPQVYTLPPSRDELTKNQVSLTCLVKGFYPSDIAVEWESNGQPENNYKTTPPVLDS DGSFFLYSKLTVDKSRWQQGNVFSCSVMHEALHNHYTQKSLSLSPGK (SEQ ID NO: 81).

1B6 humanized antibody heavy chain 1B6-huVH4-HC (IgG1):

QVQLVQSGAEVKKPGASVKVSCKASGYTFTDYNMHWVRQAPGQRLEWMGYINPN NGGTSYNQKFQGRVTITVDTSASTAYMELSSLRSEDTAVYYCVTTRYLAVWGQGTTVTV SSASTKGPSVFPLAPSSKSTSGGTAALGCLVKDYFPEPVTVSWNSGALTSGVHTFPAV LQSSGLYSLSSVVTVPSSSLGTQTYICNVNHKPSNTKVDKKVEPKSCDKTHTCPPCPA PELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQP REPQVYTLPPSRDELTKNQVSLTCLVKGFYPSDIAVEWESNGQPENNYKTTPPVLDS DGSFFLYSKLTVDKSRWQQGNVFSCSVMHEALHNHYTQKSLSLSPGK (SEQ ID NO: 82).

1B6 humanized antibody heavy chain 1B6-huVH5-HC (IgG1):

QVQLVQSGAEVKKPGASVKVSCKASGYTFTDYNMHWVRQAPGQRLEWMGYINPN NGGTSYSQKFQGRVTITRDTSASTAYMELSSLRSEDTAVYYCVTTRYLAVWGQGTTVTVS SASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAP ELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPR EPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPGK (SEQ ID NO: 83).

1B6 humanized antibody heavy chain 1B6-huVH1-HC (IgG4):

EVQLVQSGAEVKKPGASVKMSCKASGYTFTDYNMHWVRQAPGKSLEWIGYINPN NGGTSYNQKFKGKATLTVNKSSSTAYMELSSLRSEDTAVYYCVTTRYLAVWGQGTTVTV SSASTKGPSVFPLAPCSRSTSESTAALGCLVKDYFPEPVTVSWNSGALTSGVHTFPAV LQSSGLYSLSSVVTVPSSSLGTKTYTCNVDHKPSNTKVDKRVESKYGPPCPPCPAPEF LGGPSVFLFPPKPKDTLMISRTPEVTCVVVDVSQEDPEVQFNWYVDGVEVHNAKTK PREEQFNSTYRVVSVLTVLHQDWLNGKEYKCKVSNKGLPSSIEKTISKAKGQPREP QVYTLPPSQEEMTKNQVSLTCLVKGFYPSDIAVEWESNGQPENNYKTTPPVLDSDGS

FFLYSRLTVDKSRWQEGNVFSCSVMHEALHNHYTQKSLSLSLGK (SEQ ID NO: 84).

1B6 humanized antibody heavy chain 1B6-huVH2-HC (IgG4):

QVQLVQSGAEVKKPGASVKMSCKASGYTFTDYNMHWVRQAPGQRLEWIGYINPN NGGTSYNQKFKGKATLTVDTSASTAYMELSSLRSEDTAVYYCVTTRYLAVWGQGTTVTV SSASTKGPSVFPLAPCSRSTSESTAALGCLVKDYFPEPVTVSWNSGALTSGVHTFPAV LQSSGLYSLSSVVTVPSSSLGTKTYTCNVDHKPSNTKVDKRVESKYGPPCPPCPAPEF LGGPSVFLFPPKPKDTLMISRTPEVTCVVVDVSQEDPEVQFNWYVDGVEVHNAKTK PREEQFNSTYRVVSVLTVLHQDWLNGKEYKCKVSNKGLPSSIEKTISKAKGQPREP QVYTLPPSQEEMTKNQVSLTCLVKGFYPSDIAVEWESNGQPENNYKTTPPVLDSDGS FFLYSRLTVDKSRWQEGNVFSCSVMHEALHNHYTQKSLSLSLGK (SEQ ID NO: 85).

1B6 humanized antibody heavy chain 1B6-huVH3-HC (IgG4):

QVQLVQSGAEVKKPGASVKVSCKASGYTFTDYNMHWVRQAPGQRLEWIGYINPN NGGTSYNQKFKGRVTITVDTSASTAYMELSSLRSEDTAVYYCVTTRYLAVWGQGTTVTV SSASTKGPSVFPLAPCSRSTSESTAALGCLVKDYFPEPVTVSWNSGALTSGVHTFPAV LQSSGLYSLSSVVTVPSSSLGTKTYTCNVDHKPSNTKVDKRVESKYGPPCPPCPAPEF LGGPSVFLFPPKPKDTLMISRTPEVTCVVVDVSQEDPEVQFNWYVDGVEVHNAKTK PREEQFNSTYRVVSVLTVLHQDWLNGKEYKCKVSNKGLPSSIEKTISKAKGQPREP QVYTLPPSQEEMTKNQVSLTCLVKGFYPSDIAVEWESNGQPENNYKTTPPVLDSDGS FFLYSRLTVDKSRWQEGNVFSCSVMHEALHNHYTQKSLSLSLGK (SEQ ID NO: 86).

1B6 humanized antibody heavy chain 1B6-huVH4-HC (IgG4):

QVQLVQSGAEVKKPGASVKVSCKASGYTFTDYNMHWVRQAPGQRLEWMGYINPN NGGTSYNQKFQGRVTITVDTSASTAYMELSSLRSEDTAVYYCVTTRYLAVWGQGTTVTV SS**ASTKGPSVFPLAPCSRSTSESTAALGCLVKDYFPEPVTVSWNSGALTSGVHTFPAV LQSSGLYSLSSVVTVPSSSLGTKTYTCNVDHKPSNTKVDKRVESKYGPPCPPCPAPEF LGGPSVFLFPPKPKDTLMISRTPEVTCVVVDVSQEDPEVQFNWYVDGVEVHNAKTK PREEQFNSTYRVVSVLTVLHQDWLNGKEYKCKVSNKGLPSSIEKTISKAKGQPREP QVYTLPPSQEEMTKNQVSLTCLVKGFYPSDIAVEWESNGQPENNYKTTPPVLDSDGS FFLYSRLTVDKSRWQEGNVFSCSVMHEALHNHYTQKSLSLSLGK** (SEQ ID NO: 87).

1B6 humanized antibody heavy chain 1B6-huVH5-HC (IgG4):

QVQLVQSGAEVKKPGASVKVSCKASGYTFTDYNMHWVRQAPGQRLEWMGYINPN NGGTSYSQKFQGRVTITRDTSASTAYMELSSLRSEDTAVYYCVTTRYLAVWGQGTTVTVS

**SASTKGPSVFPLAPCSRSTSESTAALGCLVKDYFPEPVTVSWNSGALTSGVHTFPAVL QSSGLYSLSSVVTVPSSSLGTKTYTCNVDHKPSNTKVDKRVESKYGPPCPPCPAPEFL GGPSVFLFPPKPKDTLMISRTPEVTCVVVDVSQEDPEVQFNWYVDGVEVHNAKTKP REEQFNSTYRVVSVLTVLHQDWLNGKEYKCKVSNKGLPSSIEKTISKAKGQPREPQ VYTLPPSQEEMTKNQVSLTCLVKGFYPSDIAVEWESNGQPENNYKTTPPVLDSDGSF FLYSRLTVDKSRWQEGNVFSCSVMHEALHNHYTQKSLSLSLGK** (SEQ ID NO: 88).

According to some specific embodiment of the present invention, the antibody has a light chain with an amino acid sequence as shown in any one of SEQ ID NO: 89-91 and heavy chain with an amino acid sequence as shown in any one of SEQ ID NO: 92-101.

1E7 humanized antibody light chain 1E7-huVL1-LC (Kappa):

DIVMTQSPSSLSVSVGDRVTMTCKSSQSLLNSGNQKNYLTWYQQKPGQPPKLLIYW ASTRESGVPDRFSGSGSGTDFTLTISSVQPEDFATYYCQNDYSYPFTFGGGTKLEIK**RTVA APSVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC** (SEQ ID NO: 89).

1E7 humanized antibody light chain 1E7-huVL2-LC (Kappa):

DIQMTQSPSSLSASVGDRVTMTCKSSQSLLNSGNQKNYLTWYQQKPGKAPKLLIYW ASTRESGVPSRFSGSGSGTDFTLTISSLQPEDFATYYCQNDYSYPFTFGGGTKLEIK**RTVAA PSVFIFPPSDEQLKSGTASVVCLLNNFYPREAKVQWKVDNALQSGNSQESVTEQDSK DSTYSLSSTLTLSKADYEKHKVYACEVTHQGLSSPVTKSFNRGEC** (SEQ ID NO: 90).

1E7 humanized antibody light chain 1E7-huVL3-LC (Kappa):

DIQMTQSPSSLSASVGDRVTITCRSSQSLLNSGNQKNYLTWYQQKPGKAPKLLIYWA STRESGVPSRFSGSGSGTDFTLTISSLQPEDFATYYCQNDYSYPFTFGGGTKLEIK**RTVAAP SVFIFPPSDEQLKSGTASVVCLLNNFYPREAKVQWKVDNALQSGNSQESVTEQDSKD STYSLSSTLTLSKADYEKHKVYACEVTHQGLSSPVTKSFNRGEC** (SEQ ID NO: 91).

1E7 humanized antibody heavy chain 1E7-huVH1-HC (IgG1):

QVQLVQSGAEVKKPGASVKLSCKASGYTFTSYWMHWVRQRPGQGLEWIGMIHPN SGSTNYNEKFKSKATLTVDKSTSTAYMELSSLRSEDTAVYYCARRYYGSISPDYWGQGTT VTVSS**ASTKGPSVFPLAPSSKSTSGGTAALGCLVKDYFPEPVTVSWNSGALTSGVHTF PAVLQSSGLYSLSSVVTVPSSSLGTQTYICNVNHKPSNTKVDKKVEPKSCDKTHTCPP CPAPELLGGPSVFLFPPKPKDTLMISRTPEVTCVVVDVSHEDPEVKFNWYVDGVEV HNAKTKPREEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSPGK** (SEQ ID NO: 92).

1E7 humanized antibody heavy chain 1E7-huVH2-HC (IgG1):

QVQLVQSGAEVKKPGASVKLSCKASGYTFTSYWMHWVRQAPGQGLEWIGMIHPN SGSTNYNEKFKSRATLTVDTSTSTAYMELSSLRSEDTAVYYCARRYYGSISPDYWGQGTT VTVSS**ASTKGPSVFPLAPSSKSTSGGTAALGCLVKDYFPEPVTVSWNSGALTSGVHTF PAVLQSSGLYSLSSVVTVPSSSLGTQTYICNVNHKPSNTKVDKKVEPKSCDKTHTCPP CPAPELLGGPSVFLFPPKPKDTLMISRTPEVTCVVVDVSHEDPEVKFNWYVDGVEV HNAKTKPREEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSPGK** (SEQ ID NO: 93).

1E7 humanized antibody heavy chain 1E7-huVH3-HC (IgG1):

QVQLVQSGAEVKKPGASVKVSCKASGYTFTSYWMHWVRQAPGQGLEWIGMIHPN SGSTNYNEKFKSRVTMTVDTSTSTAYMELSSLRSEDTAVYYCARRYYGSISPDYWGQGTT VTVSS**ASTKGPSVFPLAPSSKSTSGGTAALGCLVKDYFPEPVTVSWNSGALTSGVHTF PAVLQSSGLYSLSSVVTVPSSSLGTQTYICNVNHKPSNTKVDKKVEPKSCDKTHTCPP CPAPELLGGPSVFLFPPKPKDTLMISRTPEVTCVVVDVSHEDPEVKFNWYVDGVEV HNAKTKPREEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSPGK** (SEQ ID NO: 94).

1E7 humanized antibody heavy chain 1E7-huVH4-HC (IgG1):

QVQLVQSGAEVKKPGASVKVSCKASGYTFTSYWMHWVRQAPGQGLEWMGMIHP NSGSTNYNEKFKSRVTMTVDTSTSTVYMELSSLRSEDTAVYYCARRYYGSISPDYWGQG TTVTVSS**ASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSCDKTHTC PPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKA KGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPGK** (SEQ ID NO: 95).

1E7 humanized antibody heavy chain 1E7-huVH5-HC (IgG1):

QVQLVQSGAEVKKPGASVKVSCKASGYTFTSYWMHWVRQAPGQGLEWMGMIHP NSGSTNYAQKFQGRVTMTRDTSTSTVYMELSSLRSEDTAVYYCARRYYGSISPDYWGQG TTVTVSS**ASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSCDKTHTC PPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKA KGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPGK** (SEQ ID NO: 96).

1E7 humanized antibody heavy chain 1E7-huVH1-HC (IgG4):

QVQLVQSGAEVKKPGASVKLSCKASGYTFTSYWMHWVRQRPGQGLEWIGMIHPN SGSTNYNEKFKSKATLTVDKSTSTAYMELSSLRSEDTAVYYCARRYYGSISPDYWGQGTT VTVSS**ASTKGPSVFPLAPCSRSTSESTAALGCLVKDYFPEPVTVSWNSGALTSGVHTF PAVLQSSGLYSLSSVVTVPSSSLGTKTYTCNVDHKPSNTKVDKRVESKYGPPCPPCPA PEFLGGPSVFLFPPKPKDTLMISRTPEVTCVVVDVSQEDPEVQFNWYVDGVEVHNA KTKPREEQFNSTYRVVSVLTVLHQDWLNGKEYKCKVSNKGLPSSIEKTISKAKGQP REPQVYTLPPSQEEMTKNQVSLTCLVKGFYPSDIAVEWESNGQPENNYKTTPPVLDS DGSFFLYSRLTVDKSRWQEGNVFSCSVMHEALHNHYTQKSLSLSLGK** (SEQ ID NO:97).

1E7 humanized antibody heavy chain 1E7-huVH2-HC (IgG4):

QVQLVQSGAEVKKPGASVKLSCKASGYTFTSYWMHWVRQAPGQGLEWIGMIHPN SGSTNYNEKFKSRATLTVDTSTSTAYMELSSLRSEDTAVYYCARRYYGSISPDYWGQGTT VTVSS**ASTKGPSVFPLAPCSRSTSESTAALGCLVKDYFPEPVTVSWNSGALTSGVHTF PAVLQSSGLYSLSSVVTVPSSSLGTKTYTCNVDHKPSNTKVDKRVESKYGPPCPPCPA PEFLGGPSVFLFPPKPKDTLMISRTPEVTCVVVDVSQEDPEVQFNWYVDGVEVHNA KTKPREEQFNSTYRVVSVLTVLHQDWLNGKEYKCKVSNKGLPSSIEKTISKAKGQP REPQVYTLPPSQEEMTKNQVSLTCLVKGFYPSDIAVEWESNGQPENNYKTTPPVLDS DGSFFLYSRLTVDKSRWQEGNVFSCSVMHEALHNHYTQKSLSLSLGK** (SEQ ID NO: 98).

1E7 humanized antibody heavy chain 1E7-huVH3-HC (IgG4):

QVQLVQSGAEVKKPGASVKVSCKASGYTFTSYWMHWVRQAPGQGLEWIGMIHPN SGSTNYNEKFKSRVTMTVDTSTSTAYMELSSLRSEDTAVYYCARRYYGSISPDYWGQGTT VTVSS**ASTKGPSVFPLAPCSRSTSESTAALGCLVKDYFPEPVTVSWNSGALTSGVHTF PAVLQSSGLYSLSSVVTVPSSSLGTKTYTCNVDHKPSNTKVDKRVESKYGPPCPPCPA PEFLGGPSVFLFPPKPKDTLMISRTPEVTCVVVDVSQEDPEVQFNWYVDGVEVHNA KTKPREEQFNSTYRVVSVLTVLHQDWLNGKEYKCKVSNKGLPSSIEKTISKAKGQP REPQVYTLPPSQEEMTKNQVSLTCLVKGFYPSDIAVEWESNGQPENNYKTTPPVLDS DGSFFLYSRLTVDKSRWQEGNVFSCSVMHEALHNHYTQKSLSLSLGK** (SEQ ID NO: 99).

1E7 humanized antibody heavy chain 1E7-huVH4-HC (IgG4):

QVQLVQSGAEVKKPGASVKVSCKASGYTFTSYWMHWVRQAPGQGLEWMGMIHPNSGSTNYNEKFKSRVTMTVDTSTSTVYMELSSLRSEDTAVYYCARRYYGSISPDYWGQGTTVTVSS**ASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGK** (SEQ ID NO: 100).

1E7 humanized antibody heavy chain 1E7-huVH5-HC (IgG4):

QVQLVQSGAEVKKPGASVKVSCKASGYTFTSYWMHWVRQAPGQGLEWMGMIHPNSGSTNYAQKFQGRVTMTRDTSTSTVYMELSSLRSEDTAVYYCARRYYGSISPDYWGQGTTVTVSS**ASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGK** (SEQ ID NO: 101).

According to some specific embodiments of the present invention, the antibody comprises: a light chain as shown in SEQ ID NO: 76, a heavy chain as shown in SEQ ID NO: 79, or a light chain as shown in SEQ ID NO: 77, a heavy chain as shown in SEQ ID NO: 79, or a light chain as shown in SEQ ID NO: 76, a heavy chain as shown in SEQ ID NO: 80, or a light chain as shown in SEQ ID NO: 77, a heavy chain as shown in SEQ ID NO: 80, or a light chain as shown in SEQ ID NO: 76, a heavy chain as shown in SEQ ID NO: 81, or a light chain as shown in SEQ ID NO: 77, a heavy chain as shown in SEQ ID NO: 81, or a light chain as shown in SEQ ID NO: 78, a heavy chain as shown in SEQ ID NO: 81, or a light chain as shown in SEQ ID NO: 77, a heavy chain as shown in SEQ ID NO: 82, or a light chain as shown in SEQ ID NO: 78, a heavy chain as shown in SEQ ID NO: 82, or a light chain as shown in SEQ ID NO: 77, a heavy chain as shown in SEQ ID NO: 83, or a light chain as shown in SEQ ID NO: 78, a heavy chain as shown in SEQ ID NO: 83, or a light chain as shown in SEQ ID NO: 76, a heavy chain as shown in SEQ ID NO: 84, or a light chain as shown in SEQ ID NO: 77, a heavy chain as shown in SEQ ID NO: 84, or a light chain as shown in SEQ ID NO: 76, a heavy chain as shown in SEQ ID NO: 85, or a light chain as shown in SEQ ID NO: 77, a heavy chain as shown in SEQ ID NO: 85, or a light chain as shown in SEQ ID NO: 76, a heavy chain as shown in SEQ ID NO: 86, or a light chain as shown in SEQ ID NO: 77, a heavy chain as shown in SEQ ID NO: 86, or a light chain as shown in SEQ ID NO: 78, a heavy chain as shown in SEQ ID NO: 86, or a light chain as shown in SEQ ID NO: 77, a heavy chain as shown in SEQ ID NO: 87, or a light chain as shown in SEQ ID NO: 78, a heavy chain as shown in SEQ ID NO: 87, or a light chain as shown in SEQ ID NO: 77, a heavy chain as shown in SEQ ID NO: 88, or a light chain as shown in SEQ ID NO: 78, a heavy chain as shown in SEQ ID NO: 88.

According to some specific embodiments of the present invention, the antibody comprises: a light chain as shown in SEQ ID NO: 89, a heavy chain as shown in SEQ ID NO: 92, or a light chain as shown in SEQ ID NO: 90, a heavy chain as shown in SEQ ID NO: 92, or a light chain as shown in SEQ ID NO: 89, a heavy chain as shown in SEQ ID NO: 93, or a light chain as shown in SEQ ID NO: 90, a heavy chain as shown in SEQ ID NO: 93, or a light chain as shown in SEQ ID NO: 89, a heavy chain as shown in SEQ ID NO: 94, or a light chain as shown in SEQ ID NO: 90, a heavy chain as shown in SEQ ID NO: 94, or a light chain as shown in SEQ ID NO: 91, a heavy chain as shown in SEQ ID NO: 94, or a light chain as shown in SEQ ID NO: 90, a heavy chain as shown in SEQ ID NO: 95, or a light chain as shown in SEQ ID NO: 91, a heavy chain as shown in SEQ ID NO: 95, or a light chain as shown in SEQ ID NO: 90, a heavy chain as shown in SEQ ID NO: 96, or a light chain as shown in SEQ ID NO: 91, a heavy chain as shown in SEQ ID NO: 96, or a light chain as shown in SEQ ID NO: 89, a heavy chain as shown in SEQ ID NO: 97, or a light chain as shown in SEQ ID NO: 90, a heavy chain as shown in SEQ ID NO: 97, or a light chain as shown in SEQ ID NO: 89, a heavy chain as shown in SEQ ID NO: 98, or a light chain as shown in SEQ ID NO: 90, a heavy chain as shown in SEQ ID NO: 98, or a light chain as shown in SEQ ID NO: 89, a heavy chain as shown in SEQ ID NO: 99, or a light chain as shown in SEQ ID NO: 90, a heavy chain as shown in SEQ ID NO: 99, or a light chain as shown in SEQ ID NO: 91, a heavy chain as shown in SEQ ID NO: 99, or a light chain as shown in SEQ ID NO: 90, a heavy chain as shown in SEQ ID NO: 100, or a light chain as shown in SEQ ID NO: 91, a heavy chain as shown in SEQ ID NO: 100, or a light chain as shown in SEQ ID NO: 90, a heavy chain as shown in SEQ ID NO: 101, or a light chain as shown in SEQ ID NO: 91, a heavy chain as shown in SEQ ID NO: 101.

According to some specific embodiments of the present invention, the antibody is a single chain antibody, a multimeric antibody, a CDR-grafted antibody or a small molecule antibody.

According to some specific embodiments of the present invention, the small molecule antibody includes at least one of a Fab antibody, a Fv antibody, a single domain antibody and a minimum recognition unit.

## Immune cell, CAR-T cell

The term "chimeric antigen receptor (CAR)" is a molecule that combines antibody-based specificity against a desired antigen (e.g., tumor antigen) with T cell receptor-activating intracellular domain to produce a chimeric protein that exhibits specific anti-tumor cell immune activity.

"CAR-T cell" refer to chimeric antigen receptor T cell, which can be understood as T cell that can express chimeric antigen receptor so that T cell can perform specific killing function.

In some embodiments, the present invention provides an immune cell, the immune cell carries the nucleic acid molecule described above, or expresses the antibody or antigen-binding fragment thereof described above. According to some specific embodiments of the present invention, the immune cell can express the aforementioned antibody or antigen-binding fragment thereof that specifically recognize Claudin18.2 in large quantities, so as to obtain the antibody or antigen-binding fragment thereof in large quantities in vitro. Furthermore, the immune cell can be reinfused *in vivo* to treat Claudin18.2-related diseases.

According to some specific embodiments of the present invention, the aforementioned immune cell may further include at least one of the following additional technical features:

According to some specific embodiments of the present invention, the immune cells include at least one of T lymphocytes, DC cells, NK cells, and NKT lymphocytes. The immune cells according to some embodiments of the present invention use the aforementioned antibody to recognize and kill target proteins, cells, tissues, organs, *etc.,* and then perform biological functions.

In some embodiments, the present invention provides a CAR-T cell including a chimeric antigen receptor, wherein the chimeric antigen receptor includes: an extracellular region, the extracellular region includes a heavy chain variable region and a light chain variable region of a single-chain antibody, the light chain variable region has an amino acid sequence as shown in SEQ ID NO: 57-59 or SEQ ID NO: 65-67, and the heavy chain variable region has an amino acid sequence as shown in SEQ ID NO: 60-64 or SEQ ID NO: 68-72; a transmembrane region, the transmembrane region is connected to the extracellular region and embedded in the cell membrane of the T lymphocyte; and an intracellular region, the intracellular region is connected to the transmembrane region. CAR-T cells expressing this chimeric antigen receptor can kill tumor cells expressing Claudin18.2.

In some embodiments, the transmembrane region is an OX40 transmembrane region, an ICOS transmembrane region or a CD8 transmembrane region, preferably, the transmembrane region is a CD8 transmembrane region.

In some embodiments, the amino acid sequence of the CD8 trans-membrane region (TM) is shown in SEQ ID NO: 106:

IYIWAPLAGTCGVLLLSLVITLYC (SEQ ID NO: 106)

In some embodiments, the amino acid sequence of the ICOS trans-membrane region (TM) is shown in SEQ ID NO: 107:

FWLPIGCAAFVVVCILGCILICWL (SEQ ID NO: 107)

In some embodiments, the amino acid sequence of the OX40 trans-membrane region (TM) is shown in SEQ ID NO: 108:

VAAILGLGLVLGLLGPLAILLALYLL (SEQ ID NO: 108)

In some embodiments, the intracellular segment is a 4-1BB intracellular segment and an ICOS or OX-40 intracellular segment; preferably, the intracellular segment is a 4-1BB intracellular segment and an ICOS intracellular segment.

The amino acid sequence of the intracellular segment of the 4-1BB immune co-stimulator is shown in SEQ ID NO: 109:

KRGRKKLLYIFKQPFMRPVQTTQEEDGCSCRFPEEEEGGCEL (SEQ ID NO: 109)

The amino acid sequence of the intracellular segment of the ICOS immune co-stimulator is shown in SEQ ID NO: 110:

CWLTKKKYSSSVHDPNGEYMFMRAVNTAKKSRLTDVTL (SEQ ID NO: 110)

In some embodiments, the extracellular region further comprises a CD8 hinge region. The amino acid sequence of the CD8 hinge region is shown in SEQ ID NO: 111:

TTTPAPRPPTPAPTIASQPLSLRPEACRPAAGGAVHTRGLDFACD (SEQ ID NO:111)

In some embodiments, the intracellular segment further comprises a CD3ζ chain. The amino acid sequence of the CD3ζ chain is shown in SEQ ID NO: 112:

RVKFSRSADAPAYQQGQNQLYNELNLGRREEYDVLDKRRGRDPEMGGKPQRRKNP

QEGLYNELQKDKMAEAYSEIGMKGERRRGKGHDGLYQGLSTATKDTYDALHMQALPPR

(SEQ ID NO:112)

In some embodiments, the N-terminus of the ICOS intracellular segment is connected to the C-terminus of the CD8 transmembrane region, the C-terminus of the ICOS intracellular segment is connected to the N-terminus of the 4-1BB intracellular segment, and the C-terminus of the 4-1BB intracellular segment is connected to the N-terminus of the CD3ζ chain.

According to an embodiment of the present invention, the structure of the chimeric antigen receptor is: signal peptide-Anti-Claudin 18.2 scfv-CD8 hinge + CD8TM-ICOS -4-1BB -CD3ζ. The Anti-Claudin18.2 scfv comprises a light chain variable region with an amino acid sequence as shown in SEQ ID NO: 57-59 or SEQ ID NO: 65-67, and the heavy chain variable region with an amino acid sequence as shown in SEQ ID NO: 60-64 or SEQ ID NO: 68-72.

**Nucleic acid molecule, expression vector, recombinant cell**

In the process of preparing or obtaining these antibodies or chimeric antigen receptors, nucleic acid molecules expressing these antibodies or chimeric antigen receptors can be linked to different vectors and expressed in different cells to obtain corresponding antibodies or chimeric antigen receptors.

In some embodiments, the present invention provides a nucleic acid molecule, the nucleic acid molecule encodes the antibody or antigen-binding fragment thereof provided in the first aspect. The antibodies obtained from the nucleic acid molecules of some specific embodiments of the present invention can not only specifically target and bind to Claudin18.2 and have the same binding activity as the chimeric antibody, but also have a longer *in vivo* half-life.

According to some specific embodiments of the present invention, the aforementioned nucleic acid molecule may further include at least one of the following additional technical features:

According to some specific embodiments of the present invention, the nucleic acid molecule is DNA.

It should be noted that, for the nucleic acids mentioned in the specification and claims of the present invention, those skilled in the art should understand that they actually include any one or both of the complementary double chains. For convenience, in the present specification and claims, although only one chain is shown in most cases, another chain complementary thereto is actually also disclosed. In addition, the nucleic acid sequences in the present application include DNA form or RNA form, and disclosure of one of them means that the other is also disclosed.

In some embodiments, the present invention provides an expression vector, the expression vector carries the nucleic acid molecule described above. When the nucleic acid molecule described above is connected to the vector, the nucleic acid molecule can be directly or indirectly connected to the control elements on the vector, as long as these control elements can control the translation and expression of the nucleic acid molecule. Of course, these control elements can come directly from the carrier itself, or exogenous, that is, not from the carrier itself. Of course, tthe nucleic acid molecule can be operably linked to the control element. Here, "operably linked" refers to the connection of the exogenous gene to the vector, so that the control elements in the vector, such as transcription control sequence and translation control sequence, *etc.,* can perform its expected function of regulating the transcription and translation of the exogenous gene. Of course, the nucleic acid molecule used to encode the heavy chain and light chain of an antibody can be inserted into different vectors independently, and it is common to insert into the same vector. Commonly used vectors can be, for example, plasmids, bacteriophages, and the like. After the expression vector according to some specific embodiments of the present invention is introduced into a suitable recipient cell, it can effectively realize the expression of the aforementioned antibody or antigen-binding fragment thereof that specifically recognizes Claudin18.2 under the mediation of the regulatory system, thereby realizing the mass acquisition of the antibody or antigen-binding fragment in vitro.

According to some specific embodiments of the present invention, the aforementioned expression vector may further include at least one of the following additional technical features:

According to some specific embodiments of the present invention, the expression vector is a eukaryotic expression vector or a virus. Then, the above-mentioned antibody or antigen-binding fragment thereof which specifically recognizes Claudin18.2 is expressed in suitable receptor cells, such as CHO cells.

According to some specific embodiments of the present invention, the virus is a lentivirus.

In some embodiments, the present invention provides a recombinant cell, the recombinant cell carries the nucleic acid molecule described above, or expresses the antibody or antigen-binding fragment thereof described above. The recombinant cells according to some specific embodiments of the present invention can be used for the in vitro expression and mass acquisition of the aforementioned antibodies or antigen-binding fragments specifically recognizing Claudin18.2.

According to some specific embodiments of the present invention, the aforementioned recombinant cell may further include at least one of the following additional technical features:

According to some specific embodiments of the present invention, the recombinant cell is obtained by introducing the aforementioned expression vector into a host cell.

According to some specific embodiments of the present invention, the expression vector is introduced into the host cell by electrotransduction.

According to some specific embodiments of the present invention, the recombinant cell is a eukaryotic cell.

According to some specific embodiments of the present invention, the recombinant cell is a mammalian cell.

According to some specific embodiments of the present invention, the eukaryotic cell does not include animal germ cell, oosperm or embryonic stem cell.

It should be noted that the recombinant cell of the present invention is not particularly limited and may be prokaryotic cell, eukaryotic cell or bacteriophage. The prokaryotic cell may be Escherichia coli, Bacillus subtilis, Streptomyces or Proteus mirabilis. The eukaryotic cell may be fungi such as Pichia pastori, Saccharomyce cerevisiae, Schizosaccharomyces pombe, Trichoderma, insect cell such as S. frugiperda, plant cell such as tobacco, mammalian cell such as BHK cell, CHO cell, COS cell, myeloma cell, etc. In some embodiments, the recombinant cell of the present invention is preferably mammalian cell, including BHK cell, CHO cell, NSO cell or COS cell, and do not include animal germ cell, oosperm or embryonic stem cell.

It should be noted that the "suitable conditions" described in the specification of this application refer to conditions suitable for the expression of the recombinant antibodies described in this application. It is easily understood by those skilled in the art that conditions suitable for expression of recombinant antibodies include, but are not limited to, suitable transformation or transfection methods, suitable transformation or transfection conditions, healthy host cell status, suitable host cell density, suitable cell culture environment, and suitable cell culture time. The "suitable conditions" are not particularly limited, and those skilled in the art can optimize the most suitable conditions for expressing the recombinant antibody according to the specific environment of the laboratory.

**Pharmaceutical composition, kit and pharmaceutical use and use in the preparation of kit**

In some embodiments, the present invention provides a pharmaceutical composition, the pharmaceutical composition includes the aforementioned antibody, nucleic acid molecule, expression vector, immune cell, recombinant cell or CAR-T cell. As mentioned above, the antibody, as well as the nucleic acid molecule, expression vector, immune cell, recombinant cell or CAR-T cell that can obtain the antibody after expression can not only specifically target and bind to Claudin18.2, and have the same binding activity as the chimeric antibody, but also have a longer *in vivo* half-life. Therefore, the antibody or expressed antibody contained in the pharmaceutical composition according to some specific embodiments of the present invention can specifically target and bind to Claudin18.2, has strong specificity, and exerts a good targeting effect, thereby realizing the biological effects of other drugs in the pharmaceutical composition, such as the activity inhibition of Claudin18.2 molec ule, the killing of cells expressing Claudin18.2 molecule, and the like.

In some embodiments, these pharmaceutical compositions further include a pharmaceutically acceptable carrier, including any solvents, solid excipients, diluents, binders, disintegrants, or other liquid excipients, dispersing agents, flavoring or suspending agents, surfactants, isotonic agents, thickeners, emulsifiers, preservatives, solid binders, glidants or lubricants, *etc.,* which are suitable for specific target dosage forms. Except insofar as any conventional excipients incompatible with the compounds disclosed herein, such as by producing any undesirable biological effect or otherwise interacting in a deleterious manner with any other components of the pharmaceutically acceptable composition, its use is contemplated to be within the scope of this invention.

For example, the antibodies of the present invention can be incorporated into pharmaceutical compositions suitable for parenteral administration (e.g., intravenous, subcutaneous, intraperitoneal, intramuscular). These pharmaceutical compositions can be prepared in various forms. For example, liquid, semi-solid and solid dosage forms, including but not limited to liquid solutions (for example, injection solutions and infusion solutions), dispersions or suspensions, tablets, pills, powders, liposomes, and suppositories. Typical pharmaceutical compositions are in the form of injection solutions or infusion solutions. The antibody can be administered by intravenous infusion or injection, or intramuscular or subcutaneous injection.

It should be noted that the composition includes combinations separated in time and/or space, as long as they can work together to achieve the purpose of the present invention. For example, the components contained in the composition may be administered to the subject as a whole, or may be administered to the subject separately. When the components contained in the composition are administered to the subject separately, the respective components may be administered to the subject simultaneously or sequentially.

In some embodiments, the present invention provides the use of the aforementioned antibody, the aforementioned nucleic acid molecule, the aforementioned expression vector, immune cell, recombinant cell, CAR-T cell or the aforementioned pharmaceutical composition in the manufacture of of a medicament for the treatment or prevention of Claudin18.2 related diseases. As mentioned above, the antibody, as well as the nucleic acid molecule, expression vector, immune cell, recombinant cell or CAR-T cell that can obtain the antibody after expression can not only specifically target and bind to Claudin18.2, and have the same binding activity as the chimeric antibody, but also have a longer *in vivo* half-life. Therefore, drugs containing the above substances can also effectively target and bind to Claudin18.2, have a longer half-life *in vivo,* and can effectively kill cells expressing Claudin18.2 molecules.

According to some specific embodiments of the present invention, the aforementioned use may further include at least one of the following additional technical features:

According to some specific embodiments of the present invention, the Claudin18.2 related diseases include: at least one of gastric cancer, esophageal cancer, pancreatic cancer, lung cancer, colon cancer and rectal cancer. Those skilled in the art will appreciate that the drug of the present invention can be effective for any tissue or organ that highly expresses Claudin18.2, and therefore, the drug can be used to treat or alleviate lesions of the corresponding tissue or organ.

In some embodiments, the present invention provides a kit for detecting Claudin18.2, the kit includes any one of the antibody described above. The kit provided by the present invention can be used, for example, for immunoblotting, immunoprecipitation, *etc.,* which involve the use of the specific binding properties of Claudin18.2 antigen and antibody to detect. These kits may contain any one or more of the following: antagonist, Claudin18.2 antibody or drug reference material; protein purification column; immunoglobulin affinity purification buffer; cell assay diluent; instructions or literature, *etc.* The Claudin18.2 antibodies can be used in different types of diagnostic tests, for example, it can detect the presence of various diseases or drugs, toxins or other proteins in vitro or *in vivo.* For example, the subject's serum or blood can be tested for related diseases. Such as cancers or tumors, these cancers or tumors can be any unregulated cell growth.

In some embodiments, the present invention provides the use of the aforementioned antibody, the aforementioned nucleic acid molecule, the aforementioned expression vector or the aforementioned recombinant cell in the manufacture of a kit for detecting Claudin 18.2 or diagnosing Claudin 18.2 related diseases. The Claudin18.2 antibodies can be used in combination with any detection reagent or therapeutic agent, for example, in combination with diagnostic nuclides, nanomaterials, *etc.,* to detect the target site through the radioactivity of the nuclide, so as to obtain information about the target site; it can also be used in combination with therapeutic nuclides to use the radioactivity of the nuclides to specifically kill target cells, tissues, *etc.*

The scheme of the present invention will be explained below with reference to embodiments. Those skilled in the art will appreciate that the following embodiments are only used to illustrate the present invention and should not be construed as limiting the scope of the present invention. If the specific technology or conditions are not indicated in the examples, the technology or conditions described in the literature in the art or the product descriptions are performed. If the reagents or instruments used are not specified by the manufacturers, they are all conventional products that are commercially available.

Example 1 Design and analysis of humanized antibody sequences

The applicant used Discovery Studio and Schrõdinger Antibody Modeling for the 1B6 mouse antibody sequence (SEQ ID NO: 102, 103) and the 1E7 mouse antibody sequence (SEQ ID NO: 104, 105), respectively, and used PDB BLAST to retrieve the 10 antibody crystal structure models with the closest sequences (with a structural resolution higher than 2.5 angstroms), compared them with the automatic modeling models, and selected the optimal structural model. IgBLAST was used to align the 1B6 and 1E7 murine antibody sequences with the human GermLine sequences, and the GermLine with the highest homology was selected. Based on the modeled structural model, human GermLine sequence alignment results and the characteristics of different subtypes (IgG1, IgG4), the murine FR region was replaced with the human FR region, and some key residues in the human FR region were back mutated to murine residues. The humanization design was completed when the degree of humanization of the variable region (excluding the CDR region) was greater than 90%. The specific sequence of the obtained human antibody 1B6 is shown in SEQ ID NO:57-64, and the specific sequence of the obtained human antibody 1E7 is shown in SEQ ID NO:65-72. In addition, the inventors replaced the murine light chain constant region with the human light chain constant region (Kappa) (SEQ ID NO: 73), and replaced the murine heavy chain constant region with the heavy chain constant region of the human IgG1 antibody (SEQ ID NO: 74) or the heavy chain constant region of the human IgG4 antibody (SEQ ID NO: 75).

Murine antibody 1B6 light chain variable region (1B6-VL):

DIVMTQSPSSLTVTAGEKVTMSCKSSQSLFNSGNQKNYLTWYQQKPGQPPKLLIYW ASTRESGVPDRFTGSGSGTDFTLTISSVQAEDLAVYFCQNDYSYPLTFGAGTKLELK (SEQ ID NO: 102).

Murine antibody 1B6 heavy chain variable region (1B6-VH):

EVQLQQSGPELVRPGASVKMSCKASGYTFTDYNMHWVKQSHGKSLEWIGYINPNN GGTSYNQKFKGKATLTVNKSSSTAYMELRSLTSEDSAVYYCVTTRYLAVWGTGTTVTVS S (SEQ ID NO: 103).

Murine antibody 1E7 light chain variable region (1E7-VL):

DIVMTQSPSSLSVTAGEKVTMSCKSSQSLLNSGNQKNYLTWYQQKPGQPPKLLIYW ASTRESGVPDRFTGSGSGTDFTLTISSVQAEDLAVYYCQNDYSYPFTFGSGTKLEIK (SEQ ID NO: 104).

Murine antibody 1E7 heavy chain variable region (1E7-VH):

QVQLQQPGSELVKPGASVKLSCKASGYTFTSYWMHWVKQRPGQGLEWIGMIHPNS GSTNYNEKFKSKATLTVDKSSSTAYMQLSSLTSEDSAVYYCARRYYGSISPDYWGQGTTL TVSS (SEQ ID NO: 105).

Humanized 1B6 antibody light chain variable region 1 (1B6-huVL1):

DIVMTQSPSSLSVSVGDRVTMTCKSSQSLFNSGNQKNYLTWYQQKPGKAPKLLIYW ASTRESGVPDRFSGSGSGTDFTLTISSVQPEDFATYFCQNDYSYPLTFGGGTKLEIK (SEQ ID NO: 57).

Humanized 1B6 antibody light chain variable region 2 (1B6-huVL2):

DIQMTQSPSSLSASVGDRVTMTCKSSQSLFNSGNQKNYLTWYQQKPGKAPKLLIYW ASTRESGVPSRFSGSGSGTDFTLTISSLQPEDFATYYCQNDYSYPLTFGGGTKLEIK (SEQ ID NO: 58).

Humanized 1B6 antibody light chain variable region 3 (1B6-huVL3):

DIQMTQSPSSLSASVGDRVTITCRSSQSLFNSGNQKNYLTWYQQKPGKAPKLLIYWA
STRESGVPSRFSGSGSGTDFTLTISSLQPEDFATYYCQNDYSYPLTFGGGTKLEIK (SEQ ID NO: 59).

Humanized 1B6 antibody heavy chain variable region 1 (1B6-huVH1):

EVQLVQSGAEVKKPGASVKMSCKASGYTFTDYNMHWVRQAPGKSLEWIGYINPNN
GGTSYNQKFKGKATLTVNKSSSTAYMELSSLRSEDTAVYYCVTTRYLAVWGQGTTVTVS
S (SEQ ID NO: 60).

Humanized 1B6 antibody heavy chain variable region 2 (1B6-huVH2):

QVQLVQSGAEVKKPGASVKMSCKASGYTFTDYNMHWVRQAPGQRLEWIGYINPN
NGGTSYNQKFKGKATLTVDTSASTAYMELSSLRSEDTAVYYCVTTRYLAVWGQGTTVTV
SS (SEQ ID NO: 61).

Humanized 1B6 antibody heavy chain variable region 3 (1B6-huVH3):

QVQLVQSGAEVKKPGASVKVSCKASGYTFTDYNMHWVRQAPGQRLEWIGYINPN
NGGTSYNQKFKGRVTITVDTSASTAYMELSSLRSEDTAVYYCVTTRYLAVWGQGTTVTV
SS (SEQ ID NO: 62).

Humanized 1B6 antibody heavy chain variable region 4 (1B6-huVH4):

QVQLVQSGAEVKKPGASVKVSCKASGYTFTDYNMHWVRQAPGQRLEWMGYINPN
NGGTSYNQKFQGRVTITVDTSASTAYMELSSLRSEDTAVYYCVTTRYLAVWGQGTTVTV
SS (SEQ ID NO: 63).

Humanized 1B6 antibody heavy chain variable region 5 (1B6-huVH5):

QVQLVQSGAEVKKPGASVKVSCKASGYTFTDYNMHWVRQAPGQRLEWMGYINPN
NGGTSYSQKFQGRVTITRDTSASTAYMELSSLRSEDTAVYYCVTTRYLAVWGQGTTVTVS
S (SEQ ID NO: 64).

Humanized 1E7 antibody light chain variable region 1 (1E7-huVL1):

DIVMTQSPSSLSVSVGDRVTMTCKSSQSLLNSGNQKNYLTWYQQKPGQPPKLLIYW
ASTRESGVPDRFSGSGSGTDFTLTISSVQPEDFATYYCQNDYSYPFTFGGGTKLEIK (SEQ
ID NO: 65).

Humanized 1E7 antibody light chain variable region 2 (1E7-huVL2):

DIQMTQSPSSLSASVGDRVTMTCKSSQSLLNSGNQKNYLTWYQQKPGKAPKLLIYW ASTRESGVPSRFSGSGSGTDFTLTISSLQPEDFATYYCQNDYSYPFTFGGGTKLEIK (SEQ ID NO: 66).

Humanized 1E7 antibody light chain variable region 3 (1E7-huVL3):

DIQMTQSPSSLSASVGDRVTITCRSSQSLLNSGNQKNYLTWYQQKPGKAPKLLIYWA STRESGVPSRFSGSGSGTDFTLTISSLQPEDFATYYCQNDYSYPFTFGGGTKLEIK (SEQ ID NO: 67).

Humanized 1E7 antibody heavy chain variable region 1 (1E7-huVH1):

QVQLVQSGAEVKKPGASVKLSCKASGYTFTSYWMHWVRQRPGQGLEWIGMIHPNS GSTNYNEKFKSKATLTVDKSTSTAYMELSSLRSEDTAVYYCARRYYGSISPDYWGQGTTV TVSS (SEQ ID NO: 68).

Humanized 1E7 antibody heavy chain variable region 2 (1E7-huVH2):

QVQLVQSGAEVKKPGASVKLSCKASGYTFTSYWMHWVRQAPGQGLEWIGMIHPN SGSTNYNEKFKSRATLTVDTSTSTAYMELSSLRSEDTAVYYCARRYYGSISPDYWGQGTT VTVSS (SEQ ID NO: 69).

Humanized 1E7 antibody heavy chain variable region 3 (1E7-huVH3):

QVQLVQSGAEVKKPGASVKVSCKASGYTFTSYWMHWVRQAPGQGLEWIGMIHPN SGSTNYNEKFKSRVTMTVDTSTSTAYMELSSLRSEDTAVYYCARRYYGSISPDYWGQGTT VTVSS (SEQ ID NO: 70).

Humanized 1E7 antibody heavy chain variable region 4 (1E7-huVH4):

QVQLVQSGAEVKKPGASVKVSCKASGYTFTSYWMHWVRQAPGQGLEWMGMIHP NSGSTNYNEKFKSRVTMTVDTSTSTVYMELSSLRSEDTAVYYCARRYYGSISPDYWGQG TTVTVSS (SEQ ID NO: 71).

Humanized 1E7 antibody heavy chain variable region 5 (1E7-huVH5):

QVQLVQSGAEVKKPGASVKVSCKASGYTFTSYWMHWVRQAPGQGLEWMGMIHP NSGSTNYAQKFQGRVTMTRDTSTSTVYMELSSLRSEDTAVYYCARRYYGSISPDYWGQG TTVTVSS (SEQ ID NO: 72).

Human light chain constant region LC (Kappa):

RTVAAPSVFIFPPSDEQLKSGTASVVCLLNNFYPREAKVQWKVDNALQSGNSQESVT

EQDSKDSTYSLSSTLTLSKADYEKHKVYACEVTHQGLSSPVTKSFNRGEC (SEQ ID NO: 73).

Human IgG1 heavy chain constant region HC:

ASTKGPSVFPLAPSSKSTSGGTAALGCLVKDYFPEPVTVSWNSGALTSGVHTFPAVL

QSSGLYSLSSVVTVPSSSLGTQTYICNVNHKPSNTKVDKKVEPKSCDKTHTCPPCPAPELL

GGPSVFLFPPKPKDTLMISRTPEVTCVVVDVSHEDPEVKFNWYVDGVEVHNAKTKPREE

QYNSTYRVVSVLTVLHQDWLNGKEYKCKVSNKALPAPIEKTISKAKGQPREPQVYTLPPS

RDELTKNQVSLTCLVKGFYPSDIAVEWESNGQPENNYKTTPPVLDSDGSFFLYSKLTVDKS

RWQQGNVFSCSVMHEALHNHYTQKSLSLSPGK (SEQ ID NO: 74).

Human IgG4 heavy chain constant region HC:

ASTKGPSVFPLAPCSRSTSESTAALGCLVKDYFPEPVTVSWNSGALTSGVHTFPAVLQ

SSGLYSLSSVVTVPSSSLGTKTYTCNVDHKPSNTKVDKRVESKYGPPCPPCPAPEFLGGPS

VFLFPPKPKDTLMISRTPEVTCVVVDVSQEDPEVQFNWYVDGVEVHNAKTKPREEQFNS

TYRVVSVLTVLHQDWLNGKEYKCKVSNKGLPSSIEKTISKAKGQPREPQVYTLPPSQEE

MTKNQVSLTCLVKGFYPSDIAVEWESNGQPENNYKTTPPVLDSDGSFFLYSRLTVDKSRW

QEGNVFSCSVMHEALHNHYTQKSLSLSLGK (SEQ ID NO: 75).

**Example 2 Preparation and identification of humanized antibodies**

According to the results of the humanized design in Example 1, the sequence was constructed into two subtypes of pcDNA3.4 vectors, and plasmids were obtained through PCR, restriction digestion, ligation, transformation, identification, sequencing, alignment, and extraction. According to the expression combination recommended after humanization design in Example 1 (Table 1), the above plasmid was expressed in CHO cells for 7 days as required using the ExpiCHO-S expression system, and the expression level was measured by the Gator label-free bioanalyzer on the 6th day of expression. On the 7th day, the CHO cell expression fluid was collected, centrifuged, filtered with a filter, and the supernatant was taken. The recombinant antibody was purified using Protein-A affinity chromatography. The protein samples obtained by the Protein A column were identified by SDS-PAGE. The specific experimental results are shown in Figures 1-8. The purity of the reduced band was calculated by ImageJ software according to the peak area normalization method. Compared with the reference product IPI, the non-reduced band molecular weight is about 150 kDa, and the purity is greater than 90%; the reduced heavy chain molecular weight is about 55 kDa, the light chain molecular weight is about 25 kDa, and the purity of the heavy chain plus the light chain is greater than 90%. All humanized designed antibodies meet the characteristics of antibody molecular weight and have good purity, meeting the drugability requirements.

Table 1:

| Sample name | The heavy chain amino acid sequence | The light chain amino acid sequence |
|---|---|---|
| 1B6-huVH1-HC (IgG1) huVL1 | SEQ ID NO: 79 | SEQ ID NO: 76 |
| 1B6-huVH1-HC (IgG1) huVL2 | SEQ ID NO: 79 | SEQ ID NO: 77 |
| 1B6-huVH2-HC (IgG1) huVL1 | SEQ ID NO: 80 | SEQ ID NO: 76 |

(continued)

| Sample name | The heavy chain amino acid sequence | The light chain amino acid sequence |
|---|---|---|
| 1B6-huVH2-HC (IgG1) huVL2 | SEQ ID NO: 80 | SEQ ID NO: 77 |
| 1B6-huVH3-HC (IgG1) huVL1 | SEQ ID NO: 81 | SEQ ID NO: 76 |
| 1B6-huVH3-HC (IgG1) huVL2 | SEQ ID NO: 81 | SEQ ID NO: 77 |
| 1B6-huVH3-HC (IgG1) huVL3 | SEQ ID NO: 81 | SEQ ID NO: 78 |
| 1B6-huVH4-HC (IgG1) huVL2 | SEQ ID NO: 82 | SEQ ID NO: 77 |
| 1B6-huVH4-HC (IgG1) huVL3 | SEQ ID NO: 82 | SEQ ID NO: 78 |
| 1B6-huVH5-HC (IgG1) huVL2 | SEQ ID NO: 83 | SEQ ID NO: 77 |
| 1B6-huVH5-HC (IgG1) huVL3 | SEQ ID NO: 83 | SEQ ID NO: 78 |
| 1E7-huVH1-HC (IgG1) huVL1 | SEQ ID NO: 92 | SEQ ID NO: 89 |
| 1E7-huVH1-HC (IgG1) huVL2 | SEQ ID NO: 92 | SEQ ID NO: 90 |
| 1E7-huVH2-HC (IgG1) huVL1 | SEQ ID NO: 93 | SEQ ID NO: 89 |
| 1E7-huVH2-HC (IgG1) huVL2 | SEQ ID NO: 93 | SEQ ID NO: 90 |
| 1E7-huVH3-HC (IgG1) huVL1 | SEQ ID NO: 94 | SEQ ID NO: 89 |
| 1E7-huVH3-HC (IgG1) huVL2 | SEQ ID NO: 94 | SEQ ID NO: 90 |
| 1E7-huVH3-HC (IgG1) huVL3 | SEQ ID NO: 94 | SEQ ID NO: 91 |
| 1E7-huVH4-HC (IgG1) huVL2 | SEQ ID NO: 95 | SEQ ID NO: 90 |
| 1E7-huVH4-HC (IgG1) huVL3 | SEQ ID NO: 95 | SEQ ID NO: 91 |
| 1E7-huVH5-HC (IgG1) huVL2 | SEQ ID NO: 96 | SEQ ID NO: 90 |
| 1E7-huVH5-HC (IgG1) huVL3 | SEQ ID NO: 96 | SEQ ID NO: 91 |
| 1B6-huVH1huVL1 | SEQ ID NO: 84 | SEQ ID NO: 76 |
| 1B6-huVH1huVL2 | SEQ ID NO: 84 | SEQ ID NO: 77 |
| 1B6-huVH2huVL1 | SEQ ID NO: 85 | SEQ ID NO: 76 |
| 1B6-huVH2huVL2 | SEQ ID NO: 85 | SEQ ID NO: 77 |
| 1B6-huVH3huVL1 | SEQ ID NO: 86 | SEQ ID NO: 76 |
| 1B6-huVH3huVL2 | SEQ ID NO: 86 | SEQ ID NO: 77 |
| 1B6-huVH3huVL3 | SEQ ID NO: 86 | SEQ ID NO: 78 |
| 1B6-huVH4huVL2 | SEQ ID NO: 87 | SEQ ID NO: 77 |
| 1B6-huVH4huVL3 | SEQ ID NO: 87 | SEQ ID NO: 78 |
| 1B6-huVH5huVL2 | SEQ ID NO: 88 | SEQ ID NO: 77 |
| 1B6-huVH5huVL3 | SEQ ID NO: 88 | SEQ ID NO: 78 |
| 1E7-huVH1huVL1 | SEQ ID NO: 97 | SEQ ID NO: 89 |
| 1E7-huVH1huVL2 | SEQ ID NO: 97 | SEQ ID NO: 90 |
| 1E7-huVH2huVL1 | SEQ ID NO: 98 | SEQ ID NO: 89 |
| 1E7-huVH2huVL2 | SEQ ID NO: 98 | SEQ ID NO: 90 |
| 1E7-huVH3huVL1 | SEQ ID NO: 99 | SEQ ID NO: 89 |
| 1E7-huVH3huVL2 | SEQ ID NO: 99 | SEQ ID NO: 90 |
| 1E7-huVH3huVL3 | SEQ ID NO: 99 | SEQ ID NO: 91 |
| 1E7-huVH4huVL2 | SEQ ID NO: 100 | SEQ ID NO: 90 |
| 1E7-huVH4huVL3 | SEQ ID NO: 100 | SEQ ID NO: 91 |

(continued)

| Sample name | The heavy chain amino acid sequence | The light chain amino acid sequence |
| --- | --- | --- |
| 1E7-huVH5huVL2 | SEQ ID NO: 101 | SEQ ID NO: 90 |
| 1E7-huVH5huVL3 | SEQ ID NO: 101 | SEQ ID NO: 91 |

**Example 3 Assessment of aggregate content of humanized antibodies (SEC)**

The inventors collected data using an Agilent HPLC 1100 instrument, an XBridge BEH200Å, SEC 3.5 $\mu$m, 7.8 × 300 mm (batch number: ZL20200823) chromatographic column, with a mobile phase of 0.15M PB (pH 7.4), 20 $\mu$L of sample was loaded at a flow rate of 0.8 mL/min, and the detector (parameters: detection wavelength 280 nm, bandwidth 16 nm, reference wavelength 360 nm, bandwidth 100 nm, peak width (response time) >0.1 min (2 s); slit 4 nm; negative absorbance baseline 100 mAU). The specific data are shown in Table 2. Except for antibody 1E7-huVH5huVL3 (with a monomer ratio of 67.67%), the monomer ratios of the remaining antibodies are all greater than 90%. The monomer ratios of most humanized antibodies are better or equivalent to those of chimeric antibodies, indicating that the humanized designed antibodies have good uniformity and meet the drugability requirements.

Table 2:

| Sample name | Monomer retention time (min) | High polymer (%) | Monomer (%) | Low molecular weight substance (%) |
| --- | --- | --- | --- | --- |
| 1B6-mVH-HC (IgG1) mVL | 8.64 | 1.94 | 98.06 | / |
| 1B6-huVH1-HC (IgG1) huVL1 | 8.61 | 1.96 | 97.34 | 0.70 |
| 1B6-huVH1-HC (IgG1) huVL2 | 8.59 | 1.06 | 98.94 | / |
| 1B6-huVH2-HC (IgG1) huVL1 | 8.66 | 1.01 | 98.56 | 0.43 |
| 1B6-huVH2-HC (IgG1) huVL2 | 8.64 | 1.13 | 98.87 | / |
| 1B6-huVH3-HC (IgG1) huVL1 | 8.66 | 1.42 | 98.17 | 0.41 |
| 1B6-huVH3-HC (IgG1) huVL2 | 8.64 | 1.41 | 98.08 | 0.50 |
| 1B6-huVH3-HC (IgG1) huVL3 | 8.64 | 1.62 | 98.38 | / |
| 1B6-huVH4-HC (IgG1) huVL2 | 8.68 | 1.65 | 98.35 | / |
| 1B6-huVH4-HC (IgG1) huVL3 | 8.69 | 1.29 | 98.71 | / |
| 1B6-huVH5-HC (IgG1) huVL2 | 8.68 | 1.54 | 97.89 | 0.57 |
| 1B6-huVH5-HC (IgG1) huVL3 | 8.69 | 1.13 | 98.46 | 0.41 |
| 1E7-mVH-HC (IgG1) mVL | 8.69 | 1.35 | 98.65 | / |
| 1E7-huVH1-HC (IgG1) huVL1 | 8.68 | 0.39 | 99.14 | 0.47 |
| 1E7-huVH1-HC (IgG1) huVL2 | 8.66 | 0.47 | 99.53 | / |
| 1E7-huVH2-HC (IgG1) huVL1 | 8.72 | 0.32 | 99.68 | / |
| 1E7-huVH2-HC (IgG1) huVL2 | 8.70 | 1.16 | 98.55 | 0.30 |
| 1E7-huVH3-HC (IgG1) huVL1 | 8.73 | 0.40 | 99.26 | 0.34 |
| 1E7-huVH3-HC (IgG1) huVL2 | 8.71 | 0.37 | 99.43 | 0.20 |
| 1E7-huVH3-HC (IgG1) huVL3 | 8.72 | 0.58 | 99.42 | / |
| 1E7-huVH4-HC (IgG1) huVL2 | 8.73 | 0.39 | 99.21 | 0.40 |
| 1E7-huVH4-HC (IgG1) huVL3 | 8.74 | 0.69 | 98.79 | 0.52 |
| 1E7-huVH5-HC (IgG1) huVL2 | 8.77 | 4.16 | 90.37 | 5.46 |
| 1E7-huVH5-HC (IgG1) huVL3 | 8.77 | 0.52 | 98.44 | 1.04 |
| 1B6-mVHmVL | 8.74 | 1.22 | 98.78 | / |
| 1B6-huVH1huVL1 | 8.69 | 0.97 | 99.03 | / |

(continued)

| Sample name | Monomer retention time (min) | High polymer (%) | Monomer (%) | Low molecular weight substance (%) |
|---|---|---|---|---|
| 1B6-huVH1huVL2 | 8.68 | 0.98 | 99.02 | / |
| 1B6-huVH2huVL1 | 8.74 | 1.00 | 99.00 | / |
| 1B6-huVH2huVL2 | 8.73 | 0.66 | 99.34 | / |
| 1B6-huVH3huVL1 | 8.74 | 1.07 | 98.93 | / |
| 1B6-huVH3huVL2 | 8.73 | 0.41 | 99.59 | / |
| 1B6-huVH3huVL3 | 8.74 | 1.60 | 98.40 | / |
| 1B6-huVH4huVL2 | 8.77 | 1.70 | 98.30 | / |
| 1B6-huVH4huVL3 | 8.78 | 1.94 | 98.06 | / |
| 1B6-huVH5huVL2 | 8.77 | 0.90 | 99.10 | / |
| 1B6-huVH5huVL3 | 8.78 | 4.18 | 95.82 | / |
| 1E7-mVHmVL | 8.79 | 1.43 | 98.57 | / |
| 1E7-huVH1huVL1 | 8.75 | 1.53 | 98.47 | / |
| 1E7-huVH1huVL2 | 8.73 | 1.07 | 98.93 | / |
| 1E7-huVH2huVL1 | 8.79 | 0.78 | 99.22 | / |
| 1E7-huVH2huVL2 | 8.77 | 1.19 | 98.81 | / |
| 1E7-huVH3huVL1 | 8.80 | 5.34 | 94.66 | / |
| 1E7-huVH3huVL2 | 8.78 | 1.89 | 98.11 | / |
| 1E7-huVH3huVL3 | 8.79 | 2.33 | 97.67 | / |
| 1E7-huVH4huVL2 | 8.79 | 1.04 | 98.96 | / |
| 1E7-huVH4huVL3 | 8.80 | 1.05 | 98.95 | / |
| 1E7-huVH5huVL2 | 8.83 | 3.72 | 96.28 | / |
| 1E7-huVH5huVL3 | 8.84 | 32.33 | 67.67 | / |

**Example 4 Thermal stability evaluation of humanized antibodies (DSF)**

In an eight-well tube strip or a 96-well plate, samples diluted to 0.2 mg/mL with 1XPBS (pH7.4) were added, and then 100X SYPRO Orange working solution was added to make the final concentration 5X. The final volume was 20 $\mu$L. The tube wall was flicked to mix, and centrifuged at 2000 rpm for 10 seconds; 3 replicates were prepared for each sample. The samples were placed on an ABI7500 Fast Real-Time PCR instrument, the melting curve was selected as the experimental type, the continuous mode was adopted, the scanning temperature was 25-99 °C, the equilibrium was 25 °C for 5 min, the heating rate was 1%, the reporter group was ROX, and the quencher group was None. Result determination: the temperature corresponding to the first peak and valley of the derivative function of the melting curve was determined as the denaturation temperature Tm1 of the protein, and the temperature corresponding to the second peak and valley was determined as the denaturation temperature Tm2 of the protein. The specific experimental results are shown in Table 3. The minimum Tm values of all antibodies are greater than 64 °C, indicating that the humanized antibodies have good thermal stability and meet the drugability requirements.

Table 3:

| Sample name | Tm 1 (°C) | Tm 2 (°C) |
|---|---|---|
| 1B6-mVH-HC (IgG1) mVL | 77.2 | N/A |
| 1B6-huVH1-HC (IgG1) huVL1 | 68.95 | 77.04 |
| 1B6-huVH1-HC (IgG1) huVL2 | 69.19 | 77.26 |
| 1B6-huVH2-HC (IgG1) huVL1 | 68.76 | 77.94 |

(continued)

| Sample name | Tm 1 (°C) | Tm 2 (°C) |
|---|---|---|
| 1B6-huVH2-HC (IgG1) huVL2 | 68.92 | 77.79 |
| 1B6-huVH3-HC (IgG1) huVL1 | 69.23 | 79.8 |
| 1B6-huVH3-HC (IgG1) huVL2 | 69.28 | 78.88 |
| 1B6-huVH3-HC (IgG1) huVL3 | 69.08 | 77.76 |
| 1B6-huVH4-HC (IgG1) huVL2 | 68.77 | 78.39 |
| 1B6-huVH4-HC (IgG1) huVL3 | 69.27 | 77.61 |
| 1B6-huVH5-HC (IgG1) huVL2 | 74.41 | N/A |
| 1B6-huVH5-HC (IgG1) huVL3 | 73.91 | N/A |
| 1E7-mVH-HC (IgG1) mVL | 75.35 | N/A |
| 1E7-huVH1-HC (IgG1) huVL1 | 69.05 | 75.60 |
| 1E7-huVH1-HC (IgG1) huVL2 | 73.75 | N/A |
| 1E7-huVH2-HC (IgG1) huVL1 | 69.05 | 76.01 |
| 1E7-huVH2-HC (IgG1) huVL2 | 72.92 | N/A |
| 1E7-huVH3-HC (IgG1) huVL1 | 69.08 | 76.08 |
| 1E7-huVH3-HC (IgG1) huVL2 | 74.22 | N/A |
| 1E7-huVH3-HC (IgG1) huVL3 | 73.29 | N/A |
| 1E7-huVH4-HC (IgG1) huVL2 | 73.85 | N/A |
| 1E7-huVH4-HC (IgG1) huVL3 | 72.95 | N/A |
| 1E7-huVH5-HC (IgG1) huVL2 | 71.22 | N/A |
| 1E7-huVH5-HC (IgG1) huVL3 | 70.38 | N/A |
| 1B6-mVHmVL | 65.16 | 75.31 |
| 1B6-huVH1huVL1 | 65.43 | 75.50 |
| 1B6-huVH1huVL2 | 65.40 | 75.65 |
| 1B6-huVH2huVL1 | 65.37 | 76.09 |
| 1B6-huVH2huVL2 | 65.16 | 76.18 |
| 1B6-huVH3huVL1 | 65.09 | 77.02 |
| 1B6-huVH3huVL2 | 64.79 | 76.18 |
| 1B6-huVH3huVL3 | 64.72 | 75.37 |
| 1B6-huVH4huVL2 | 65.06 | 76.46 |
| 1B6-huVH4huVL3 | 65.40 | 76.09 |
| 1B6-huVH5huVL2 | 65.56 | 73.64 |
| 1B6-huVH5huVL3 | 65.68 | 73.01 |
| 1E7-mVHmVL | 65.19 | 74.57 |
| 1E7-huVH1huVL1 | 65.13 | 74.04 |
| 1E7-huVH1huVL2 | 65.19 | 72.48 |
| 1E7-huVH2huVL1 | 64.75 | 74.35 |
| 1E7-huVH2huVL2 | 72.45 | N/A |
| 1E7-huVH3huVL1 | 65.06 | 74.50 |
| 1E7-huVH3huVL2 | 64.69 | 73.42 |

(continued)

| Sample name | Tm 1 (°C) | Tm 2 (°C) |
|---|---|---|
| 1E7-huVH3huVL3 | 64.20 | 72.67 |
| 1E7-huVH4huVL2 | 65.53 | 73.11 |
| 1E7-huVH4huVL3 | 65.22 | 72.49 |
| 1E7-huVH5huVL2 | 71.28 | N/A |
| 1E7-huVH5huVL3 | 65.19 | 74.57 |

**Example 5 Affinity evaluation of humanized antibodies by ELISA**

ELISA plates were coated with claudin-18.2 antigen (manufacturer: GenScript, batch number: R50092011) at 2 μg/mL, 30 μL per well, and incubated overnight at 4 °C. The ELISA plates were washed three times with PBST and blocked with 5% PBS-milk for 2 h at room temperature. The ELISA plate was washed three times with PBST, and the diluted antibodies were added to the ELISA plate, 30 μL per well, and incubated at room temperature for 60 min. The plates were washed three times with PBST and incubated with anti-human IgG-Fc-HRP (abcam, ab97225) for 50 min at room temperature. The plate was washed 12 times with PBST, and TMB was added, 30 μL per well. The reaction was terminated with stop solution and OD450 was read. The specific experimental results are shown in Figures 9-12. The ELISA affinity EC50 was calculated. The specific experimental data are shown in Table 4, wherein the ELISA affinities of all antibodies were below 1.25 μg/mL, indicating that the humanized antibodies met the drugability requirements.

Table 4:

| Sample name | EC50 (μg/mL) |
|---|---|
| 1E7-huVH1-HC (IgG1) huVL1 | 0.04788 |
| 1E7-huVH1-HC (IgG1) huVL2 | 0.03047 |
| 1E7-huVH2-HC (IgG1) huVL1 | 0.1114 |
| 1E7-huVH2-HC (IgG1) huVL2 | 0.05551 |
| 1E7-huVH3-HC (IgG1) huVL1 | 0.06634 |
| 1E7-huVH3-HC (IgG1) huVL2 | 0.05757 |
| 1E7-huVH3-HC (IgG1) huVL3 | 0.07069 |
| 1E7-huVH4-HC (IgG1) huVL2 | 0.03966 |
| 1E7-huVH4-HC (IgG1) huVL3 | 0.04997 |
| 1E7-huVH5-HC (IgG1) huVL2 | 0.1406 |
| 1E7-huVH5-HC (IgG1) huVL3 | 0.1301 |
| 1E7-huVH1huVL1 | 0.3093 |
| 1E7-huVH1huVL2 | 0.156 |
| 1E7-huVH2huVL1 | 0.4359 |
| 1E7-huVH2huVL2 | 0.2617 |
| 1huVL1 | 0.6061 |
| 1E7-huVH3huVL2 | 0.5605 |
| 1E7-huVH3huVL3 | 0.5089 |
| 1E7-huVH4huVL2 | 0.3091 |
| 1E7-huVH4huVL3 | 0.4412 |
| 1E7-huVH5huVL2 | 0.4965 |
| 1E7-huVH5huVL3 | 0.09306 |
| 1B6-huVH1-HC (IgG1) huVL1 | 0.02478 |

(continued)

| Sample name | EC50 (μg/mL) |
|---|---|
| 1B6-huVH1-HC (IgG1) huVL2 | 0.01621 |
| 1B6-huVH2-HC (IgG1) huVL1 | 0.04025 |
| 1B6-huVH2-HC (IgG1) huVL2 | 0.03015 |
| 1B6-huVH3-HC (IgG1) huVL1 | 0.05919 |
| 1B6-huVH3-HC (IgG1) huVL2 | 0.06137 |
| 1B6-huVH3-HC (IgG1) huVL3 | 0.07251 |
| 1B6-huVH4-HC (IgG1) huVL2 | 0.04313 |
| 1B6-huVH4-HC (IgG1) huVL3 | 0.05188 |
| 1B6-huVH5-HC (IgG1) huVL2 | 0.02978 |
| 1B6-huVH5-HC (IgG1) huVL3 | 0.03496 |
| 1B6-huVH1huVL1 | 0.08392 |
| 1B6-huVH1huVL2 | 0.07751 |
| 1B6-huVH2huVL1 | 0.09779 |
| 1B6-huVH2huVL2 | 0.1918 |
| 1B6-huVH3huVL1 | 0.0569 |
| 1B6-huVH3huVL2 | 0.245 |
| 1B6-huVH3huVL3 | 0.2094 |
| 1B6-huVH4huVL2 | 0.2101 |
| 1B6-huVH4huVL3 | 0.3457 |
| 1B6-huVHShuVL2 | 0.2018 |
| 1B6-huVHShuVL3 | 0.1744 |

**Example 6 Affinity kinetics evaluation of humanized antibodies**

The GATOR instrument and associated software were opened and the Kientics experimental mode was selected. The affinity kinetics of the humanized antibody was evaluated according to the analysis program shown in Table 5. After data collection, Kientics fitting was performed to calculate the affinity kinetic parameters. The correlation coefficient $R^2$ of all antibodies in the Global fitting mode was greater than 0.95, which met the system suitability requirements and the results were reliable. The experimental data of affinity kinetics testing of humanized antibodies are shown in Table 6, wherein all antibody KD values are between E-08 and E-11, indicating that humanized antibodies meet the drugability requirements, and some humanized antibodies have KD values comparable to or even better than mouse antibodies.

Table 5:

| Assay step | Assay time (s) | Sample |
|---|---|---|
| Baseline1 | 60 sec | Q buffer |
| Loading | 120 sec | 30 nM antibody in Q buffer |
| Baseline2 | 60 sec | Q buffer |
| Association | 120 sec | 2-fold diluted antigen from 2000 nM to 500 nM with Q buffer |
| Dissociation | 230 sec | Q buffer |
| Regeneration | 25 sec | Q buffer |

Table 6:

| Sample name | KD (M) | ka (1/Ms) | kd (1/s) | $R^2$ |
|---|---|---|---|---|
| 1B6-mVH-HC (IgG1) mVL | 4.44E-08 | 1.78E+04 | 7.92E-04 | 0.992 |
| 1B6-huVH1-HC (IgG1) huVL1 | 6.33E-08 | 1.94E+04 | 1.23E-03 | 0.990 |
| 1B6-huVH1-HC (IgG1) huVL2 | 8.67E-08 | 1.97E+04 | 1.71E-03 | 0.990 |
| 1B6-huVH2-HC (IgG1) huVL1 | 9.84E-09 | 1.03E+04 | 1.01E-04 | 0.996 |
| 1B6-huVH2-HC (IgG1) huVL2 | 1.18E-08 | 1.10E+04 | 1.30E-04 | 0.996 |
| 1B6-huVH3-HC (IgG1) huVL1 | 7.65E-09 | 1.02E+04 | 7.83E-05 | 0.996 |
| 1B6-huVH3-HC (IgG1) huVL2 | 1.09E-08 | 1.11E+04 | 1.20E-04 | 0.996 |
| 1B6-huVH3-HC (IgG1) huVL3 | 9.64E-09 | 1.21E+04 | 1.16E-04 | 0.996 |
| 1B6-huVH4-HC (IgG1) huVL2 | 2.05E-08 | 1.01E+04 | 2.07E-04 | 0.996 |
| 1B6-huVH4-HC (IgG1) huVL3 | 4.39E-08 | 1.82E+04 | 7.99E-04 | 0.99 |
| 1B6-huVH5-HC (IgG1) huVL2 | 5.97E-08 | 2.16E+04 | 1.29E-03 | 0.987 |
| 1B6-huVH5-HC (IgG1) huVL3 | 7.68E-08 | 2.27E+04 | 1.75E-03 | 0.989 |
| 1B6-mVHmVL | 8.42E-08 | 2.23E+04 | 1.88E-03 | 0.989 |
| 1B6-huVH1huVL1 | 9.88E-08 | 2.24E+04 | 2.22E-03 | 0.989 |
| 1B6-huVH1huVL2 | 1.06E-08 | 1.55E+04 | 1.64E-04 | 0.996 |
| 1B6-huVH2huVL1 | 4.57E-09 | 1.39E+04 | 6.36E-05 | 0.995 |
| 1B6-huVH2huVL2 | 4.86E-09 | 1.39E+04 | 6.78E-05 | 0.996 |
| 1B6-huVH3huVL1 | 9.80E-10 | 1.30E+04 | 1.27E-05 | 0.996 |
| 1B6-huVH3huVL2 | 1.27E-08 | 9.48E+03 | 1.21E-04 | 0.997 |
| 1B6-huVH3huVL3 | 8.11E-09 | 1.06E+04 | 8.56E-05 | 0.996 |
| 1B6-huVH4huVL2 | 7.06E-09 | 9.99E+03 | 7.06E-05 | 0.996 |
| 1B6-huVH4huVL3 | 7.24E-09 | 1.11E+04 | 8.07E-05 | 0.996 |
| 1B6-huVH5huVL2 | 1.06E-08 | 1.36E+04 | 1.44E-04 | 0.996 |
| 1B6-huVH5huVL3 | 7.51E-09 | 1.30E+04 | 9.78E-05 | 0.996 |
| 1E7-mVHmVL | 2.66E-09 | 1.02E+04 | 2.71E-05 | 0.995 |
| 1E7-huVH1huVL1 | 3.58E-09 | 1.06E+04 | 3.81E-05 | 0.995 |
| 1E7-huVH1huVL2 | 5.08E-09 | 1.09E+04 | 5.56E-05 | 0.995 |
| 1E7-huVH2huVL1 | 2.77E-10 | 9.55E+03 | 2.65E-06 | 0.995 |
| 1E7-huVH2huVL2 | 2.16E-08 | 1.24E+04 | 2.69E-04 | 0.992 |
| 1huVL1 | 1.20E-08 | 1.10E+04 | 1.32E-04 | 0.995 |
| 1E7-huVH3huVL2 | 2.02E-09 | 1.03E+04 | 2.08E-05 | 0.995 |
| 1E7-huVH3huVL3 | 1.35E-08 | 1.10E+04 | 1.49E-04 | 0.995 |
| 1E7-huVH4huVL2 | 4.60E-09 | 1.06E+04 | 4.86E-05 | 0.995 |
| 1E7-huVH4huVL3 | 6.19E-09 | 1.01E+04 | 6.27E-05 | 0.995 |
| 1E7-huVH5huVL2 | 5.24E-09 | 1.01E+04 | 5.29E-05 | 0.996 |
| 1E7-huVH5huVL3 | 3.21E-09 | 9.61E+03 | 3.09E-05 | 0.995 |
| 1E7-mVH-HC (IgG1) mVL | 3.82E-09 | 1.17E+04 | 4.48E-05 | 0.995 |
| 1E7-huVH1-HC (IgG1) huVL1 | 4.90E-09 | 1.22E+04 | 5.97E-05 | 0.995 |
| 1E7-huVH1-HC (IgG1) huVL2 | 4.85E-09 | 1.21E+04 | 5.87E-05 | 0.995 |

(continued)

| Sample name | KD (M) | ka (1/Ms) | kd (1/s) | $R^2$ |
|---|---|---|---|---|
| 1E7-huVH2-HC (IgG1) huVL1 | 1.82E-09 | 1.09E+04 | 1.97E-05 | 0.995 |
| 1E7-huVH2-HC (IgG1) huVL2 | 2.89E-09 | 1.10E+04 | 3.18E-05 | 0.995 |
| 1E7-huVH3-HC (IgG1) huVL1 | 3.50E-10 | 1.03E+04 | 3.60E-06 | 0.995 |
| 1E7-huVH3-HC (IgG1) huVL2 | 2.89E-10 | 1.02E+04 | 2.94E-06 | 0.995 |
| 1E7-huVH3-HC (IgG1) huVL3 | 1.16E-08 | 1.13E+04 | 1.32E-04 | 0.994 |
| 1E7-huVH4-HC (IgG1) huVL2 | 1.07E-08 | 1.18E+04 | 1.26E-04 | 0.994 |
| 1E7-huVH4-HC (IgG1) huVL3 | 2.64E-09 | 1.13E+04 | 2.99E-05 | 0.995 |
| 1E7-huVH5-HC (IgG1) huVL2 | 1.31E-08 | 1.28E+04 | 1.67E-04 | 0.993 |
| 1E7-huVH5-HC (IgG1) huVL3 | 9.29E-10 | 1.06E+04 | 9.83E-06 | 0.995 |

**Example 7 Evaluation of ADCC activity of humanized antibodies**

KATOIII-Claudin18.2 cells (target cells) were cultured in RPMI1640 (Gibco, 22400-089) medium containing 10% FBS (Gibco, 10099-141C) and 0.5 μg/mL puromycin (Gibco, A11138-03). The cells were collected in the logarithmic growth phase, washed thoroughly with PBS (Hyclone, SHS30256.01), and resuspended in RPMI1640 medium containing 1% FBS and 1% double antibody (Hyclone, SV30010). The cell concentration was adjusted to $4 \times 10^5$ cells per ml and 5000 cells were plated in each well of a white bottom opaque 384-well microplate (Corning, 3570). The humanized antibody was diluted in a 4-fold gradient from 3.33 μg/mL to prepare 10 concentrations, and 25 μL per well was added to the cell wells. Jurkat-NFAT-Luc2/CD16 (effector cells) were cultured in RPMI1640 medium containing 10% FBS and 1% double antibody. The cells were collected in the logarithmic growth phase, washed thoroughly with PBS, and resuspended in RPMI1640 medium containing 1% FBS and 1% double antibody. The cell concentration was adjusted to $2 \times 10^6$ cells per ml and 20,000 cells per well were added to the cell wells. The 384-well microplate was placed in a 7°C, 5% $CO_2$ incubator for 12 h, 30 μL One-GloTM Luciferase detection reagent (Promega, E6120) was added to each well, and the optical luminescence signal was read using a multi-function microplate reader (SpectraMax).

The experimental results of ADCC activity evaluation of 1B6 partially humanized antibody and murine antibody are shown in figure 13, wherein the ADCC activity EC50 of the humanized antibody is not much different from that of the murine antibody, and the highest value of ADCC activity of the partially humanized antibody is comparable to that of the chimeric antibody, or even better. The results of ADCC activity evaluation of 1E7 partially humanized antibody and chimeric antibody are shown in figure 14, wherein the ADCC activity EC50 of the humanized antibody is not much different from that of the chimeric antibody, and the highest value of ADCC activity of the partially humanized antibody is comparable to that of the chimeric antibody, or even better.

**Example 8 Evaluation of CDC activity of humanized antibodies**

KATOIII-claudin18.2 cells were cultured in RPMI1640 (Gibco, 22400-089) medium containing 10% FBS (Gibco, 10099-141C) and 0.5 μg/mL puromycin (Gibco, A11138-03). The cells were collected in the logarithmic growth phase, washed thoroughly with PBS (Hyclone, SHS30256.01), and resuspended in RPMI1640 medium containing 2.5% human serum complement components (Quidel, A113) and 1% double antibody (Hyclone, SV30010). The cell concentration was adjusted to $3 \times 10^5$ cells per ml and 100μL was plated per well in a 96-well cell culture plate (Corning, 3599). The humanized antibody was diluted 4-fold from 270 μg/mL using RPMI1640 medium containing 2.5% human serum complement components and 1% double antibody to prepare 10 concentrations, and 100 μL per well was added to the cell wells. The 384-well microplate was placed in a 37°C, 5% $CO_2$ incubator for 4 h, 50 μL of 40% CCK-8 detection reagent (Japan Tongren, CK04) was added to each well, and the incubation continued for 3 h. The OD450 was read using a multi-function microplate reader (SpectraMax).

The results of CDC activity evaluation of 1B6 partially humanized antibody and chimeric antibody are shown in figure 15, wherein the CDC activity EC50 of the humanized antibody is not much different from that of the chimeric antibody, and the highest value of CDC activity of the partially humanized antibody is comparable to that of the chimeric antibody, or even better. The results of CDC activity evaluation of 1E7 partially humanized antibody and chimeric antibody are shown in figure 16, wherein the CDC activity EC50 of the humanized antibody is not much different from that of the chimeric antibody, and the highest value of CDC activity of the partially humanized antibody is comparable to that of the chimeric antibody, or even better.

**Example 9 Evaluation of internalization of humanized Claudin18.2 antibody using FACS with Zenon™ pHrodo™ iFL IgG Labeling Reagent**

Claudin18.2 stable expression cell line A20-18.2 (target cells) was constructed and cultured in 1640 complete medium (Gibco, 22400-089) containing 10% FBS (Gibco, 10099-141C), 0.05mM $\beta$-mercaptoethanol (Aladdin, M301574) and 1 $\mu$g/ml puromycin (Gibco, A11138-03). When the cells grew to about 80% of the entire culture dish, the cells were centrifuged and the viability was measured with trypan blue to ensure that the cell viability was above 95%. The cells were counted using an automatic cell counter and the cell density was adjusted to 2*10^6 cells/mL. The antibody to be tested was prepared using 1640 complete medium, and the final concentration of the antibody to be tested was 2 $\mu$g/mL. Zenon™ pHrodo™ iFL IgG Labeling Reagent (Invitrogen, Z25611, Z25612) was prepared using 1640 complete medium at a final concentration of 40 nM. The antibody to be tested of 25 $\mu$L and Zenon™ pHrodo™ iFL IgG Labeling Reagent of 25 $\mu$L were added to a 96-well plate, and the 96-well plate was incubated for 5 min at room temperature. To each well was added A20-18.2 cells in 50 $\mu$L, with a cell volume of 1*10^5 cells per well. The 96-well plate was placed in an incubator at 37°C and 5% $CO_2$ for 4 h. The remaining antibodies or reagents were washed away with PBS. The antibody internalization was detected by flow cytometry, and the proportion of cells that underwent internalization was counted.

The results are shown in Table 7 and Figures 17A-C. Claudin18.2 murine monoclonal antibody 1B6 underwent obvious antibody internalization, with the maximum internalized cell ratio reaching 92.83%. Under the same conditions, after humanization of 1B6 antibody, the internalization ratio of the antibody decreased to varying degrees, and the degree of reduction was related to the IgG subtype of the antibody and the degree of humanization. The murine monoclonal antibody 1E7 showed no obvious antibody internalization, and antibody internalization still did not occur after humanization.

Table 7: Fluorescence intensity of internalization detection of Claudin18.2 humanized monoclonal antibody and the proportion of internalized cells

| Antibody | Antibody concentration ($\mu$g/mL) | The fluorescence intensity (MFI) | The proportion of internalized cells (%) |
|---|---|---|---|
| cells | 0 | 2044 | 0.08% |
| 1B6-mVH-HC (IgG1) mVL | 2 | 10504 | 83.75% |
| 1B6-huVH4huVL3 | 2 | 3817 | 10.50% |
| 1E7-mVH-HC (IgG1) mVL | 2 | 3485 | 7.07% |
| 1E7-huVH3huVL1 | 2 | 2754 | 2.07% |
| 1E7-huVH3huVL2 | 2 | 2595 | 1.44% |
| 1E7-huVH1huVL1 | 2 | 3078 | 3.86% |
| 1E7-huVH2huVL2 | 2 | 2643 | 1.54% |
| cells | 0 | 844 | 0.02% |
| 1B6-mVH-HC (IgG1) mVL | 2 | 11592 | 92.83% |
| 1B6-huVH3huVL1 | 2 | 6303 | 72.40% |
| 1B6-huVH4-HC (IgG1) huVL3 | 2 | 6993 | 73.78% |
| 1B6-huVH3-HC (IgG1) huVL1 | 2 | 10035 | 81.59% |
| 1B6-mVHmVL | 2 | 7446 | 84.14% |
| 1B6-huVH3-HC (IgG1) huVL3 | 2 | 9698 | 80.29% |

**Example 10 Pharmacokinetic evaluation of humanized antibodies in mice**

Eighteen male Babl/c mice were randomly divided into 6 groups, with 3 mice in each group. They were subcutaneously injected with 2 mg/kg of the following: (1) chimeric antibody 1B6 and its humanized antibodies P04891 (1B6-huVH4huVL3) and P04887 (1B6-huVH3huVL1); (2) chimeric antibody 1E7 and its humanized antibodies P04898 (1E7-huVH2huVL2) and P04902 (1E7-huVH4huVL2). Blood was collected at 0.25, 2, 7, 24, 48, 72, 120, 168, 240, 336, 456, and 504 h after administration, and plasma was separated (anticoagulated with EDTA-K2). The concentration of chimeric antibodies and humanized antibodies in each sample was analyzed by FACS. Gradient concentrations of test articles and plasma samples were added to A20 cells stably expressing Claudin18.2, and then FITC-conjugated secondary antibodies (sheep anti-human IgG) were added to bind to the test sample. The cell surface fluorescence intensity was detected by flow cytometry and a fitting curve of antibody concentration-fluorescence intensity was drawn to calculate the drug concentration in plasma.

Pharmacokinetic parameters were calculated based on plasma drug concentrations. The results of main PK parameters were shown in Table 8. The results showed that after subcutaneous injection of chimeric antibody murine 1B6 monoclonal antibody (1B6-mVHmVL) and its humanized antibody P04891 (1B6-huVH4huVL3), P04887 (1B6-huVH3-huVL1) into Babl/c mice, the average plasma half-lives were 70 h, 76 h and 160 h, respectively; the average plasma half-lives of chimeric antibody murine 1E7 monoclonal antibody (1E7-mVHmVL) and its humanized antibody P04898 (1E7-huVH2huVL2), P04902 (1E7-huVH4huVL2) were 111 h, 177 h and 196 h, respectively. Compared with their respective chimeric antibodies, humanized antibodies can significantly extend the half-life of the antibody in the body.

Table 8: Half-life of Claudin18.2 chimeric mAb and its humanized mAb in mice (h)

| Species | Analyte | Group | Dosage | T1/2 (h) | |
| --- | --- | --- | --- | --- | --- |
| | | | | Mean | SD |
| Babl/c Mouse | 1B6 | sc1 | 2.0 mg/kg | 69.9 | 12 |
| | P04891 | sc2 | 2.0 mg/kg | 75.9 | 50 |
| | P04887 | sc3 | 2.0 mg/kg | 160 | 59 |
| | 1E7 | sc4 | 2.0 mg/kg | 111 | 34 |
| | P04898 | sc5 | 2.0 mg/kg | 177 | 110 |
| | P04902 | sc6 | 2.0 mg/kg | 196 | 32 |

**Example 11 Study on the *in vitro* effects of humanized Claudin18.2 antibody CAR-T**

MDA-MB-468-18.2 cells stably expressing Claudin18.2-ΔCS were cultured in DMEM complete medium (containing 10% FBS and 1% Pen/Strep double antibody) and placed in a 37°C, 5% carbon dioxide incubator.

When the tumor cells grew to the required number, the cells were taken in the logarithmic growth phase. The original medium was discarded, and the mixture was trypsinized for 3 minutes. Then, the digestion was terminated with DMEM medium containing 10% FBS, the cells were collected and centrifuged at 400 rpm for 5 min. After cell counting, cells were diluted with 10% FBS DMEM and plated on a flat-bottom 96-well plate, 200 μL per well, and 2E+04 cells per well were set. After the tumor cells adhered for 6 h, the effector-target ratio E/T was set to 0.2, 0.5, and 1.0, that is, the number of CAR-positive cells corresponding to each well was 4E+03, 1E+04, and 2E+04, respectively, and then CART cells were co-incubated with target cells. CTS complete culture was used for killing incubation, and the culture system for each well was 200 μL. Three replicate wells were set for each group, and 3 parallel wells containing only T cells were set for each effector-target ratio.

After co-incubation and plating, the cells were cultured in a 37°C, 5% carbon dioxide incubator. After culturing for 20 h, the killing assay was performed using the LDH method, and the absorbance was measured at 490 nm.

$$\text{Data processing: Positive control (PS) = (target + lysis) - (target + NC)}$$

$$\text{Experimental group killing (EFF) = (E/T=n) - (onlyT, E/T=n)}$$

$$\text{Cytotoxicity} = 100\% * (EFF)/(PS)$$

The results were as shown in Figure 18. Wherein, G1: negative control group, G2: LH2 group, G3: HL2 group, a total of 3 groups. G1 is T cells; G2 and G3 groups are T cells expressing CAR targeting Claudin18.2 molecules, respectively. The VH and VL directions of the scFv sequences in the CAR molecules of G2 and G3 are opposite, wherein the G2 group is connected with VL-GS linker-VH, and the G3 group is connected with VH-GS linker -VL, both of which are humanized 1E7 sequences (VL is IE7-huVL2, the amino acid sequence is shown in SEQ ID NO: 66, and VH is IE7-huVH4, the amino acid sequence is shown in SEQ ID NO: 71).

It can be seen from the figure that the *in vitro* killing effects of the G2 and G3 groups are significantly better than that of the G1 control group.

**Example 12 Study on the *in vivo* effects of humanized Claudin18.2 antibody CAR-T**

MDA-MB-468-18.2 cells stably expressing Claudin18.2-ΔCS were cultured in DMEM complete medium (containing 10% FBS and 1% Pen/Strep double antibody) and placed in a 37°C, 5% carbon dioxide incubator. When the cells grew to the required number, the cells were taken in the logarithmic growth phase. The original medium was discarded, and 1/2 volume of PBS was added for washing, and the PBS was discarded. Then an appropriate amount of 0.05% trypsin was added for digestion for 3 minutes. The digestion was then terminated with complete DMEM medium. The cells were collected and centrifuged at 1000 rpm for 5 min. 0.5 ml of culture medium was used to extract the genome and the mycoplasma test was negative by PCR. After cell counting, the cell density was adjusted to 5E+07/mL with a serum-free DMEM medium and Matrigel mixture (at a ratio of 1:1). Fixed NCG female mice were grabbed, and the cell suspension was injected into the right back of the mouse subcutaneously with 100μL/mouse.

In the MDA-MB-468-18.2 model, when the tumor grew to about 100 mm$^3$, the animals were divided into groups and given medication. There were three groups in total: G1: Vehicle group, G2: LH2 group, and G3: HL2 group. Wherein G1 was the negative control group and was injected with PBS; G2 and G3 respectively expressed CAR targeting Claudin18.2 molecules. The VH and VL directions of the scFv sequences of the CAR molecules of G2 and G3 were opposite. The G2 group was connected with VL-GS linker-VH, and the G3 group was connected with VH-GS linker -VL. Both the VH and VL sequences were obtained after humanization of the original 1E7 sequence (wherein VL is IE7-huVL2, with an amino acid sequence as shown in SEQ ID NO: 66, and VH is IE7-huVH4, with an amino acid sequence as shown in SEQ ID NO: 71).

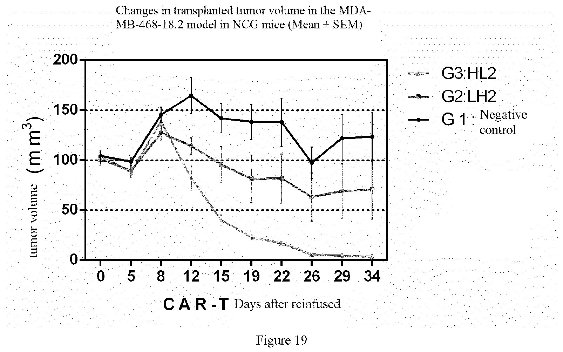

LH2 CAR-T and HL2 CAR-T cells were collected, and the cell density was adjusted to 1.25E+07/ml of CAR-positive cells with PBS solution. Each mouse was administered 200 μL of cell solution through the tail vein, i.e., 2.5E+06 CAR-positive cells. The G1 group was injected with 200 μL of PBS through the tail vein. After the end of administration, the length (L) and width (W) of the tumor were measured twice a week, and the tumor volume (V) was calculated, V=L*W*W*0.5; the tumor growth inhibition rate (TGI) was then calculated. If $T_X > T_0$, TGI=[1-Tx/Cx] * 100%; if $T_X < T_0$, TGI=[1-$(T_X-T_0)/T_0$] * 100%; wherein Tx and Cx are the tumor volumes of the experimental group and the control group on the measurement day, and $T_0$ and $C_0$ are the tumor volumes of the experimental group and the control group on the day of administration. Statistical analysis was performed using GraphPad Prism 6, and the results are shown in Figure 19.

The results show that both LH2 CAR-T and HL2 CAR-T can significantly inhibit tumor growth after intravenous administration in the transplanted tumor model established in NCG mice. At the end of the experiment, the tumor growth inhibition rate of LH2 CAR-T was 131%, and that of HL2 CAR-T was 197%, with both showing significant statistical differences (P<0.01).

Reference throughout this specification to "an embodiment," "some embodiments," "one embodiment", "another example," "an example," "a specific example," or "some examples," means that a particular feature, structure, material, or characteristic described in connection with the embodiment or example is included in at least one embodiment or example of the present disclosure. Thus, the appearances of the above terms throughout this specification are not necessarily referring to the same embodiment or example of the present disclosure. Furthermore, the particular features, structures, materials, or characteristics may be combined in any suitable manner in one or more embodiments or examples. In addition, those skilled in the art can integrate and combine different embodiments, examples or the features of them as long as they are not contradictory to one another.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

**Claims**

1. An antibody or antigen-binding fragment capable of specifically recognizing Claudin18.2, wherein the antibody comprises at least one CDR sequence selected from the following or an amino acid sequence having at least 90% identity with it:

   the CDR sequences of light chain variable region:

   $X_1$SSQSLFNSGNQKNYLT (SEQ ID NO: 1),
   WASTRES (SEQ ID NO: 2),
   QNDYSYPLT (SEQ ID NO: 3),
   $X_4$SSQSLLNSGNQKNYLT (SEQ ID NO: 7),
   WASTRES (SEQ ID NO: 8), and

QNDYSYPFT (SEQ ID NO: 9);

the CDR sequences of heavy chain variable region:

DYNMH (SEQ ID NO: 4),
YINPNNGGTSYX$_2$QKFX$_3$G (SEQ ID NO: 5),
TRYLAV (SEQ ID NO: 6),
SYWMH (SEQ ID NO: 10),
MIHPNSGSTNYX$_5$X$_6$KFX$_7$X$_8$ (SEQ ID NO: 11), and
RYYGSISPDY (SEQ ID NO: 12);

wherein, X$_1$ is K or R, X$_2$ is N or S, X$_3$ is K or Q, X$_4$ is K or R, X$_5$ is N or A, X$_6$ is E or Q, X$_7$ is K or Q, X$_8$ is S or G; wherein, the antibody or antigen-binding fragment thereof has a humanization modification.

2. The antibody or antigen-binding fragment thereof according to claim 1, wherein the antibody comprises:

the light chain variable region CDR1, CDR2, and CDR3 having sequences shown in SEQ ID NO: 1, 2 and 3 respectively or having at least 90% identity with SEQ ID NO: 1, 2 and 3; or
the light chain variable region CDR1, CDR2, and CDR3 having sequences shown in SEQ ID NO: 7, 8 and 9 respectively or having at least 90% identity with SEQ ID NO: 7, 8 and 9.

3. The antibody or antigen-binding fragment thereof according to claim 1, wherein the antibody comprises:

the heavy chain variable region CDR1, CDR2, and CDR3 having sequences shown in SEQ ID NO: 4, 5 and 6 respectively or having at least 90% identity with SEQ ID NO: 4, 5 and 6; or
the heavy chain variable region CDR1, CDR2, and CDR3 having sequences shown in SEQ ID NO: 10, 11 and 12 respectively or having at least 90% identity with SEQ ID NO: 10, 11 and 12.

4. The antibody or antigen-binding fragment thereof according to claim 1, the antibody comprises at least one of a heavy chain framework region sequence and a light chain framework region sequence, wherein at least a part of at least one of the heavy chain framework region sequence and the light chain framework region sequence is derived from at least one of a human antibody or a mutant thereof.

5. The antibody or antigen-binding fragment thereof according to claim 4, wherein the antibody comprises:

the light chain framework region FRL1, FRL2, FRL3, and FRL4 having sequences shown in SEQ ID NO: 13-16 respectively,
DIX$_9$MTQSPSSLX$_{10}$X$_{11}$X$_{12}$X$_{13}$GX$_{14}$X$_{15}$VTX$_{16}$SX$_{17}$C (SEQ ID NO: 13),
WYQQKPGX$_{18}$X$_{19}$PKLLIY (SEQ ID NO: 14),
GVPX$_{20}$DRFX$_{21}$GSGSGTDFTLTISSX$_{22}$QX$_{23}$EDX$_{24}$AX$_{25}$YX$_{26}$C (SEQ ID NO: 15),
FGX$_{27}$GTKLEX$_{28}$K (SEQ ID NO: 16); or
the light chain framework region FRL1, FRL2, FRL3, and FRL4 having sequences shown in SEQ ID NO: 21-24 respectively,
DIX$_{53}$MTQSPSSLSX$_{54}$X$_{55}$AX$_{56}$GX$_{57}$X$_{58}$VTX$_{59}$X$_{60}$C (SEQ ID NO: 21),
WYQQKPGX$_{61}$X$_{62}$PKLLIY (SEQ ID NO: 22),
GVPX$_{63}$RFX$_{64}$GSGSGTDFTLTISSX$_{65}$QX$_{66}$EDX$_{67}$AX$_{68}$YYC (SEQ ID NO: 23),
FGX$_{69}$GTKLEIK (SEQ ID NO: 24),
wherein, X$_9$ is V or Q, X$_{10}$ is S or T, X$_{11}$ is V or A, X$_{12}$ is T or S, X$_{13}$ is A or V, X$_{14}$ is E or D, X$_{15}$ is K or R, X$_{16}$ is M or I, X$_{17}$ is S or T, X$_{18}$ is Q or K, X$_{19}$ is P or A, X$_{20}$ is D or S, X$_{21}$ is T or S, X$_{22}$ is V or L, X$_{23}$ is A or P, X$_{24}$ is L or F, X$_{25}$ is V or T, X$_{26}$ is F or Y, X$_{27}$ is A or G, X$_{28}$ is L or I, X$_{53}$ is V or Q, X$_{54}$ is V or A, X$_{55}$ is T or S, X$_{56}$ is A or V, X$_{57}$ is E or D, X$_{58}$ is K or R, X$_{59}$ is M or I, X$_{60}$ is S or T, X$_{61}$ is Q or K, X$_{62}$ is P or A, X$_{63}$ is D or S, X$_{64}$ is T or S, X$_{65}$ is V or L, X$_{66}$ is A or P, X$_{67}$ is L or F, X$_{68}$ is V or T, X$_{69}$ is S or G;
the condition is that when the antibody comprises: the light chain variable region CDR1, CDR2, and CDR3 sequences shown in KSSQSLFNSGNQKNYLT (SEQ ID NO: 29), WASTRES (SEQ ID NO: 30), and QNDYSYPLT (SEQ ID NO: 31), respectively, X$_9$ is V, X$_{10}$ is T, X$_{11}$ is V, X$_{12}$ is T, X$_{13}$ is A, X$_{14}$ is E, X$_{15}$ is K, X$_{16}$ is M, X$_{17}$ is S, X$_{18}$ is Q, X$_{19}$ is P, X$_{20}$ is D, X$_{21}$ is T, X$_{22}$ is V, X$_{23}$ is A, X$_{24}$ is L, X$_{25}$ is V, X$_{26}$ is F, X$_{27}$ is A, and X$_{28}$ is L, which are not established at the same time;
when the antibody comprises: the light chain variable region CDR1, CDR2, and CDR3 sequences shown in KSSQSLLNSGNQKNYLT (SEQ ID NO: 35), WASTRES (SEQ ID NO: 36), QNDYSYPFT (SEQ ID NO: 37), respectively, $X_{53}$ is V, $X_{54}$ is V, $X_{55}$ is T, $X_{56}$ is A, $X_{57}$ is E, $X_{58}$ is K, $X_{59}$ is M, $X_{60}$ is S, $X_{61}$ is Q, $X_{62}$ is P, $X_{63}$ is D, $X_{64}$ is T, $X_{65}$ is V, $X_{66}$ is A, $X_{67}$ is L, $X_{68}$ is V, and $X_{69}$ is S, which are not established at the same time.

6. The antibody or antigen-binding fragment thereof according to claim 4, wherein the antibody comprises:

the light chain framework region FRL1, FRL2, FRL3, and FRL4 having sequences shown in SEQ ID NO: 41-44 respectively,

$DIX_{90}MTQSPSSLSX_{91}TAGEKVTX_{92}SC$ (SEQ ID NO: 41),

WYQQKPGQPPKLLIY (SEQ ID NO: 42),

$GVPX_{93}DRFTGSGSGTDFTLTISSX_{94}QAEDLAVYX_{95}C$ (SEQ ID NO: 43),

FGAGTKLELK (SEQ ID NO: 44); or

the light chain framework region FRL1, FRL2, FRL3, and FRL4 having sequences shown in SEQ ID NO: 49-52 respectively,

$DIX_{107}MTQSPSSLSX_{108}TAGEKVTX_{109}SC$ (SEQ ID NO: 49),

$WYQQKPGX_{110}X_{111}PKLLIY$ (SEQ ID NO: 50),

$GVPX_{112}RFTGSGSGTDFTLTISSX_{113}QAEDLAVYYC$ (SEQ ID NO: 51),

FGSGTKLEIK (SEQ ID NO: 52),

wherein, $X_{90}$ is V or Q, $X_{91}$ is V or A, $X_{92}$ is M or I, $X_{93}$ is D or S, $X_{94}$ is V or L, $X_{95}$ is F or Y, $X_{107}$ is V or Q, $X_{108}$ is V or A, $X_{109}$ is M or I, $X_{110}$ is Q or K, $X_{111}$ is P or A, $X_{112}$ is D or S, and $X_{113}$ is V or L.

7. The antibody or antigen-binding fragment thereof according to claim 4, wherein the antibody comprises:

the heavy chain framework region FRH1, FRH2, FRH3, and FRH4 having sequences shown in SEQ ID NO: 17-20 respectively,

$X_{29}VQLX_{30}QSGX_{31}EX_{32}X_{33}X_{34}PGASVKX_{35}SCKASGYTFT$ (SEQ ID NO: 17),

$WVX_{36}QX_{37}X_{38}GX_{39}X_{40}LEWX_{41}G$ (SEQ ID NO: 18),

$X_{42}X_{43}TX_{44}TX_{45}X_{46}X_{47}SX_{48}STAYMELX_{49}SLX_{50}SEDX_{51}AVYYCVT$ (SEQ ID NO: 19),

$WGX_{52}GTTVTVSS$ (SEQ ID NO: 20); or

the heavy chain framework region FRH1, FRH2, FRH3, and FRH4 having sequences shown in SEQ ID NO: 25-28 respectively,

$QVQLX_{70}QX_{71}GX_{72}EX_{73}X_{74}KPGASVKX_{75}SCKASGYTFT$ (SEQ ID NO: 25),

$WVX_{76}QX_{77}PGQGLEWX_{78}G$ (SEQ ID NO: 26),

$X_{79}X_{8o}X_{81}TX_{82}TVDX_{83}SX_{84}STX_{85}YMX_{86}LSSLX_{87}SEDX_{88}AVYYCAR$ (SEQ ID NO: 27),

$WGQGTTX_{89}TVSS$ (SEQ ID NO: 28),

wherein, $X_{29}$ is E or Q, $X_{30}$ is Q or V, $X_{31}$ is P or A, $X_{32}$ is L or V, $X_{33}$ is V or K, $X_{34}$ is R or K, $X_{35}$ is M or V, $X_{36}$ is K or R, $X_{37}$ is S or A, $X_{38}$ is H or P, $X_{39}$ is K or Q, $X_{40}$ is S or R, $X_{41}$ is I or M, $X_{42}$ is K or R, $X_{43}$ is A or V, $X_{44}$ is L or I, $X_{45}$ is V or R, $X_{46}$ is N or D, $X_{47}$ is K or T, $X_{48}$ is S or A, $X_{49}$ is R or S, $X_{50}$ is T or R, $X_{51}$ is S or T, $X_{52}$ is T or Q, $X_{70}$ is Q or V, $X_{71}$ is P or S, $X_{72}$ is S or A, $X_{73}$ is L or V, $X_{74}$ is V or K, $X_{75}$ is L or V, $X_{76}$ is K or R, $X_{77}$ is R or A, $X_{78}$ is I or M, $X_{79}$ is S or G, $X_{80}$ is R or K, $X_{81}$ is A or V, $X_{82}$ is L or M, $X_{83}$ is K or T, $X_{84}$ is S or T, $X_{85}$ is A or V, $X_{86}$ is Q or E, $X_{87}$ is T or R, $X_{88}$ is S or T, $X_{89}$ is L or V;

the condition is that when the antibody comprises: the heavy chain variable region CDR1, CDR2, and CDR3 sequences shown in DYNMH (SEQ ID NO: 32), YINPNNGGTSYNQKFKG (SEQ ID NO: 33), TRYLAV (SEQ ID NO: 34), respectively, $X_{29}$ is E, $X_{30}$ is Q, $X_{31}$ is P, $X_{32}$ is L, $X_{33}$ is V, $X_{34}$ is R, $X_{35}$ is M, $X_{36}$ is K, $X_{37}$ is S, $X_{38}$ is H, $X_{39}$ is K, $X_{40}$ is S, $X_{41}$ is I, $X_{42}$ is K, $X_{43}$ is A, $X_{44}$ is L, $X_{45}$ is V, $X_{46}$ is N, $X_{47}$ is K, $X_{48}$ is S, $X_{49}$ is R, $X_{50}$ is T, $X_{51}$ is S, $X_{52}$ is T, which are not established at the same time;

when the antibody comprises: the heavy chain variable region CDR1, CDR2, and CDR3 sequences shown in SYWMH (SEQ ID NO: 38), MIHPNSGSTNYNEKFKS (SEQ ID NO: 39), RYYGSISPDY (SEQ ID NO: 40), respectively, $X_{70}$ is Q, $X_{71}$ is P, $X_{72}$ is S, $X_{73}$ is L, $X_{74}$ is V, $X_{75}$ is L, $X_{76}$ is K, $X_{77}$ is R, $X_{78}$ is I, $X_{79}$ is S, $X_{80}$ is K, $X_{81}$ is A, $X_{82}$ is L, $X_{83}$ is K, $X_{84}$ is S, $X_{85}$ is A, $X_{86}$ is Q, $X_{87}$ is T, $X_{88}$ is S, $X_{89}$ is L, which are not established at the same time.

8. The antibody or antigen-binding fragment thereof according to claim 4, wherein the antibody comprises:

the heavy chain framework region FRH1, FRH2, FRH3, and FRH4 having sequences shown in SEQ ID NO: 45-48 respectively,

$X_{96}VQLQQSGPELVRPGASVKX_{97}SCKASGYTFT$ (SEQ ID NO: 45),

$WVKQSHGX_{98}X_{99}LEWX_{100}G$ (SEQ ID NO: 46),

$X_{101}X_{102}TX_{103}TVX_{104}X_{105}SX_{106}STAYMELRSLTSEDSAVYYCVT$ (SEQ ID NO: 47),

WGTGTTVTVSS (SEQ ID NO: 48); or
the heavy chain framework region FRH1, FRH2, FRH3, and FRH4 having sequences shown in SEQ ID NO: 53-56 respectively,
QVQLQQPGSELVKPGASVKX$_{114}$SCKASGYTFT (SEQ ID NO: 53),
WVKQX$_{115}$PGQGLEWX$_{116}$G (SEQ ID NO: 54),
X$_{117}$X$_{118}$TX$_{119}$TVDX$_{120}$SSSTX$_{121}$YMQLSSLTSEDSAVYYCAR (SEQ ID NO: 55),
WGQGTTLTVSS (SEQ ID NO: 56),
wherein, X$_{96}$ is E or Q, X$_{97}$ is M or V, X$_{98}$ is K or Q, X$_{99}$ is S or R, X$_{100}$ is I or M, X$_{101}$ is K or R, X$_{102}$ is A or V, X$_{103}$ is L or I, X$_{104}$ is N or D, X$_{105}$ is K or T, X$_{106}$ is S or A, X$_{114}$ is L or V, X$_{115}$ is R or A, X$_{116}$ is I or M, X$_{117}$ is R or K, X$_{118}$ is A or V, X$_{119}$ is L or M, X$_{120}$ is K or T, X$_{121}$ is A or V

9. The antibody or antigen-binding fragment thereof according to claim 1, wherein the antibody has a light chain variable region with an amino acid sequence shown in SEQ ID NO: 57-59 or SEQ ID NO: 65-67.

10. The antibody or antigen-binding fragment thereof according to claim 1, wherein the antibody has a heavy chain variable region with an amino acid sequence shown in SEQ ID NO: 60-64 or SEQ ID NO: 68-72,

11. The antibody or antigen-binding fragment thereof according to claim 1, wherein the antibody comprises at least one of a heavy chain constant region and a light chain constant region, and at least a part of at least one of the heavy chain constant region and the light chain constant region is derived from at least one of a human antibody;

   optionally, both the light chain constant region and the heavy chain constant region of the antibody are derived from a human IgG antibody or a mutant thereof;
   optionally, the light chain constant region of the antibody is derived from light chain constant region of human Kappa; the heavy chain constant region of the antibody is derived from human IgG4 or IgG1, or a mutant of IgG4 or IgG1.

12. The antibody or antigen-binding fragment thereof according to claim 9, wherein the full-length sequence of the antibody constant region is shown in SEQ ID NO:73, 74 or 75.

13. The antibody or antigen-binding fragment thereof according to claim 9, wherein the antibody has a light chain as shown in any one of SEQ ID NO: 76-78 and heavy chain as shown in any one of SEQ ID NO: 79-88.

14. The antibody or antigen-binding fragment thereof according to claim 9, wherein the antibody has a light chain as shown in any one of SEQ ID NO: 89-91 and a heavy chain as shown in any one of SEQ ID NO: 92-101.

15. The antibody or antigen-binding fragment thereof according to claim 9, wherein the antibody comprises:

   the light chain as shown in SEQ ID NO: 76, the heavy chain as shown in SEQ ID NO: 79, or
   the light chain as shown in SEQ ID NO: 77, the heavy chain as shown in SEQ ID NO: 79, or
   the light chain as shown in SEQ ID NO: 76, the heavy chain as shown in SEQ ID NO: 80, or
   the light chain as shown in SEQ ID NO: 77, the heavy chain as shown in SEQ ID NO: 80, or
   the light chain as shown in SEQ ID NO: 76, the heavy chain as shown in SEQ ID NO: 81, or
   the light chain as shown in SEQ ID NO: 77, the heavy chain as shown in SEQ ID NO: 81, or
   the light chain as shown in SEQ ID NO: 78, the heavy chain as shown in SEQ ID NO: 81, or
   the light chain as shown in SEQ ID NO: 77, the heavy chain as shown in SEQ ID NO: 82, or
   the light chain as shown in SEQ ID NO: 78, the heavy chain as shown in SEQ ID NO: 82, or
   the light chain as shown in SEQ ID NO: 77, the heavy chain as shown in SEQ ID NO: 83, or
   the light chain as shown in SEQ ID NO: 78, the heavy chain as shown in SEQ ID NO: 83, or
   the light chain as shown in SEQ ID NO: 76, the heavy chain as shown in SEQ ID NO: 84, or
   the light chain as shown in SEQ ID NO: 77, the heavy chain as shown in SEQ ID NO: 84, or
   the light chain as shown in SEQ ID NO: 76, the heavy chain as shown in SEQ ID NO: 85, or
   the light chain as shown in SEQ ID NO: 77, the heavy chain as shown in SEQ ID NO: 85, or
   the light chain as shown in SEQ ID NO: 76, the heavy chain as shown in SEQ ID NO: 86, or
   the light chain as shown in SEQ ID NO: 77, the heavy chain as shown in SEQ ID NO: 86, or
   the light chain as shown in SEQ ID NO: 78, the heavy chain as shown in SEQ ID NO: 86, or
   the light chain as shown in SEQ ID NO: 77, the heavy chain as shown in SEQ ID NO: 87, or
   the light chain as shown in SEQ ID NO: 78, the heavy chain as shown in SEQ ID NO: 87, or

the light chain as shown in SEQ ID NO: 77, the heavy chain as shown in SEQ ID NO: 88, or
the light chain as shown in SEQ ID NO: 78, the heavy chain as shown in SEQ ID NO: 88.

16. The antibody or antigen-binding fragment thereof according to claim 9, wherein the antibody comprises:

the light chain as shown in SEQ ID NO: 89, the heavy chain as shown in SEQ ID NO: 92, or
the light chain as shown in SEQ ID NO: 90, the heavy chain as shown in SEQ ID NO: 92, or
the light chain as shown in SEQ ID NO: 89, the heavy chain as shown in SEQ ID NO: 93, or
the light chain as shown in SEQ ID NO: 90, the heavy chain as shown in SEQ ID NO: 93, or
the light chain as shown in SEQ ID NO: 89, the heavy chain as shown in SEQ ID NO: 94, or
the light chain as shown in SEQ ID NO: 90, the heavy chain as shown in SEQ ID NO: 94, or
the light chain as shown in SEQ ID NO: 91, the heavy chain as shown in SEQ ID NO: 94, or
the light chain as shown in SEQ ID NO: 90, the heavy chain as shown in SEQ ID NO: 95, or
the light chain as shown in SEQ ID NO: 91, the heavy chain as shown in SEQ ID NO: 95, or
the light chain as shown in SEQ ID NO: 90, the heavy chain as shown in SEQ ID NO: 96, or
the light chain as shown in SEQ ID NO: 91, the heavy chain as shown in SEQ ID NO: 96, or
the light chain as shown in SEQ ID NO: 89, the heavy chain as shown in SEQ ID NO: 97, or
the light chain as shown in SEQ ID NO: 90, the heavy chain as shown in SEQ ID NO: 97, or
the light chain as shown in SEQ ID NO: 89, the heavy chain as shown in SEQ ID NO: 98, or
the light chain as shown in SEQ ID NO: 90, the heavy chain as shown in SEQ ID NO: 98, or
the light chain as shown in SEQ ID NO: 89, the heavy chain as shown in SEQ ID NO: 99, or
the light chain as shown in SEQ ID NO: 90, the heavy chain as shown in SEQ ID NO: 99, or
the light chain as shown in SEQ ID NO: 91, the heavy chain as shown in SEQ ID NO: 99, or
the light chain as shown in SEQ ID NO: 90, the heavy chain as shown in SEQ ID NO: 100, or
the light chain as shown in SEQ ID NO: 91, the heavy chain as shown in SEQ ID NO: 100, or
the light chain as shown in SEQ ID NO: 90, the heavy chain as shown in SEQ ID NO: 101, or
the light chain as shown in SEQ ID NO: 91, the heavy chain as shown in SEQ ID NO: 101.

17. The antibody or antigen-binding fragment thereof according to claim 9, wherein the antibody is a single chain antibody, a multimeric antibody, or a CDR-grafted antibody or a small molecule antibody;
optionally, the small molecule antibody includes at least one of a Fab antibody, a Fv antibody, a single domain antibody and a minimum recognition unit.

18. A nucleic acid molecule, wherein the nucleic acid molecule encodes the antibody or antigen-binding fragment thereof according to any one of claims 1~17.

19. An expression vector, wherein the expression vector carries the nucleic acid molecule of any one of claims 18-23.

20. The expression vector according to claim 19, wherein the expression vector is eukaryotic expression vector or virus;
optionally, the virus is a lentivirus.

21. An immune cell, wherein the immune cell carries the nucleic acid molecule of claim 18, the expression vector of any one of claims 19-20, or expresses the antibody or antigen-binding fragment thereof of any one of claims 1~17;
optionally, the immune cell comprises at least one of T lymphocytes, DC cells, NK cells, and NKT lymphocytes.

22. A recombinant cell, wherein the recombinant cell carries the nucleic acid molecule of claim 18, the expression vector of any one of claims 19-20, or expresses the antibody or antigen-binding fragment thereof of any one of claims 1-17.

23. The recombinant cell according to claim 22, wherein the recombinant cell is obtained by introducing the expression vector of any one of claims 19-20 into a host cell;
optionally, the expression vector is introduced into the host cell by electrotransduction.

24. The recombinant cell according to claim 22, wherein the recombinant cell is a eukaryotic cell;
optionally, the recombinant cell is a mammalian cell.

25. A CAR-T cell, wherein the CAR-T cell comprises a chimeric antigen receptor, wherein,
the chimeric antigen receptor includes:

an extracellular region, wherein the extracellular region includes a heavy chain variable region and a light chain variable region of a single-chain antibody, wherein the light chain variable region has an amino acid sequence as shown in SEQ ID NO: 57-59 or SEQ ID NO: 65-67, and the heavy chain variable region has an amino acid sequence as shown in SEQ ID NO: 60-64 or SEQ ID NO: 68-72;

a transmembrane region, wherein the transmembrane region is connected to the extracellular region and embedded in the cell membrane of the T lymphocyte; and

an intracellular region, wherein the intracellular region is connected to the transmembrane region.

26. The CAR-T cell according to claim 25, wherein the transmembrane region is an ICOS transmembrane region, an CD8 transmembrane region or a OX40 transmembrane region;

preferably, the transmembrane region is a CD8 transmembrane region;
more preferably, the amino acid sequence of the transmembrane region is shown in SEQ ID NO: 106.

27. The CAR-T cell according to claim 25, wherein the intracellular segment is a 4-1BB intracellular segment and an ICOS or OX-40 intracellular segment;

preferably, the intracellular segment is a 4-1BB intracellular segment and an ICOS intracellular segment;
more preferably, the amino acid sequence of the 4-1BB intracellular segment is shown in SEQ ID NO: 109; the amino acid sequence of the ICOS intracellular segment is shown in SEQ ID NO: 110.

28. The CAR-T cell according to claim 25, wherein the extracellular region further comprises a CD8 hinge region;
preferably, the amino acid sequence of the CD8 hinge region is shown in SEQ ID NO: 111.

29. The CAR-T cell according to claim 25, wherein the intracellular segment further comprises a CD3ζ chain;
preferably, the amino acid sequence of the CD3ζ chain is shown in SEQ ID NO: 112.

30. The CAR-T cell according to claim 28, wherein the N-terminus of the intracellular segment of ICOS is connected to the C-terminus of the CD8 transmembrane region, the C-terminus of the intracellular segment of ICOS is connected to the N-terminus of the intracellular segment of 4-1BB, and the C-terminus of the intracellular segment of 4-1BB is connected to the N-terminus of the CD3ζ chain.

31. A pharmaceutical composition, wherein the pharmaceutical composition includes the antibody of any one of claims 1-17, the nucleic acid molecule of claim 18, the expression vector of any one of claims 19-20, the immune cell of claim 21, the recombinant cell of any one of claims 22-24 or the CAR-T cell of any one of claims 25-30.

32. Use of the antibody of any one of claims 1-17, the nucleic acid molecule of claim 18, the expression vector of any one of claims 19-20, the immune cell of claim 21, the recombinant cell of any one of claims 22-24 or the CAR-T cell of any one of claims 25-30 or the pharmaceutical composition of claim 31 in the preparation of a medicine for treating or preventing Claudin18.2 related diseases;
optionally, the Claudin18.2 related diseases include: at least one of gastric cancer, esophageal cancer, pancreatic cancer, lung cancer, colon cancer and rectal cancer.

33. A kit for detecting Claudin18.2, wherein the kit comprises the antibody of any one of claims 1-17.

34. Use of the antibody of any one of claims 1-17, the nucleic acid molecule of claim 18, the expression vector of any one of claims 19-20 or the recombinant cell of any one of claims 22-24 in the preparation of a kit for treating or preventing Claudin18.2 related diseases.

35. A method for treating or preventing Claudin18.2 related diseases, wherein the method comprising administering to a subject the antibody of any one of claims 1-17, the nucleic acid molecule of claim 18, the expression vector of any one of claims 19-20, the immune cell of claim 21, the recombinant cell of any one of claims 22-24, the CAR-T cell of any one of claims 25-30 or the pharmaceutical composition of claim 31;
optionally, the Claudin18.2 related diseases include: at least one of gastric cancer, esophageal cancer, pancreatic cancer, lung cancer, colon cancer and rectal cancer.

36. The antibody of any one of claims 1-17, the nucleic acid molecule of claim 18, the expression vector of any one of claims 19-20, the immune cell of claim 21, the recombinant cell of any one of claims 22-24 or the CAR-T cell of any one of claims 25-30 or the pharmaceutical composition of claim 31 for use in treating or preventing Claudin18.2 related diseases.
optionally, the Claudin18.2 related diseases include: at least one of gastric cancer, esophageal cancer, pancreatic cancer, lung cancer, colon cancer and rectal cancer.

| Sample name | MW (kDa) | Reduced purity(%) |
|---|---|---|
| 1B6-mVH-HC(IgG1)mVL | 145.74 | >95.0 |
| 1B6-huVH1-HC(IgG1)huVL1 | 145.68 | >95.0 |
| 1B6-huVH1-HC(IgG1)huVL2 | 145.68 | >95.0 |
| 1B6-huVH2-HC(IgG1)huVL1 | 145.74 | >95.0 |
| 1B6-huVH2-HC(IgG1)huVL2 | 145.74 | >95.0 |
| 1B6-huVH3-HC(IgG1)huVL1 | 145.78 | >95.0 |

Figure 1

| Sample name | MW (kDa) | Reduced Purity (%) |
|---|---|---|
| 1B6-huVH3-HC(IgG1)huVL2 | 145.78 | >95.0 |
| 1B6-huVH3-HC(IgG1)huVL3 | 145.8 | >95.0 |
| 1B6-huVH4-HC(IgG1)huVL2 | 145.82 | >95.0 |
| 1B6-huVH4-HC(IgG1)huVL3 | 145.84 | >95.0 |
| 1B6-huVH5-HC(IgG1)huVL2 | 145.88 | >95.0 |
| 1B6-huVH5-HC(IgG1)huVL3 | 145.9 | >95.0 |

Figure 2

| | Non-Reduced | | | | | | | M | Reduced | | | | | | | Sample name | MW (kDa) | Reduced purity(%) |
|---|---|---|---|---|---|---|---|---|---|---|---|---|---|---|---|---|---|---|
| Samples | 1 | 2 | 3 | 4 | 5 | 6 | IPI | | 1 | 2 | 3 | 4 | 5 | 6 | IPI | | | |
| | | | | | | | | | | | | | | | | 1E7-mVH-HC(IgG1)mVL | 146.88 | >95.0 |
| | | | | | | | | | | | | | | | | 1E7-huVH1-HC(IgG1)huVL1 | 147.12 | >95.0 |
| | | | | | | | | | | | | | | | | 1E7-huVH1-HC(IgG1)huVL2 | 147.04 | >95.0 |
| | | | | | | | | | | | | | | | | 1E7-huVH2-HC(IgG1)huVL1 | 146.94 | >95.0 |
| | | | | | | | | | | | | | | | | 1E7-huVH2-HC(IgG1)huVL2 | 146.86 | >95.0 |
| | | | | | | | | | | | | | | | | 1E7-huVH3-HC(IgG1)huVL1 | 147.0 | >95.0 |

Figure 3

| | Non-Reduced | | | | | | | M | Reduced | | | | | | | Sample name | MW (kDa) | Reduced purity(%) |
|---|---|---|---|---|---|---|---|---|---|---|---|---|---|---|---|---|---|---|
| Samples | 1 | 2 | 3 | 4 | 5 | 6 | IPI | | 1 | 2 | 3 | 4 | 5 | 6 | IPI | | | |
| | | | | | | | | | | | | | | | | 1E7-huVH3-HC(IgG1)huVL2 | 146.94 | >95.0 |
| | | | | | | | | | | | | | | | | 1E7-huVH3-HC(IgG1)huVL3 | 146.96 | >95.0 |
| | | | | | | | | | | | | | | | | 1E7-huVH4-HC(IgG1)huVL2 | 147.02 | >95.0 |
| | | | | | | | | | | | | | | | | 1E7-huVH4-HC(IgG1)huVL3 | 147.04 | >95.0 |
| | | | | | | | | | | | | | | | | 1E7-huVH5-HC(IgG1)huVL2 | 146.98 | >95.0 |
| | | | | | | | | | | | | | | | | 1E7-huVH5-HC(IgG1)huVL3 | 147.0 | >95.0 |

Figure 4

| Non-Reduced | | | | | | | | Reduced | | | | | | | | Sample name | MW (kDa) | Reduced purity(%) |
|---|---|---|---|---|---|---|---|---|---|---|---|---|---|---|---|---|---|---|
| Samples | 1 | 2 | 3 | 4 | 5 | 6 | IPI | M | 1 | 2 | 3 | 4 | 5 | 6 | IPI | 1B6-mVHmVL | 145.44 | >95.0 |
| | | | | | | | | | | | | | | | | 1B6-huVH1huVL1 | 145.38 | >95.0 |
| | | | | | | | | | | | | | | | | 1B6-huVH1huVL2 | 145.38 | >95.0 |
| | | | | | | | | | | | | | | | | 1B6-huVH2huVL1 | 145.42 | >95.0 |
| | | | | | | | | | | | | | | | | 1B6-huVH2huVL2 | 145.44 | >95.0 |
| | | | | | | | | | | | | | | | | 1B6-huVH3huVL1 | 145.48 | >95.0 |

Figure 5

| Non-Reduced | | | | | | | | Reduced | | | | | | | | Sample name | MW (kDa) | Reduced purity(%) |
|---|---|---|---|---|---|---|---|---|---|---|---|---|---|---|---|---|---|---|
| Samples | 1 | 2 | 3 | 4 | 5 | 6 | IPI | M | 1 | 2 | 3 | 4 | 5 | 6 | IPI | 1B6-huVH3huVL2 | 145.48 | >95.0 |
| | | | | | | | | | | | | | | | | 1B6-huVH3huVL3 | 145.5 | >95.0 |
| | | | | | | | | | | | | | | | | 1B6-huVH4huVL2 | 145.52 | >95.0 |
| | | | | | | | | | | | | | | | | 1B6-huVH4huVL3 | 145.54 | >95.0 |
| | | | | | | | | | | | | | | | | 1B6-huVH5huVL2 | 145.58 | >95.0 |
| | | | | | | | | | | | | | | | | 1B6-huVH5huVL3 | 145.6 | >95.0 |

Figure 6

49

EP 4 495 140 A1

Figure 7

Figure 8

50

Figure 9

Figure 10

Figure 11

Figure 12

## ADCC 1B6

- 1B6-mVH-HC(IgG1)Mvl
- 1B6-huVH2-HC(IgG1)huVL1
- 1B6-huVH2-HC(IgG1)huVL2
- 1B6-mVH-HC(IgG1)mVL
- 1B6-huVH2-HC(IgG1)huVL1
- 1B6-huVH4-HC(IgG1)huVL3

Figure 13

## ADCC 1E7

- 1E7-mVH-HC(IgG1)mVL
- 1E7-huVH1-HC(IgG1)huVL1
- 1E7-huVH1-HC(IgG1)huVL2
- 1E7-huVH2-HC(IgG1)huVL1
- 1E7-huVH3-HC(IgG1)huVL2

Figure 14

## CDC 1B6

- 1B6-mVH-HC(IgG1)Mvl
- 1B6-huVH2-HC(IgG1)huVL1
- 1B6-huVH3-HC(IgG1)huVL1

X=Log(Concentration)(ug/ml)

Figure 15

## CDC 1E7

- 1E7-huVH1-HC(IgG1)huVL1
- 1E7-huVH1-HC(IgG1)huVL2
- 1E7-huVH2-HC(IgG1)huVL1
- 1E7-mVH-HC(IgG1)Mvl
- 1E7-huVH3-HC(IgG1)huVL2

X=Log(Concentration)(ug/ml)

Figure 16

Figure 17-A

Figure 17-B

Figure 17-C

Figure 18

Changes in transplanted tumor volume in the MDA-MB-468-18.2 model in NCG mice (Mean ± SEM)

Figure 19

## INTERNATIONAL SEARCH REPORT

International application No.

**PCT/CN2023/082174**

### A. CLASSIFICATION OF SUBJECT MATTER

C07K16/28(2006.01)i; C07K19/00(2006.01)i; C12N5/10(2006.01)i; C12N15/13(2006.01)i; C12N15/62(2006.01)i; C12N15/867(2006.01)i; G01N33/574(2006.01)i; G01N33/68(2006.01)i; A61K39/00(2006.01)i; A61P35/00(2006.01)i

According to International Patent Classification (IPC) or to both national classification and IPC

### B. FIELDS SEARCHED

Minimum documentation searched (classification system followed by classification symbols)

IPC: C07K16; C07K19; C12N5; C12N15; G01N33; A61K39; A61P35; C07K14

Documentation searched other than minimum documentation to the extent that such documents are included in the fields searched

Electronic data base consulted during the international search (name of data base and, where practicable, search terms used)

CNTXT, ENTXTC, ENTXT, OETXT, VEN, CJFD, PubMed, ISI web of knowledge, GenBank, EMBL, STN, CNKI, 万方, WANFANG, 百度, BAIDU, 百度学术, BAIDU SCHOLAR, 中国专利生物序列检索系统, China Patent Biological Sequence Search System: 抗体, 结合蛋白, 结合片段, 嵌合抗原受体, 人源化, CLDN18.2, Claudin18.2, antibody, CAR-T, humanized, 根据氨基酸序列SEQ ID NO: 1-12的序列进行了序列检索, sequence search based on amino acid sequence SEQ ID NO: 1-12

### C. DOCUMENTS CONSIDERED TO BE RELEVANT

| Category* | Citation of document, with indication, where appropriate, of the relevant passages | Relevant to claim No. |
|---|---|---|
| PY | CN 114560941 A (GUANGDONG HEC PHARMACEUTICAL CO., LTD.) 31 May 2022 (2022-05-31)<br>    description paragraphs 0031-0058, 0075-0094 | 26-32, 36 |
| Y | CN 110831961 A (OBSIDIAN THERAPEUTICS INC.) 21 February 2020 (2020-02-21)<br>    description paragraphs 0011-0034, 0228-0295, sequence table | 26-32, 36 |
| A | CN 112940124 A (NANJING KAEDI MEDICAL TECHNOLOGY CO., LTD.) 11 June 2021 (2021-06-11)<br>    description paragraphs 0006-0101 | 1-34, 36 |
| A | CN 114127109 A (SHANDONG BOAN BIOTECHNOLOGY CO., LTD.) 01 March 2022 (2022-03-01)<br>    description paragraphs 0006-0059 | 1-34, 36 |
| A | CN 113788894 A (SHENZHEN XIANKANGDA LIFE SCIENCE CO., LTD.) 14 December 2021 (2021-12-14)<br>    description paragraphs 0008-0048 | 1-34, 36 |

☐ Further documents are listed in the continuation of Box C.    ☑ See patent family annex.

| * | Special categories of cited documents: | | |
|---|---|---|---|
| "A" | document defining the general state of the art which is not considered to be of particular relevance | "T" | later document published after the international filing date or priority date and not in conflict with the application but cited to understand the principle or theory underlying the invention |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **15 June 2023** | **28 June 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2023/082174** |

**Box No. I      Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  [✓]  forming part of the international application as filed.

    b.  [ ]  furnished subsequent to the international filing date for the purposes of international search (Rule 13*ter*.1(a)),

          [ ]  accompanied by a statement to the effect that the sequence listing does not go beyond the disclosure in the international application as filed.

2.  [ ]  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**

| International application No. |
|---|
| **PCT/CN2023/082174** |

| Box No. II      Observations where certain claims were found unsearchable (Continuation of item 2 of first sheet) |
|---|

This international search report has not been established in respect of certain claims under Article 17(2)(a) for the following reasons:

1. ☑ Claims Nos.: **35**
   because they relate to subject matter not required to be searched by this Authority, namely:

   Claim 35 relates to a method for treating or preventing Claudin18.2-related diseases, wherein a method for treating diseases falls within subject matter for which no search is required by the International Searching Authority as defined in PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2023/082174**

| Patent document cited in search report | | | Publication date (day/month/year) | Patent family member(s) | | | Publication date (day/month/year) |
|---|---|---|---|---|---|---|---|
| CN | 114560941 | A | 31 May 2022 | WO | 2022111633 | A1 | 02 June 2022 |
| CN | 110831961 | A | 21 February 2020 | KR | 20200010181 | A | 30 January 2020 |
| | | | | JP | 2020511529 | A | 16 April 2020 |
| | | | | SG | 10202001869 | VA | 29 April 2020 |
| | | | | WO | 2018161017 | A1 | 07 September 2018 |
| | | | | CA | 3055202 | A1 | 07 September 2018 |
| | | | | SG | 11201907922 | PA | 27 September 2019 |
| | | | | AU | 2018227583 | A1 | 17 October 2019 |
| | | | | AU | 2018227583 | B2 | 01 June 2023 |
| | | | | EP | 3589646 | A1 | 08 January 2020 |
| | | | | IN | 201917035410 | A | 01 November 2019 |
| CN | 112940124 | A | 11 June 2021 | CN | 112940124 | B | 11 June 2021 |
| CN | 114127109 | A | 01 March 2022 | JP | 2022527129 | A | 30 May 2022 |
| | | | | JP | 7266117 | B2 | 27 April 2023 |
| | | | | KR | 20220006085 | A | 14 January 2022 |
| | | | | EP | 3929214 | A1 | 29 December 2021 |
| | | | | CA | 3136281 | A1 | 03 December 2020 |
| | | | | US | 2022204609 | A1 | 30 June 2022 |
| | | | | AU | 2020281380 | A1 | 07 October 2021 |
| | | | | WO | 2020239005 | A1 | 03 December 2020 |
| | | | | BR | 112021023897 | A2 | 25 January 2022 |
| | | | | CN | 114127109 | B | 21 June 2022 |
| CN | 113788894 | A | 14 December 2021 | CN | 114751984 | B | 14 February 2023 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• CN 202210272549